# EUROPEAN PATENT SPECIFICATION

(11) **EP 3 124 625 B1**
(45) Date of publication and mention of the grant of the patent: **24.07.2019**
(21) Application number: 16189740.0
(22) Date of filing: 30.12.2010
(51) Int. Cl.: C12Q 1/68

(54) **miRNA fingerprint in the diagnosis of prostate cancer**
miRNA-Fingerabdruck für die Krankheitsdiagnose
Empreinte ARNm dans le diagnostic de maladies

(30) Priority: 30.12.2009 US 291074 P; 30.12.2009 EP 09181024
(43) Date of publication of application: 01.02.2017
(62) Divisional of application: 10801172.7
(73) Proprietor: Hummingbird Diagnostics GmbH, 69120 Heidelberg (DE)
(72) Inventor: Keller, Andreas, 66346 Püttlingen (DE); Meese, Eckart, 66882 Huetschenhausen (DE); Borries, Anne, 68169 Mannheim (DE); Beier, Markus, 69469 Weinheim (DE)
(74) Representative: Geling, Andrea

(56) References cited:
- WO-A1-2009/070653
- WO-A2-2009/108860
- PORKKA KATI P ET AL: "MicroRNA expression profiling in prostate cancer", CANCER RESEARCH, AMERICAN ASSOCIATION FOR CANCER RESEARCH, US, vol. 67, no. 13, 1 July 2007 (2007-07-01), pages 6130-6135, XP002602579, ISSN: 0008-5472, DOI: 10.1158/0008-5472.CAN-07-0533
- KELLER ANDREAS ET AL: "miRNAs in lung cancer - Studying complex fingerprints in patient's blood cells by microarray experiments", BMC CANCER, BIOMED CENTRAL, LONDON, GB, vol. 9, no. 1, 6 October 2009 (2009-10-06) , page 353, XP021062692, ISSN: 1471-2407, DOI: DOI:10.1186/1471-2407-9-353
- OTAEGUI DAVID ET AL: "Differential micro RNA expression in PBMC from multiple sclerosis patients", PL O S ONE, PUBLIC LIBRARY OF SCIENCE, US, vol. 4, no. 7, 1 January 2009 (2009-01-01) , pages E6309.1-E6309.9, XP002596361, ISSN: 1932-6203
- YOONTAE LEE ET AL: "MicroRNA genes are transcribed by RNA polymerase II", EMBO JOURNAL., vol. 23, no. 20, 13 October 2004 (2004-10-13), pages 4051-4060, XP055309182, GB ISSN: 0261-4189, DOI: 10.1038/sj.emboj.7600385

### SEQUENCE LISTING

<110> Hummingbird Diagnostics GmbH
<120> miRNA fingerprint in the diagnosis of prostate cancer
<130> 505-53 PCTEPT1
<140>
   <141>
<160> 962
<170> PatentIn version 3.3
<210> 1
   <211> 22
   <212> RNA
   <213> Homo sapiens
<400> 1
   caagcucgug ucuguggguc cg 22
<210> 2
   <211> 22
   <212> RNA
   <213> Homo sapiens
<400> 2
   cacccguaga accgaccuug cg 22
<210> 3
   <211> 22
   <212> RNA
   <213> Homo sapiens
<400> 3
   caagcucgcu ucuauggguc ug 22
<210> 4
   <211> 22
   <212> RNA
   <213> Homo sapiens
<400> 4
   aacccguaga uccgaucuug ug 22
<210> 5
   <211> 22
   <212> RNA
   <213> Homo sapiens
<400> 5
   ugagguagua aguuguauug uu 22
<210> 6
   <211> 22
   <212> RNA
   <213> Homo sapiens
<400> 6
   aaucaugugc agugccaaua ug 22
<210> 7
   <211> 23
   <212> RNA
   <213> Homo sapiens
<400> 7
   uuuggcacua gcacauuuuu gcu 23
<210> 8
   <211> 22
   <212> RNA
   <213> Homo sapiens
<400> 8
   uucaacgggu auuuauugag ca 22
<210> 9
   <211> 22
   <212> RNA
   <213> Homo sapiens
<400> 9
   aaauuauugu acaucggaug ag 22
<210> 10
   <211> 21
   <212> RNA
   <213> Homo sapiens
<400> 10
   cugacuguug ccguccucca g 21
<210> 11
   <211> 22
   <212> RNA
   <213> Homo sapiens
<400> 11
   ucuucucugu uuuggccaug ug 22
<210> 12
   <211> 23
   <212> RNA
   <213> Homo sapiens
<400> 12
   cacccggcug ugugcacaug ugc 23
<210> 13
   <211> 21
   <212> RNA
   <213> Homo sapiens
<400> 13
   aaggcagggc ccccgcuccc c 21
<210> 14
   <211> 24
   <212> RNA
   <213> Homo sapiens
<400> 14
   uggggagcug aggcucuggg ggug 24
<210> 15
   <211> 22
   <212> RNA
   <213> Homo sapiens
<400> 15
   ugcccuuaaa ggugaaccca gu 22
<210> 16
   <211> 22
   <212> RNA
   <213> Homo sapiens
<400> 16
   auccgcgcuc ugacucucug cc 22
<210> 17
   <211> 22
   <212> RNA
   <213> Homo sapiens
<400> 17
   acaguagagg gaggaaucgc ag 22
<210> 18
   <211> 23
   <212> RNA
   <213> Homo sapiens
<400> 18
   ccaguuaccg cuuccgcuac cgc 23
<210> 19
   <211> 22
   <212> RNA
   <213> Homo sapiens
<400> 19
   ugucuacuac uggagacacu gg 22
<210> 20
   <211> 22
   <212> RNA
   <213> Homo sapiens
<400> 20
   ugugcgcagg gagaccucuc cc 22
<210> 21
   <211> 22
   <212> RNA
   <213> Homo sapiens
<400> 21
   acugcugagc uagcacuucc cg 22
<210> 22
   <211> 23
   <212> RNA
   <213> Homo sapiens
<400> 22
   caaagugcug uucgugcagg uag 23
<210> 23
   <211> 22
   <212> RNA
   <213> Homo sapiens
<400> 23
   agggacggga cgcggugcag ug 22
<210> 24
   <211> 22
   <212> RNA
   <213> Homo sapiens
<400> 24
   uauugcacuc gucccggccu cc 22
<210> 25
   <211> 22
   <212> RNA
   <213> Homo sapiens
<400> 25
   ggguggggau uuguugcauu ac 22
<210> 26
   <211> 23
   <212> RNA
   <213> Homo sapiens
<400> 26
   agguugggau cgguugcaau gcu 23
<210> 27
   <211> 22
   <212> RNA
   <213> Homo sapiens
<400> 27
   uauugcacuu gucccggccu gu 22
<210> 28
   <211> 20
   <212> RNA
   <213> Homo sapiens
<400> 28
   agagucuugu gaugucuugc 20
<210> 29
   <211> 23
   <212> RNA
   <213> Homo sapiens
<400> 29
   gcagcagaga auaggacuac guc 23
<210> 30
   <211> 25
   <212> RNA
   <213> Homo sapiens
<400> 30
   cuagugaggg acagaaccag gauuc 25
<210> 31
   <211> 20
   <212> RNA
   <213> Homo sapiens
<400> 31
   ggggagcugu ggaagcagua 20
<210> 32
   <211> 22
   <212> RNA
   <213> Homo sapiens
<400> 32
   auaaagcuag auaaccgaaa gu 22
<210> 33
   <211> 23
   <212> RNA
   <213> Homo sapiens
<400> 33
   ucuuugguua ucuagcugua uga 23
<210> 34
   <211> 22
   <212> RNA
   <213> Homo sapiens
<400> 34
   cacuggcucc uuucugggua ga 22
<210> 35
   <211> 21
   <212> RNA
   <213> Homo sapiens
<400> 35
   cacugugucc uuucugcgua g 21
<210> 36
   <211> 22
   <212> RNA
   <213> Homo sapiens
<400> 36
   ugcaacuuac cugagucauu ga 22
<210> 37
   <211> 22
   <212> RNA
   <213> Homo sapiens
<400> 37
   ugcaacgaac cugagccacu ga 22
<210> 38
   <211> 21
   <212> RNA
   <213> Homo sapiens
<400> 38
   uacuuggaaa ggcaucaguu g 21
<210> 39
   <211> 21
   <212> RNA
   <213> Homo sapiens
<400> 39
   uuaauaucgg acaaccauug u 21
<210> 40
   <211> 22
   <212> RNA
   <213> Homo sapiens
<400> 40
   gacugacacc ucuuugggug aa 22
<210> 41
   <211> 21
   <212> RNA
   <213> Homo sapiens
<400> 41
   uacucaaaaa gcugucaguc a 21
<210> 42
   <211> 22
   <212> RNA
   <213> Homo sapiens
<400> 42
   gugaacgggc gccaucccga gg 22
<210> 43
   <211> 23
   <212> RNA
   <213> Homo sapiens
<400> 43
   cgggucggag uuagcucaag cgg 23
<210> 44
   <211> 21
   <212> RNA
   <213> Homo sapiens
<400> 44
   cgcgggugcu uacugacccu u 21
<210> 45
   <211> 22
   <212> RNA
   <213> Homo sapiens
<400> 45
   uccauuacac uacccugccu cu 22
<210> 46
   <211> 22
   <212> RNA
   <213> Homo sapiens
<400> 46
   aggcagcggg guguagugga ua 22
<210> 47
   <211> 21
   <212> RNA
   <213> Homo sapiens
<400> 47
   uccucuucuc ccuccuccca g 21
<210> 48
   <211> 20
   <212> RNA
   <213> Homo sapiens
<400> 48
   guagaggaga uggcgcaggg 20
<210> 49
   <211> 22
   <212> RNA
   <213> Homo sapiens
<400> 49
   uggauuucuu ugugaaucac ca 22
<210> 50
   <211> 22
   <212> RNA
   <213> Homo sapiens
<400> 50
   uggugguuua caaaguaauu ca 22
<210> 51
   <211> 22
   <212> RNA
   <213> Homo sapiens
<400> 51
   uauaccucag uuuuaucagg ug 22
<210> 52
   <211> 21
   <212> RNA
   <213> Homo sapiens
<400> 52
   ccuggaaaca cugagguugu g 21
<210> 53
   <211> 22
   <212> RNA
   <213> Homo sapiens
<400> 53
   cugcccuggc ccgagggacc ga 22
<210> 54
   <211> 21
   <212> RNA
   <213> Homo sapiens
<400> 54
   gcaggaacuu gugagucucc u 21
<210> 55
   <211> 23
   <212> RNA
   <213> Homo sapiens
<400> 55
   caguaacaaa gauucauccu ugu 23
<210> 56
   <211> 23
   <212> RNA
   <213> Homo sapiens
<400> 56
   uccaguacca cgugucaggg cca 23
<210> 57
   <211> 22
   <212> RNA
   <213> Homo sapiens
<400> 57
   ugagaccucu ggguucugag cu 22
<210> 58
   <211> 23
   <212> RNA
   <213> Homo sapiens
<400> 58
   cugggaucuc cggggucuug guu 23
<210> 59
   <211> 23
   <212> RNA
   <213> Homo sapiens
<400> 59
   ugcaccaugg uugucugagc aug 23
<210> 60
   <211> 23
   <212> RNA
   <213> Homo sapiens
<400> 60
   ucugcucaua ccccaugguu ucu 23
<210> 61
   <211> 22
   <212> RNA
   <213> Homo sapiens
<400> 61
   acuccagccc cacagccuca gc 22
<210> 62
   <211> 21
   <212> RNA
   <213> Homo sapiens
<400> 62
   uggaggagaa ggaaggugau g 21
<210> 63
   <211> 22
   <212> RNA
   <213> Homo sapiens
<400> 63
   gcaggugcuc acuuguccuc cu 22
<210> 64
   <211> 22
   <212> RNA
   <213> Homo sapiens
<400> 64
   ggggcugggg ccggggccga gc 22
<210> 65
   <211> 22
   <212> RNA
   <213> Homo sapiens
<400> 65
   gcagcagggu gaaacugaca ca 22
<210> 66
   <211> 20
   <212> RNA
   <213> Homo sapiens
<400> 66
   cggcucuggg ucugugggga 20
<210> 67
   <211> 22
   <212> RNA
   <213> Homo sapiens
<400> 67
   gcagagugca aacaauuuug ac 22
<210> 68
   <211> 22
   <212> RNA
   <213> Homo sapiens
<400> 68
   uuugugaccu gguccacuaa cc 22
<210> 69
   <211> 22
   <212> RNA
   <213> Homo sapiens
<400> 69
   cuguugccac uaaccucaac cu 22
<210> 70
   <211> 22
   <212> RNA
   <213> Homo sapiens
<400> 70
   ugcggggcua gggcuaacag ca 22
<210> 71
   <211> 17
   <212> RNA
   <213> Homo sapiens
<400> 71
   ucucgcuggg gccucca 17
<210> 72
   <211> 22
   <212> RNA
   <213> Homo sapiens
<400> 72
   caacaaaucc cagucuaccu aa 22
<210> 73
   <211> 21
   <212> RNA
   <213> Homo sapiens
<400> 73
   cuuccgcccc gccgggcguc g 21
<210> 74
   <211> 22
   <212> RNA
   <213> Homo sapiens
<400> 74
   gggacccagg gagagacgua ag 22
<210> 75
   <211> 22
   <212> RNA
   <213> Homo sapiens
<400> 75
   caacaaauca cagucugcca ua 22
<210> 76
   <211> 22
   <212> RNA
   <213> Homo sapiens
<400> 76
   caacuagacu gugagcuucu ag 22
<210> 77
   <211> 23
   <212> RNA
   <213> Homo sapiens
<400> 77
   aaggagcuua caaucuagcu ggg 23
<210> 78
   <211> 23
   <212> RNA
   <213> Homo sapiens
<400> 78
   uggaagacua gugauuuugu ugu 23
<210> 79
   <211> 20
   <212> RNA
   <213> Homo sapiens
<400> 79
   cuguaugccc ucaccgcuca 20
<210> 80
   <211> 23
   <212> RNA
   <213> Homo sapiens
<400> 80
   uggugcggag agggcccaca gug 23
<210> 81
   <211> 23
   <212> RNA
   <213> Homo sapiens
<400> 81
   aggaagcccu ggaggggcug gag 23
<210> 82
   <211> 21
   <212> RNA
   <213> Homo sapiens
<400> 82
   uccgguucuc agggcuccac c 21
<210> 83
   <211> 20
   <212> RNA
   <213> Homo sapiens
<400> 83
   gucccugagu guauguggug 20
<210> 84
   <211> 23
   <212> RNA
   <213> Homo sapiens
<400> 84
   ugucacucgg cucggcccac uac 23
<210> 85
   <211> 20
   <212> RNA
   <213> Homo sapiens
<400> 85
   accaggaggc ugaggccccu 20
<210> 86
   <211> 24
   <212> RNA
   <213> Homo sapiens
<400> 86
   acuggcuagg gaaaaugauu ggau 24
<210> 87
   <211> 23
   <212> RNA
   <213> Homo sapiens
<400> 87
   uauucauuua uccccagccu aca 23
<210> 88
   <211> 22
   <212> RNA
   <213> Homo sapiens
<400> 88
   gguggcccgg ccgugccuga gg 22
<210> 89
   <211> 22
   <212> RNA
   <213> Homo sapiens
<400> 89
   aggcggggcg ccgcgggacc gc 22
<210> 90
   <211> 21
   <212> RNA
   <213> Homo sapiens
<400> 90
   ucccacguug uggcccagca g 21
<210> 91
   <211> 24
   <212> RNA
   <213> Homo sapiens
<400> 91
   ugccuggguc ucuggccugc gcgu 24
<210> 92
   <211> 22
   <212> RNA
   <213> Homo sapiens
<400> 92
   uacccauugc auaucggagu ug 22
<210> 93
   <211> 22
   <212> RNA
   <213> Homo sapiens
<400> 93
   cuugguucag ggaggguccc ca 22
<210> 94
   <211> 25
   <212> RNA
   <213> Homo sapiens
<400> 94
   ggcggaggga aguagguccg uuggu 25
<210> 95
   <211> 23
   <212> RNA
   <213> Homo sapiens
<400> 95
   ggcagguucu cacccucucu agg 23
<210> 96
   <211> 21
   <212> RNA
   <213> Homo sapiens
<400> 96
   aauauuauac agucaaccuc u 21
<210> 97
   <211> 22
   <212> RNA
   <213> Homo sapiens
<400> 97
   auaauacaug guuaaccucu uu 22
<210> 98
   <211> 22
   <212> RNA
   <213> Homo sapiens
<400> 98
   uggugggccg cagaacaugu gc 22
<210> 99
   <211> 22
   <212> RNA
   <213> Homo sapiens
<400> 99
   uaugucugcu gaccaucacc uu 22
<210> 100
   <211> 21
   <212> RNA
   <213> Homo sapiens
<400> 100
   guguugaaac aaucucuacu g 21
<210> 101
   <211> 21
   <212> RNA
   <213> Homo sapiens
<400> 101
   aauggcgcca cuaggguugu g 21
<210> 102
   <211> 22
   <212> RNA
   <213> Homo sapiens
<400> 102
   uuuaggauaa gcuugacuuu ug 22
<210> 103
   <211> 21
   <212> RNA
   <213> Homo sapiens
<400> 103
   aggaggcagc gcucucagga c 21
<210> 104
   <211> 22
   <212> RNA
   <213> Homo sapiens
<400> 104
   aaaccugugu uguucaagag uc 22
<210> 105
   <211> 19
   <212> RNA
   <213> Homo sapiens
<400> 105
   aagugugcag ggcacuggu 19
<210> 106
   <211> 21
   <212> RNA
   <213> Homo sapiens
<400> 106
   guggcugcac ucacuuccuu c 21
<210> 107
   <211> 19
   <212> RNA
   <213> Homo sapiens
<400> 107
   aagcagcugc cucugaggc 19
<210> 108
   <211> 19
   <212> RNA
   <213> Homo sapiens
<400> 108
   ucuaggcugg uacugcuga 19
<210> 109
   <211> 19
   <212> RNA
   <213> Homo sapiens
<400> 109
   aguguggcuu ucuuagagc 19
<210> 110
   <211> 22
   <212> RNA
   <213> Homo sapiens
<400> 110
   acuuguaugc uagcucaggu ag 22
<210> 111
   <211> 22
   <212> RNA
   <213> Homo sapiens
<400> 111
   gucccucucc aaaugugucu ug 22
<210> 112
   <211> 24
   <212> RNA
   <213> Homo sapiens
<400> 112
   aaagacauag gauagaguca ccuc 24
<210> 113
   <211> 21
   <212> RNA
   <213> Homo sapiens
<400> 113
   augauccagg aaccugccuc u 21
<210> 114
   <211> 23
   <212> RNA
   <213> Homo sapiens
<400> 114
   aucgcugcgg uugcgagcgc ugu 23
<210> 115
   <211> 25
   <212> RNA
   <213> Homo sapiens
<400> 115
   agggaucgcg ggcggguggc ggccu 25
<210> 116
   <211> 24
   <212> RNA
   <213> Homo sapiens
<400> 116
   acugggggcu uucgggcucu gcgu 24
<210> 117
   <211> 23
   <212> RNA
   <213> Homo sapiens
<400> 117
   ugugcuugcu cgucccgccc gca 23
<210> 118
   <211> 23
   <212> RNA
   <213> Homo sapiens
<400> 118
   acuugggcac ugaaacaaug ucc 23
<210> 119
   <211> 22
   <212> RNA
   <213> Homo sapiens
<400> 119
   aaccagcacc ccaacuuugg ac 22
<210> 120
   <211> 23
   <212> RNA
   <213> Homo sapiens
<400> 120
   cuaauaguau cuaccacaau aaa 23
<210> 121
   <211> 19
   <212> RNA
   <213> Homo sapiens
<400> 121
   gugucugcuu ccuguggga 19
<210> 122
   <211> 21
   <212> RNA
   <213> Homo sapiens
<400> 122
   agaccuggcc cagaccucag c 21
<210> 123
   <211> 22
   <212> RNA
   <213> Homo sapiens
<400> 123
   aguauucugu accagggaag gu 22
<210> 124
   <211> 22
   <212> RNA
   <213> Homo sapiens
<400> 124
   guucucccaa cguaagccca gc 22
<210> 125
   <211> 21
   <212> RNA
   <213> Homo sapiens
<400> 125
   uggguuuacg uugggagaac u 21
<210> 126
   <211> 22
   <212> RNA
   <213> Homo sapiens
<400> 126
   augcugacau auuuacuaga gg 22
<210> 127
   <211> 21
   <212> RNA
   <213> Homo sapiens
<400> 127
   ucuaguaaga guggcagucg a 21
<210> 128
   <211> 22
   <212> RNA
   <213> Homo sapiens
<400> 128
   gugagucucu aagaaaagag ga 22
<210> 129
   <211> 19
   <212> RNA
   <213> Homo sapiens
<400> 129
   agcugucuga aaaugucuu 19
<210> 130
   <211> 22
   <212> RNA
   <213> Homo sapiens
<400> 130
   gacuauagaa cuuucccccu ca 22
<210> 131
   <211> 21
   <212> RNA
   <213> Homo sapiens
<400> 131
   agggggaaag uucuauaguc c 21
<210> 132
   <211> 22
   <212> RNA
   <213> Homo sapiens
<400> 132
   uaguaccagu accuuguguu ca 22
<210> 133
   <211> 21
   <212> RNA
   <213> Homo sapiens
<400> 133
   cacaagguau ugguauuacc u 21
<210> 134
   <211> 23
   <212> RNA
   <213> Homo sapiens
<400> 134
   aucccuugca ggggcuguug ggu 23
<210> 135
   <211> 21
   <212> RNA
   <213> Homo sapiens
<400> 135
   acagucugcu gagguuggag c 21
<210> 136
   <211> 21
   <212> RNA
   <213> Homo sapiens
<400> 136
   ggcuagcaac agcgcuuacc u 21
<210> 137
   <211> 20
   <212> RNA
   <213> Homo sapiens
<400> 137
   auggagauag auauagaaau 20
<210> 138
   <211> 24
   <212> RNA
   <213> Homo sapiens
<400> 138
   gaccuggaca uguuugugcc cagu 24
<210> 139
   <211> 23
   <212> RNA
   <213> Homo sapiens
<400> 139
   aaacucuacu uguccuucug agu 23
<210> 140
   <211> 22
   <212> RNA
   <213> Homo sapiens
<400> 140
   agacuuccca uuugaaggug gc 22
<210> 141
   <211> 22
   <212> RNA
   <213> Homo sapiens
<400> 141
   acucaaaacc cuucagugac uu 22
<210> 142
   <211> 22
   <212> RNA
   <213> Homo sapiens
<400> 142
   agucauugga ggguuugagc ag 22
<210> 143
   <211> 22
   <212> RNA
   <213> Homo sapiens
<400> 143
   gggggucccc ggugcucgga uc 22
<210> 144
   <211> 22
   <212> RNA
   <213> Homo sapiens
<400> 144
   uccgagccug ggucucccuc uu 22
<210> 145
   <211> 23
   <212> RNA
   <213> Homo sapiens
<400> 145
   gaacgccugu ucuugccagg ugg 23
<210> 146
   <211> 20
   <212> RNA
   <213> Homo sapiens
<400> 146
   aggaauguuc cuucuuugcc 20
<210> 147
   <211> 25
   <212> RNA
   <213> Homo sapiens
<400> 147
   gcugggcagg gcuucugagc uccuu 25
<210> 148
   <211> 23
   <212> RNA
   <213> Homo sapiens
<400> 148
   gcgaggaccc cucggggucu gac 23
<210> 149
   <211> 21
   <212> RNA
   <213> Homo sapiens
<400> 149
   ugagcuaaau gugugcuggg a 21
<210> 150
   <211> 20
   <212> RNA
   <213> Homo sapiens
<400> 150
   aggguguuuc ucucaucucu 20
<210> 151
   <211> 25
   <212> RNA
   <213> Homo sapiens
<400> 151
   agggguggug uugggacagc uccgu 25
<210> 152
   <211> 21
   <212> RNA
   <213> Homo sapiens
<400> 152
   guucaaaucc agaucuauaa c 21
<210> 153
   <211> 21
   <212> RNA
   <213> Homo sapiens
<400> 153
   aaacuacuga aaaucaaaga u 21
<210> 154
   <211> 23
   <212> RNA
   <213> Homo sapiens
<400> 154
   uaaaucccau ggugccuucu ccu 23
<210> 155
   <211> 19
   <212> RNA
   <213> Homo sapiens
<400> 155
   aggcugcgga auucaggac 19
<210> 156
   <211> 22
   <212> RNA
   <213> Homo sapiens
<400> 156
   cacacacugc aauuacuuuu gc 22
<210> 157
   <211> 23
   <212> RNA
   <213> Homo sapiens
<400> 157
   gacacgggcg acagcugcgg ccc 23
<210> 158
   <211> 22
   <212> RNA
   <213> Homo sapiens
<400> 158
   uggucuagga uuguuggagg ag 22
<210> 159
   <211> 23
   <212> RNA
   <213> Homo sapiens
<400> 159
   acuuacagac aagagccuug cuc 23
<210> 160
   <211> 20
   <212> RNA
   <213> Homo sapiens
<400> 160
   guugugucag uuuaucaaac 20
<210> 161
   <211> 22
   <212> RNA
   <213> Homo sapiens
<400> 161
   uacgucaucg uugucaucgu ca 22
<210> 162
   <211> 22
   <212> RNA
   <213> Homo sapiens
<400> 162
   ugugucacuc gaugaccacu gu 22
<210> 163
   <211> 21
   <212> RNA
   <213> Homo sapiens
<400> 163
   aagccugccc ggcuccucgg g 21
<210> 164
   <211> 21
   <212> RNA
   <213> Homo sapiens
<400> 164
   gaagugugcc gugguguguc u 21
<210> 165
   <211> 25
   <212> RNA
   <213> Homo sapiens
<400> 165
   aggcaccagc caggcauugc ucagc 25
<210> 166
   <211> 19
   <212> RNA
   <213> Homo sapiens
<400> 166
   ugucucugcu gggguuucu 19
<210> 167
   <211> 22
   <212> RNA
   <213> Homo sapiens
<400> 167
   uugugucaau augcgaugau gu 22
<210> 168
   <211> 20
   <212> RNA
   <213> Homo sapiens
<400> 168
   agaccauggg uucucauugu 20
<210> 169
   <211> 22
   <212> RNA
   <213> Homo sapiens
<400> 169
   gagcuuauuc auaaaagugc ag 22
<210> 170
   <211> 21
   <212> RNA
   <213> Homo sapiens
<400> 170
   uaauuuuaug uauaagcuag u 21
<210> 171
   <211> 24
   <212> RNA
   <213> Homo sapiens
<400> 171
   ucagaacaaa ugccgguucc caga 24
<210> 172
   <211> 22
   <212> RNA
   <213> Homo sapiens
<400> 172
   ugagaaccac gucugcucug ag 22
<210> 173
   <211> 21
   <212> RNA
   <213> Homo sapiens
<400> 173
   uuggccacaa uggguuagaa c 21
<210> 174
   <211> 21
   <212> RNA
   <213> Homo sapiens
<400> 174
   uuuccauagg ugaugaguca c 21
<210> 175
   <211> 22
   <212> RNA
   <213> Homo sapiens
<400> 175
   uaugcauugu auuuuuaggu cc 22
<210> 176
   <211> 19
   <212> RNA
   <213> Homo sapiens
<400> 176
   ugggcguauc uguaugcua 19
<210> 177
   <211> 22
   <212> RNA
   <213> Homo sapiens
<400> 177
   uuaugguuug ccugggacug ag 22
<210> 178
   <211> 21
   <212> RNA
   <213> Homo sapiens
<400> 178
   caaagaggaa ggucccauua c 21
<210> 179
   <211> 23
   <212> RNA
   <213> Homo sapiens
<400> 179
   uuacaguugu ucaaccaguu acu 23
<210> 180
   <211> 22
   <212> RNA
   <213> Homo sapiens
<400> 180
   uaacugguug aacaacugaa cc 22
<210> 181
   <211> 21
   <212> RNA
   <213> Homo sapiens
<400> 181
   ucuuguguuc ucuagaucag u 21
<210> 182
   <211> 22
   <212> RNA
   <213> Homo sapiens
<400> 182
   uugagaauga ugaaucauua gg 22
<210> 183
   <211> 23
   <212> RNA
   <213> Homo sapiens
<400> 183
   uucauuuggu auaaaccgcg auu 23
<210> 184
   <211> 21
   <212> RNA
   <213> Homo sapiens
<400> 184
   cuucuugugc ucuaggauug u 21
<210> 185
   <211> 21
   <212> RNA
   <213> Homo sapiens
<400> 185
   uagauaaaau auugguaccu g 21
<210> 186
   <211> 22
   <212> RNA
   <213> Homo sapiens
<400> 186
   auucuaauuu cuccacgucu uu 22
<210> 187
   <211> 22
   <212> RNA
   <213> Homo sapiens
<400> 187
   aagaugugga aaaauuggaa uc 22
<210> 188
   <211> 19
   <212> RNA
   <213> Homo sapiens
<400> 188
   gagccaguug gacaggagc 19
<210> 189
   <211> 23
   <212> RNA
   <213> Homo sapiens
<400> 189
   ugagugugug ugugugagug ugu 23
<210> 190
   <211> 22
   <212> RNA
   <213> Homo sapiens
<400> 190
   cacgcucaug cacacaccca ca 22
<210> 191
   <211> 24
   <212> RNA
   <213> Homo sapiens
<400> 191
   cugaagugau guguaacuga ucag 24
<210> 192
   <211> 20
   <212> RNA
   <213> Homo sapiens
<400> 192
   guccgcucgg cgguggccca 20
<210> 193
   <211> 21
   <212> RNA
   <213> Homo sapiens
<400> 193
   ugaguuggcc aucugaguga g 21
<210> 194
   <211> 22
   <212> RNA
   <213> Homo sapiens
<400> 194
   cgaaaacagc aauuaccuuu gc 22
<210> 195
   <211> 21
   <212> RNA
   <213> Homo sapiens
<400> 195
   aguuaaugaa uccuggaaag u 21
<210> 196
   <211> 20
   <212> RNA
   <213> Homo sapiens
<400> 196
   auguauaaau guauacacac 20
<210> 197
   <211> 23
   <212> RNA
   <213> Homo sapiens
<400> 197
   aguauguucu uccaggacag aac 23
<210> 198
   <211> 19
   <212> RNA
   <213> Homo sapiens
<400> 198
   gggcgccugu gaucccaac 19
<210> 199
   <211> 19
   <212> RNA
   <213> Homo sapiens
<400> 199
   aggcacggug ucagcaggc 19
<210> 200
   <211> 19
   <212> RNA
   <213> Homo sapiens
<400> 200
   agguugacau acguuuccc 19
<210> 201
   <211> 20
   <212> RNA
   <213> Homo sapiens
<400> 201
   aaaguagcug uaccauuugc 20
<210> 202
   <211> 22
   <212> RNA
   <213> Homo sapiens
<400> 202
   caaaguuuaa gauccuugaa gu 22
<210> 203
   <211> 21
   <212> RNA
   <213> Homo sapiens
<400> 203
   uaaaguaaau augcaccaaa a 21
<210> 204
   <211> 19
   <212> RNA
   <213> Homo sapiens
<400> 204
   ugagcugcug uaccaaaau 19
<210> 205
   <211> 23
   <212> RNA
   <213> Homo sapiens
<400> 205
   guuugcacgg gugggccuug ucu 23
<210> 206
   <211> 22
   <212> RNA
   <213> Homo sapiens
<400> 206
   gaugagcuca uuguaauaug ag 22
<210> 207
   <211> 22
   <212> RNA
   <213> Homo sapiens
<400> 207
   auauuaccau uagcucaucu uu 22
<210> 208
   <211> 21
   <212> RNA
   <213> Homo sapiens
<400> 208
   aggguaagcu gaaccucuga u 21
<210> 209
   <211> 21
   <212> RNA
   <213> Homo sapiens
<400> 209
   gcuaguccug acucagccag u 21
<210> 210
   <211> 21
   <212> RNA
   <213> Homo sapiens
<400> 210
   aaaacgguga gauuuuguuu u 21
<210> 211
   <211> 21
   <212> RNA
   <213> Homo sapiens
<400> 211
   aacaggugac ugguuagaca a 21
<210> 212
   <211> 22
   <212> RNA
   <213> Homo sapiens
<400> 212
   gaaaucaagc gugggugaga cc 22
<210> 213
   <211> 21
   <212> RNA
   <213> Homo sapiens
<400> 213
   gcgacccaua cuugguuuca g 21
<210> 214
   <211> 21
   <212> RNA
   <213> Homo sapiens
<400> 214
   gcgacccacu cuugguuucc a 21
<210> 215
   <211> 22
   <212> RNA
   <213> Homo sapiens
<400> 215
   ugucuuacuc ccucaggcac au 22
<210> 216
   <211> 23
   <212> RNA
   <213> Homo sapiens
<400> 216
   agugccugag ggaguaagag ccc 23
<210> 217
   <211> 21
   <212> RNA
   <213> Homo sapiens
<400> 217
   ugacaacuau ggaugagcuc u 21
<210> 218
   <211> 22
   <212> RNA
   <213> Homo sapiens
<400> 218
   gcuggugcaa aaguaauggc gg 22
<210> 219
   <211> 22
   <212> RNA
   <213> Homo sapiens
<400> 219
   uagcaaaaac ugcaguuacu uu 22
<210> 220
   <211> 22
   <212> RNA
   <213> Homo sapiens
<400> 220
   ccaaaacugc aguuacuuuu gc 22
<210> 221
   <211> 22
   <212> RNA
   <213> Homo sapiens
<400> 221
   caaaaguaau uguggauuuu gu 22
<210> 222
   <211> 21
   <212> RNA
   <213> Homo sapiens
<400> 222
   caaagguauu ugugguuuuu g 21
<210> 223
   <211> 22
   <212> RNA
   <213> Homo sapiens
<400> 223
   aaaaguauuu gcggguuuug uc 22
<210> 224
   <211> 22
   <212> RNA
   <213> Homo sapiens
<400> 224
   aaaaguacuu gcggauuuug cu 22
<210> 225
   <211> 22
   <212> RNA
   <213> Homo sapiens
<400> 225
   aaaaguaauu gcggucuuug gu 22
<210> 226
   <211> 22
   <212> RNA
   <213> Homo sapiens
<400> 226
   aaaaguaauu gcggauuuug cc 22
<210> 227
   <211> 22
   <212> RNA
   <213> Homo sapiens
<400> 227
   aaaaguaauc gcgguuuuug uc 22
<210> 228
   <211> 22
   <212> RNA
   <213> Homo sapiens
<400> 228
   aaaacuguaa uuacuuuugu ac 22
<210> 229
   <211> 19
   <212> RNA
   <213> Homo sapiens
<400> 229
   aaaaacugua auuacuuuu 19
<210> 230
   <211> 22
   <212> RNA
   <213> Homo sapiens
<400> 230
   aaaaacugag acuacuuuug ca 22
<210> 231
   <211> 22
   <212> RNA
   <213> Homo sapiens
<400> 231
   aaaaguaauu gugguuuuug cc 22
<210> 232
   <211> 22
   <212> RNA
   <213> Homo sapiens
<400> 232
   caaaaaccac aguuucuuuu gc 22
<210> 233
   <211> 22
   <212> RNA
   <213> Homo sapiens
<400> 233
   aaaaguaauu gcgguuuuug cc 22
<210> 234
   <211> 22
   <212> RNA
   <213> Homo sapiens
<400> 234
   caaaaaucuc aauuacuuuu gc 22
<210> 235
   <211> 22
   <212> RNA
   <213> Homo sapiens
<400> 235
   aaaaguaauu gugguuuugg cc 22
<210> 236
   <211> 22
   <212> RNA
   <213> Homo sapiens
<400> 236
   caagaaccuc aguugcuuuu gu 22
<210> 237
   <211> 22
   <212> RNA
   <213> Homo sapiens
<400> 237
   aaaaguaauu gcgaguuuua cc 22
<210> 238
   <211> 22
   <212> RNA
   <213> Homo sapiens
<400> 238
   caaaacuggc aauuacuuuu gc 22
<210> 239
   <211> 22
   <212> RNA
   <213> Homo sapiens
<400> 239
   ucaguaaaug uuuauuagau ga 22
<210> 240
   <211> 22
   <212> RNA
   <213> Homo sapiens
<400> 240
   ucagcaaaca uuuauugugu gc 22
<210> 241
   <211> 22
   <212> RNA
   <213> Homo sapiens
<400> 241
   auucugcauu uuuagcaagu uc 22
<210> 242
   <211> 22
   <212> RNA
   <213> Homo sapiens
<400> 242
   aaacauucgc ggugcacuuc uu 22
<210> 243
   <211> 23
   <212> RNA
   <213> Homo sapiens
<400> 243
   ucggggauca ucaugucacg aga 23
<210> 244
   <211> 22
   <212> RNA
   <213> Homo sapiens
<400> 244
   ugugacagau ugauaacuga aa 22
<210> 245
   <211> 25
   <212> RNA
   <213> Homo sapiens
<400> 245
   aaaggauucu gcugucgguc ccacu 25
<210> 246
   <211> 22
   <212> RNA
   <213> Homo sapiens
<400> 246
   uggugggcac agaaucugga cu 22
<210> 247
   <211> 22
   <212> RNA
   <213> Homo sapiens
<400> 247
   ggagaaauua uccuuggugu gu 22
<210> 248
   <211> 22
   <212> RNA
   <213> Homo sapiens
<400> 248
   caugccuuga guguaggacc gu 22
<210> 249
   <211> 22
   <212> RNA
   <213> Homo sapiens
<400> 249
   ccucccacac ccaaggcuug ca 22
<210> 250
   <211> 20
   <212> RNA
   <213> Homo sapiens
<400> 250
   cugcaaaggg aagcccuuuc 20
<210> 251
   <211> 22
   <212> RNA
   <213> Homo sapiens
<400> 251
   gaaagugcuu ccuuuuagag gc 22
<210> 252
   <211> 23
   <212> RNA
   <213> Homo sapiens
<400> 252
   cucuugaggg aagcacuuuc ugu 23
<210> 253
   <211> 22
   <212> RNA
   <213> Homo sapiens
<400> 253
   cucuagaggg aagcacuuuc ug 22
<210> 254
   <211> 21
   <212> RNA
   <213> Homo sapiens
<400> 254
   cuccagaggg augcacuuuc u 21
<210> 255
   <211> 22
   <212> RNA
   <213> Homo sapiens
<400> 255
   gaaggcgcuu cccuuuagag cg 22
<210> 256
   <211> 22
   <212> RNA
   <213> Homo sapiens
<400> 256
   cuacaaaggg aagcacuuuc uc 22
<210> 257
   <211> 21
   <212> RNA
   <213> Homo sapiens
<400> 257
   gaaggcgcuu cccuuuggag u 21
<210> 258
   <211> 22
   <212> RNA
   <213> Homo sapiens
<400> 258
   cucuagaggg aagcgcuuuc ug 22
<210> 259
   <211> 23
   <212> RNA
   <213> Homo sapiens
<400> 259
   gaacgcgcuu cccuauagag ggu 23
<210> 260
   <211> 22
   <212> RNA
   <213> Homo sapiens
<400> 260
   cucuagaggg aagcgcuuuc ug 22
<210> 261
   <211> 22
   <212> RNA
   <213> Homo sapiens
<400> 261
   aaaaugguuc ccuuuagagu gu 22
<210> 262
   <211> 22
   <212> RNA
   <213> Homo sapiens
<400> 262
   aacgcacuuc ccuuuagagu gu 22
<210> 263
   <211> 22
   <212> RNA
   <213> Homo sapiens
<400> 263
   acaaagugcu ucccuuuaga gu 22
<210> 264
   <211> 24
   <212> RNA
   <213> Homo sapiens
<400> 264
   acaaagugcu ucccuuuaga gugu 24
<210> 265
   <211> 22
   <212> RNA
   <213> Homo sapiens
<400> 265
   aagugcuucc uuuuagaggg uu 22
<210> 266
   <211> 21
   <212> RNA
   <213> Homo sapiens
<400> 266
   aaagugcuuc cuuuuugagg g 21
<210> 267
   <211> 20
   <212> RNA
   <213> Homo sapiens
<400> 267
   cuacaaaggg aagcccuuuc 20
<210> 268
   <211> 22
   <212> RNA
   <213> Homo sapiens
<400> 268
   aaagugcuuc ucuuuggugg gu 22
<210> 269
   <211> 22
   <212> RNA
   <213> Homo sapiens
<400> 269
   cucuagaggg aagcacuuuc ug 22
<210> 270
   <211> 22
   <212> RNA
   <213> Homo sapiens
<400> 270
   aaagugcuuc cuuuuagagg gu 22
<210> 271
   <211> 21
   <212> RNA
   <213> Homo sapiens
<400> 271
   aaagugcuuc cuuuuagagg g 21
<210> 272
   <211> 21
   <212> RNA
   <213> Homo sapiens
<400> 272
   cuccagaggg aaguacuuuc u 21
<210> 273
   <211> 22
   <212> RNA
   <213> Homo sapiens
<400> 273
   aaagugcuuc ccuuuggacu gu 22
<210> 274
   <211> 22
   <212> RNA
   <213> Homo sapiens
<400> 274
   uucuccaaaa gggagcacuu uc 22
<210> 275
   <211> 22
   <212> RNA
   <213> Homo sapiens
<400> 275
   aagugccucc uuuuagagug uu 22
<210> 276
   <211> 22
   <212> RNA
   <213> Homo sapiens
<400> 276
   caaagugccu cccuuuagag ug 22
<210> 277
   <211> 22
   <212> RNA
   <213> Homo sapiens
<400> 277
   cucuagaggg aagcgcuuuc ug 22
<210> 278
   <211> 22
   <212> RNA
   <213> Homo sapiens
<400> 278
   aaagugcauc uuuuuagagg au 22
<210> 279
   <211> 22
   <212> RNA
   <213> Homo sapiens
<400> 279
   cucuagaggg aagcgcuuuc ug 22
<210> 280
   <211> 22
   <212> RNA
   <213> Homo sapiens
<400> 280
   aaagugcauc cuuuuagagg uu 22
<210> 281
   <211> 22
   <212> RNA
   <213> Homo sapiens
<400> 281
   cucuagaggg aagcgcuuuc ug 22
<210> 282
   <211> 22
   <212> RNA
   <213> Homo sapiens
<400> 282
   aaagugcauc cuuuuagagu gu 22
<210> 283
   <211> 22
   <212> RNA
   <213> Homo sapiens
<400> 283
   cucuagaggg aagcacuuuc uc 22
<210> 284
   <211> 21
   <212> RNA
   <213> Homo sapiens
<400> 284
   gaaagcgcuu cucuuuagag g 21
<210> 285
   <211> 22
   <212> RNA
   <213> Homo sapiens
<400> 285
   cucuagaggg aagcgcuuuc ug 22
<210> 286
   <211> 21
   <212> RNA
   <213> Homo sapiens
<400> 286
   aaagcgcuuc ccuucagagu g 21
<210> 287
   <211> 22
   <212> RNA
   <213> Homo sapiens
<400> 287
   cucuagaggg aagcacuuuc ug 22
<210> 288
   <211> 21
   <212> RNA
   <213> Homo sapiens
<400> 288
   caaagcgcuu cccuuuggag c 21
<210> 289
   <211> 23
   <212> RNA
   <213> Homo sapiens
<400> 289
   ucucuggagg gaagcacuuu cug 23
<210> 290
   <211> 23
   <212> RNA
   <213> Homo sapiens
<400> 290
   caaagcgcuu cucuuuagag ugu 23
<210> 291
   <211> 22
   <212> RNA
   <213> Homo sapiens
<400> 291
   caaagcgcuc cccuuuagag gu 22
<210> 292
   <211> 20
   <212> RNA
   <213> Homo sapiens
<400> 292
   cugcaaaggg aagcccuuuc 20
<210> 293
   <211> 22
   <212> RNA
   <213> Homo sapiens
<400> 293
   gaaagcgcuu cccuuugcug ga 22
<210> 294
   <211> 22
   <212> RNA
   <213> Homo sapiens
<400> 294
   aucgugcauc cuuuuagagu gu 22
<210> 295
   <211> 22
   <212> RNA
   <213> Homo sapiens
<400> 295
   ucgugcaucc cuuuagagug uu 22
<210> 296
   <211> 22
   <212> RNA
   <213> Homo sapiens
<400> 296
   aucgugcauc ccuuuagagu gu 22
<210> 297
   <211> 22
   <212> RNA
   <213> Homo sapiens
<400> 297
   ccucuagaug gaagcacugu cu 22
<210> 298
   <211> 18
   <212> RNA
   <213> Homo sapiens
<400> 298
   ugcuuccuuu cagagggu 18
<210> 299
   <211> 22
   <212> RNA
   <213> Homo sapiens
<400> 299
   aucuggaggu aagaagcacu uu 22
<210> 300
   <211> 23
   <212> RNA
   <213> Homo sapiens
<400> 300
   uucucgagga aagaagcacu uuc 23
<210> 301
   <211> 18
   <212> RNA
   <213> Homo sapiens
<400> 301
   ugcuuccuuu cagagggu 18
<210> 302
   <211> 24
   <212> RNA
   <213> Homo sapiens
<400> 302
   uucuccaaaa gaaagcacuu ucug 24
<210> 303
   <211> 22
   <212> RNA
   <213> Homo sapiens
<400> 303
   gagugccuuc uuuuggagcg uu 22
<210> 304
   <211> 21
   <212> RNA
   <213> Homo sapiens
<400> 304
   auugacacuu cugugaguag a 21
<210> 305
   <211> 22
   <212> RNA
   <213> Homo sapiens
<400> 305
   uucucaagga ggugucguuu au 22
<210> 306
   <211> 22
   <212> RNA
   <213> Homo sapiens
<400> 306
   uucacaagga ggugucauuu au 22
<210> 307
   <211> 18
   <212> RNA
   <213> Homo sapiens
<400> 307
   uucacaggga ggugucau 18
<210> 308
   <211> 23
   <212> RNA
   <213> Homo sapiens
<400> 308
   uaaauuucac cuuucugaga agg 23
<210> 309
   <211> 23
   <212> RNA
   <213> Homo sapiens
<400> 309
   cacucagccu ugagggcacu uuc 23
<210> 310
   <211> 22
   <212> RNA
   <213> Homo sapiens
<400> 310
   aagugcuguc auagcugagg uc 22
<210> 311
   <211> 21
   <212> RNA
   <213> Homo sapiens
<400> 311
   gugucuuuug cucugcaguc a 21
<210> 312
   <211> 22
   <212> RNA
   <213> Homo sapiens
<400> 312
   uacucaggag aguggcaauc ac 22
<210> 313
   <211> 21
   <212> RNA
   <213> Homo sapiens
<400> 313
   uacugcagac aguggcaauc a 21
<210> 314
   <211> 22
   <212> RNA
   <213> Homo sapiens
<400> 314
   ugauugguac gucugugggu ag 22
<210> 315
   <211> 22
   <212> RNA
   <213> Homo sapiens
<400> 315
   uacugcagac guggcaauca ug 22
<210> 316
   <211> 23
   <212> RNA
   <213> Homo sapiens
<400> 316
   uacuccagag ggcgucacuc aug 23
<210> 317
   <211> 23
   <212> RNA
   <213> Homo sapiens
<400> 317
   ugauuguagc cuuuuggagu aga 23
<210> 318
   <211> 21
   <212> RNA
   <213> Homo sapiens
<400> 318
   uuuugcaccu uuuggaguga a 21
<210> 319
   <211> 21
   <212> RNA
   <213> Homo sapiens
<400> 319
   uaaggcaccc uucugaguag a 21
<210> 320
   <211> 22
   <212> RNA
   <213> Homo sapiens
<400> 320
   gggagccagg aaguauugau gu 22
<210> 321
   <211> 22
   <212> RNA
   <213> Homo sapiens
<400> 321
   cgucaacacu ugcugguuuc cu 22
<210> 322
   <211> 22
   <212> RNA
   <213> Homo sapiens
<400> 322
   agacccuggu cugcacucua uc 22
<210> 323
   <211> 23
   <212> RNA
   <213> Homo sapiens
<400> 323
   uagcagcggg aacaguucug cag 23
<210> 324
   <211> 21
   <212> RNA
   <213> Homo sapiens
<400> 324
   auccuugcua ucugggugcu a 21
<210> 325
   <211> 22
   <212> RNA
   <213> Homo sapiens
<400> 325
   aaugcaccug ggcaaggauu ca 22
<210> 326
   <211> 22
   <212> RNA
   <213> Homo sapiens
<400> 326
   aauccuuugu cccuggguga ga 22
<210> 327
   <211> 22
   <212> RNA
   <213> Homo sapiens
<400> 327
   aaugcacccg ggcaaggauu cu 22
<210> 328
   <211> 22
   <212> RNA
   <213> Homo sapiens
<400> 328
   augcaccugg gcaaggauuc ug 22
<210> 329
   <211> 23
   <212> RNA
   <213> Homo sapiens
<400> 329
   uaauccuugc uaccugggug aga 23
<210> 330
   <211> 21
   <212> RNA
   <213> Homo sapiens
<400> 330
   uuaagacuug cagugauguu u 21
<210> 331
   <211> 22
   <212> RNA
   <213> Homo sapiens
<400> 331
   aacaucacag caagucugug cu 22
<210> 332
   <211> 23
   <212> RNA
   <213> Homo sapiens
<400> 332
   uuucaagcca gggggcguuu uuc 23
<210> 333
   <211> 22
   <212> RNA
   <213> Homo sapiens
<400> 333
   caaaccacac ugugguguua ga 22
<210> 334
   <211> 21
   <212> RNA
   <213> Homo sapiens
<400> 334
   cagcagcaca cugugguuug u 21
<210> 335
   <211> 22
   <212> RNA
   <213> Homo sapiens
<400> 335
   ugaguauuac auggccaauc uc 22
<210> 336
   <211> 22
   <212> RNA
   <213> Homo sapiens
<400> 336
   aaacaaacau ggugcacuuc uu 22
<210> 337
   <211> 22
   <212> RNA
   <213> Homo sapiens
<400> 337
   ugaaacauac acgggaaacc uc 22
<210> 338
   <211> 22
   <212> RNA
   <213> Homo sapiens
<400> 338
   uuguacaugg uaggcuuuca uu 22
<210> 339
   <211> 22
   <212> RNA
   <213> Homo sapiens
<400> 339
   ugaaggucua cugugugcca gg 22
<210> 340
   <211> 23
   <212> RNA
   <213> Homo sapiens
<400> 340
   aggaccugcg ggacaagauu cuu 23
<210> 341
   <211> 22
   <212> RNA
   <213> Homo sapiens
<400> 341
   aguggggaac ccuuccauga gg 22
<210> 342
   <211> 22
   <212> RNA
   <213> Homo sapiens
<400> 342
   cuuaugcaag auucccuucu ac 22
<210> 343
   <211> 20
   <212> RNA
   <213> Homo sapiens
<400> 343
   ccauggaucu ccaggugggu 20
<210> 344
   <211> 22
   <212> RNA
   <213> Homo sapiens
<400> 344
   caaccuggag gacuccaugc ug 22
<210> 345
   <211> 22
   <212> RNA
   <213> Homo sapiens
<400> 345
   gugacaucac auauacggca gc 22
<210> 346
   <211> 21
   <212> RNA
   <213> Homo sapiens
<400> 346
   cccagauaau ggcacucuca a 21
<210> 347
   <211> 21
   <212> RNA
   <213> Homo sapiens
<400> 347
   uugaaaggcu auuucuuggu c 21
<210> 348
   <211> 22
   <212> RNA
   <213> Homo sapiens
<400> 348
   aaucguacag ggucauccac uu 22
<210> 349
   <211> 22
   <212> RNA
   <213> Homo sapiens
<400> 349
   aaucauacag ggacauccag uu 22
<210> 350
   <211> 22
   <212> RNA
   <213> Homo sapiens
<400> 350
   uccuguacug agcugccccg ag 22
<210> 351
   <211> 21
   <212> RNA
   <213> Homo sapiens
<400> 351
   cggggcagcu caguacagga u 21
<210> 352
   <211> 22
   <212> RNA
   <213> Homo sapiens
<400> 352
   agaggcuggc cgugaugaau uc 22
<210> 353
   <211> 22
   <212> RNA
   <213> Homo sapiens
<400> 353
   gucauacacg gcucuccucu cu 22
<210> 354
   <211> 22
   <212> RNA
   <213> Homo sapiens
<400> 354
   ucaggcucag uccccucccg au 22
<210> 355
   <211> 22
   <212> RNA
   <213> Homo sapiens
<400> 355
   aagacgggag gaaagaaggg ag 22
<210> 356
   <211> 21
   <212> RNA
   <213> Homo sapiens
<400> 356
   ucacuccucu ccucccgucu u 21
<210> 357
   <211> 22
   <212> RNA
   <213> Homo sapiens
<400> 357
   uaugugccuu uggacuacau cg 22
<210> 358
   <211> 21
   <212> RNA
   <213> Homo sapiens
<400> 358
   gcaguccaug ggcauauaca c 21
<210> 359
   <211> 22
   <212> RNA
   <213> Homo sapiens
<400> 359
   acccuaucaa uauugucucu gc 22
<210> 360
   <211> 23
   <212> RNA
   <213> Homo sapiens
<400> 360
   uagugcaaua uugcuuauag ggu 23
<210> 361
   <211> 23
   <212> RNA
   <213> Homo sapiens
<400> 361
   agguuguccg uggugaguuc gca 23
<210> 362
   <211> 22
   <212> RNA
   <213> Homo sapiens
<400> 362
   cucaucugca aagaaguaag ug 22
<210> 363
   <211> 22
   <212> RNA
   <213> Homo sapiens
<400> 363
   aacuguuugc agaggaaacu ga 22
<210> 364
   <211> 22
   <212> RNA
   <213> Homo sapiens
<400> 364
   aaaccguuac cauuacugag uu 22
<210> 365
   <211> 22
   <212> RNA
   <213> Homo sapiens
<400> 365
   uuuugcaaua uguuccugaa ua 22
<210> 366
   <211> 22
   <212> RNA
   <213> Homo sapiens
<400> 366
   uugggaucau uuugcaucca ua 22
<210> 367
   <211> 22
   <212> RNA
   <213> Homo sapiens
<400> 367
   uuuugcgaug uguuccuaau au 22
<210> 368
   <211> 23
   <212> RNA
   <213> Homo sapiens
<400> 368
   uugcuaguug cacuccucuc ugu 23
<210> 369
   <211> 25
   <212> RNA
   <213> Homo sapiens
<400> 369
   uaggcagugu auugcuagcg gcugu 25
<210> 370
   <211> 22
   <212> RNA
   <213> Homo sapiens
<400> 370
   cagccacaac uacccugcca cu 22
<210> 371
   <211> 22
   <212> RNA
   <213> Homo sapiens
<400> 371
   aggcagugua uuguuagcug gc 22
<210> 372
   <211> 22
   <212> RNA
   <213> Homo sapiens
<400> 372
   uggcagugua uuguuagcug gu 22
<210> 373
   <211> 22
   <212> RNA
   <213> Homo sapiens
<400> 373
   uugcauaugu aggauguccc au 22
<210> 374
   <211> 22
   <212> RNA
   <213> Homo sapiens
<400> 374
   aucaugaugg gcuccucggu gu 22
<210> 375
   <211> 21
   <212> RNA
   <213> Homo sapiens
<400> 375
   cuggauggcu ccuccauguc u 21
<210> 376
   <211> 23
   <212> RNA
   <213> Homo sapiens
<400> 376
   ucuuggagua ggucauuggg ugg 23
<210> 377
   <211> 22
   <212> RNA
   <213> Homo sapiens
<400> 377
   caggucgucu ugcagggcuu cu 22
<210> 378
   <211> 21
   <212> RNA
   <213> Homo sapiens
<400> 378
   ugucuugcag gccgucaugc a 21
<210> 379
   <211> 22
   <212> RNA
   <213> Homo sapiens
<400> 379
   uaauacuguc ugguaaaacc gu 22
<210> 380
   <211> 22
   <212> RNA
   <213> Homo sapiens
<400> 380
   aucgggaaug ucguguccgc cc 22
<210> 381
   <211> 23
   <212> RNA
   <213> Homo sapiens
<400> 381
   aaugacacga ucacucccgu uga 23
<210> 382
   <211> 21
   <212> RNA
   <213> Homo sapiens
<400> 382
   caaaacguga ggcgcugcua u 21
<210> 383
   <211> 22
   <212> RNA
   <213> Homo sapiens
<400> 383
   cagcagcaau ucauguuuug aa 22
<210> 384
   <211> 23
   <212> RNA
   <213> Homo sapiens
<400> 384
   ugaggggcag agagcgagac uuu 23
<210> 385
   <211> 23
   <212> RNA
   <213> Homo sapiens
<400> 385
   agcucggucu gaggccccuc agu 23
<210> 386
   <211> 22
   <212> RNA
   <213> Homo sapiens
<400> 386
   acuggacuua gggucagaag gc 22
<210> 387
   <211> 23
   <212> RNA
   <213> Homo sapiens
<400> 387
   aucaacagac auuaauuggg cgc 23
<210> 388
   <211> 23
   <212> RNA
   <213> Homo sapiens
<400> 388
   acuucaccug guccacuagc cgu 23
<210> 389
   <211> 22
   <212> RNA
   <213> Homo sapiens
<400> 389
   uauguaacac gguccacuaa cc 22
<210> 390
   <211> 21
   <212> RNA
   <213> Homo sapiens
<400> 390
   uaguagaccg uauagcguac g 21
<210> 391
   <211> 21
   <212> RNA
   <213> Homo sapiens
<400> 391
   aauauaacac agauggccug u 21
<210> 392
   <211> 23
   <212> RNA
   <213> Homo sapiens
<400> 392
   agguuacccg agcaacuuug cau 23
<210> 393
   <211> 22
   <212> RNA
   <213> Homo sapiens
<400> 393
   gaauguugcu cggugaaccc cu 22
<210> 394
   <211> 20
   <212> RNA
   <213> Homo sapiens
<400> 394
   auuccuagaa auuguucaua 20
<210> 395
   <211> 22
   <212> RNA
   <213> Homo sapiens
<400> 395
   agaucagaag gugauugugg cu 22
<210> 396
   <211> 22
   <212> RNA
   <213> Homo sapiens
<400> 396
   gaaguuguuc gugguggauu cg 22
<210> 397
   <211> 22
   <212> RNA
   <213> Homo sapiens
<400> 397
   uauacaaggg caagcucucu gu 22
<210> 398
   <211> 22
   <212> RNA
   <213> Homo sapiens
<400> 398
   ugguugacca uagaacaugc gc 22
<210> 399
   <211> 22
   <212> RNA
   <213> Homo sapiens
<400> 399
   uauguaauau gguccacauc uu 22
<210> 400
   <211> 22
   <212> RNA
   <213> Homo sapiens
<400> 400
   uauguaacau gguccacuaa cu 22
<210> 401
   <211> 21
   <212> RNA
   <213> Homo sapiens
<400> 401
   ugguagacua uggaacguag g 21
<210> 402
   <211> 22
   <212> RNA
   <213> Homo sapiens
<400> 402
   cuccugacuc cagguccugu gu 22
<210> 403
   <211> 21
   <212> RNA
   <213> Homo sapiens
<400> 403
   acuggacuug gagucagaag g 21
<210> 404
   <211> 22
   <212> RNA
   <213> Homo sapiens
<400> 404
   agagguugcc cuuggugaau uc 22
<210> 405
   <211> 22
   <212> RNA
   <213> Homo sapiens
<400> 405
   aucacacaaa ggcaacuuuu gu 22
<210> 406
   <211> 21
   <212> RNA
   <213> Homo sapiens
<400> 406
   aacauagagg aaauuccacg u 21
<210> 407
   <211> 22
   <212> RNA
   <213> Homo sapiens
<400> 407
   aucauagagg aaaauccaug uu 22
<210> 408
   <211> 22
   <212> RNA
   <213> Homo sapiens
<400> 408
   guagauucuc cuucuaugag ua 22
<210> 409
   <211> 21
   <212> RNA
   <213> Homo sapiens
<400> 409
   aucauagagg aaaauccacg u 21
<210> 410
   <211> 22
   <212> RNA
   <213> Homo sapiens
<400> 410
   uuuguucguu cggcucgcgu ga 22
<210> 411
   <211> 22
   <212> RNA
   <213> Homo sapiens
<400> 411
   cuuagcaggu uguauuauca uu 22
<210> 412
   <211> 22
   <212> RNA
   <213> Homo sapiens
<400> 412
   auauaauaca accugcuaag ug 22
<210> 413
   <211> 22
   <212> RNA
   <213> Homo sapiens
<400> 413
   cuuaucagau uguauuguaa uu 22
<210> 414
   <211> 22
   <212> RNA
   <213> Homo sapiens
<400> 414
   uuauaauaca accugauaag ug 22
<210> 415
   <211> 22
   <212> RNA
   <213> Homo sapiens
<400> 415
   acucaaaaug ggggcgcuuu cc 22
<210> 416
   <211> 23
   <212> RNA
   <213> Homo sapiens
<400> 416
   gaagugcuuc gauuuugggg ugu 23
<210> 417
   <211> 23
   <212> RNA
   <213> Homo sapiens
<400> 417
   aaagugcugc gacauuugag cgu 23
<210> 418
   <211> 20
   <212> RNA
   <213> Homo sapiens
<400> 418
   acucaaacug ugggggcacu 20
<210> 419
   <211> 23
   <212> RNA
   <213> Homo sapiens
<400> 419
   aagugccgcc aucuuuugag ugu 23
<210> 420
   <211> 22
   <212> RNA
   <213> Homo sapiens
<400> 420
   gccugcuggg guggaaccug gu 22
<210> 421
   <211> 22
   <212> RNA
   <213> Homo sapiens
<400> 421
   agaucgaccg uguuauauuc gc 22
<210> 422
   <211> 21
   <212> RNA
   <213> Homo sapiens
<400> 422
   aauaauacau gguugaucuu u 21
<210> 423
   <211> 22
   <212> RNA
   <213> Homo sapiens
<400> 423
   acuguugcua auaugcaacu cu 22
<210> 424
   <211> 22
   <212> RNA
   <213> Homo sapiens
<400> 424
   aauugcacuu uagcaauggu ga 22
<210> 425
   <211> 22
   <212> RNA
   <213> Homo sapiens
<400> 425
   agggacuuuc aggggcagcu gu 22
<210> 426
   <211> 22
   <212> RNA
   <213> Homo sapiens
<400> 426
   uaaugccccu aaaaauccuu au 22
<210> 427
   <211> 22
   <212> RNA
   <213> Homo sapiens
<400> 427
   cggguggauc acgaugcaau uu 22
<210> 428
   <211> 22
   <212> RNA
   <213> Homo sapiens
<400> 428
   aauugcacgg uauccaucug ua 22
<210> 429
   <211> 24
   <212> RNA
   <213> Homo sapiens
<400> 429
   aauccuugga accuaggugu gagu 24
<210> 430
   <211> 22
   <212> RNA
   <213> Homo sapiens
<400> 430
   aacacaccua uucaaggauu ca 22
<210> 431
   <211> 22
   <212> RNA
   <213> Homo sapiens
<400> 431
   uuaucagaau cuccaggggu ac 22
<210> 432
   <211> 23
   <212> RNA
   <213> Homo sapiens
<400> 432
   ucccccaggu gugauucuga uuu 23
<210> 433
   <211> 23
   <212> RNA
   <213> Homo sapiens
<400> 433
   aggcagugua guuagcugau ugc 23
<210> 434
   <211> 22
   <212> RNA
   <213> Homo sapiens
<400> 434
   aaucacuaac cacacggcca gg 22
<210> 435
   <211> 23
   <212> RNA
   <213> Homo sapiens
<400> 435
   uaggcagugu cauuagcuga uug 23
<210> 436
   <211> 22
   <212> RNA
   <213> Homo sapiens
<400> 436
   caaucacuaa cuccacugcc au 22
<210> 437
   <211> 22
   <212> RNA
   <213> Homo sapiens
<400> 437
   caaucagcaa guauacugcc cu 22
<210> 438
   <211> 22
   <212> RNA
   <213> Homo sapiens
<400> 438
   uggcaguguc uuagcugguu gu 22
<210> 439
   <211> 23
   <212> RNA
   <213> Homo sapiens
<400> 439
   ugucugcccg caugccugcc ucu 23
<210> 440
   <211> 22
   <212> RNA
   <213> Homo sapiens
<400> 440
   gcugacuccu aguccagggc uc 22
<210> 441
   <211> 21
   <212> RNA
   <213> Homo sapiens
<400> 441
   aggggugcua ucugugauug a 21
<210> 442
   <211> 23
   <212> RNA
   <213> Homo sapiens
<400> 442
   ucucacacag aaaucgcacc cgu 23
<210> 443
   <211> 22
   <212> RNA
   <213> Homo sapiens
<400> 443
   uccgucucag uuacuuuaua gc 22
<210> 444
   <211> 22
   <212> RNA
   <213> Homo sapiens
<400> 444
   uuauaaagca augagacuga uu 22
<210> 445
   <211> 22
   <212> RNA
   <213> Homo sapiens
<400> 445
   cagugccucg gcagugcagc cc 22
<210> 446
   <211> 20
   <212> RNA
   <213> Homo sapiens
<400> 446
   gugcauugcu guugcauugc 20
<210> 447
   <211> 22
   <212> RNA
   <213> Homo sapiens
<400> 447
   caauguuucc acagugcauc ac 22
<210> 448
   <211> 21
   <212> RNA
   <213> Homo sapiens
<400> 448
   gugcauugua guugcauugc a 21
<210> 449
   <211> 23
   <212> RNA
   <213> Homo sapiens
<400> 449
   ucccuguccu ccaggagcuc acg 23
<210> 450
   <211> 23
   <212> RNA
   <213> Homo sapiens
<400> 450
   ugagcgccuc gacgacagag ccg 23
<210> 451
   <211> 22
   <212> RNA
   <213> Homo sapiens
<400> 451
   aacaauaucc uggugcugag ug 22
<210> 452
   <211> 22
   <212> RNA
   <213> Homo sapiens
<400> 452
   uccagcauca gugauuuugu ug 22
<210> 453
   <211> 21
   <212> RNA
   <213> Homo sapiens
<400> 453
   gaacggcuuc auacaggagu u 21
<210> 454
   <211> 22
   <212> RNA
   <213> Homo sapiens
<400> 454
   cuccuauaug augccuuucu uc 22
<210> 455
   <211> 22
   <212> RNA
   <213> Homo sapiens
<400> 455
   uuuuucauua uugcuccuga cc 22
<210> 456
   <211> 23
   <212> RNA
   <213> Homo sapiens
<400> 456
   ucaagagcaa uaacgaaaaa ugu 23
<210> 457
   <211> 22
   <212> RNA
   <213> Homo sapiens
<400> 457
   cuagguaugg ucccagggau cc 22
<210> 458
   <211> 21
   <212> RNA
   <213> Homo sapiens
<400> 458
   gccccugggc cuauccuaga a 21
<210> 459
   <211> 22
   <212> RNA
   <213> Homo sapiens
<400> 459
   ucucugggcc ugugucuuag gc 22
<210> 460
   <211> 23
   <212> RNA
   <213> Homo sapiens
<400> 460
   gcaaagcaca cggccugcag aga 23
<210> 461
   <211> 22
   <212> RNA
   <213> Homo sapiens
<400> 461
   aacacaccug guuaaccucu uu 22
<210> 462
   <211> 22
   <212> RNA
   <213> Homo sapiens
<400> 462
   cuggcccucu cugcccuucc gu 22
<210> 463
   <211> 20
   <212> RNA
   <213> Homo sapiens
<400> 463
   ccucugggcc cuuccuccag 20
<210> 464
   <211> 23
   <212> RNA
   <213> Homo sapiens
<400> 464
   ccuaguaggu guccaguaag ugu 23
<210> 465
   <211> 23
   <212> RNA
   <213> Homo sapiens
<400> 465
   cgcauccccu agggcauugg ugu 23
<210> 466
   <211> 20
   <212> RNA
   <213> Homo sapiens
<400> 466
   acugccccag gugcugcugg 20
<210> 467
   <211> 22
   <212> RNA
   <213> Homo sapiens
<400> 467
   aggugguccg uggcgcguuc gc 22
<210> 468
   <211> 21
   <212> RNA
   <213> Homo sapiens
<400> 468
   cacauuacac ggucgaccuc u 21
<210> 469
   <211> 19
   <212> RNA
   <213> Homo sapiens
<400> 469
   aaaagcuggg uugagagga 19
<210> 470
   <211> 20
   <212> RNA
   <213> Homo sapiens
<400> 470
   aaaagcuggg uugagagggu 20
<210> 471
   <211> 22
   <212> RNA
   <213> Homo sapiens
<400> 471
   aaaagcuggg uugagagggc aa 22
<210> 472
   <211> 22
   <212> RNA
   <213> Homo sapiens
<400> 472
   aaaagcuggg uugagagggc ga 22
<210> 473
   <211> 22
   <212> RNA
   <213> Homo sapiens
<400> 473
   caauuuagug ugugugauau uu 22
<210> 474
   <211> 22
   <212> RNA
   <213> Homo sapiens
<400> 474
   uauugcacau uacuaaguug ca 22
<210> 475
   <211> 22
   <212> RNA
   <213> Homo sapiens
<400> 475
   ugcuaugcca acauauugcc au 22
<210> 476
   <211> 21
   <212> RNA
   <213> Homo sapiens
<400> 476
   aggcaagaug cuggcauagc u 21
<210> 477
   <211> 22
   <212> RNA
   <213> Homo sapiens
<400> 477
   cuuucagucg gauguuuaca gc 22
<210> 478
   <211> 22
   <212> RNA
   <213> Homo sapiens
<400> 478
   uguaaacauc cuugacugga ag 22
<210> 479
   <211> 22
   <212> RNA
   <213> Homo sapiens
<400> 479
   cuuucaguca gauguuugcu gc 22
<210> 480
   <211> 22
   <212> RNA
   <213> Homo sapiens
<400> 480
   uguaaacauc cccgacugga ag 22
<210> 481
   <211> 22
   <212> RNA
   <213> Homo sapiens
<400> 481
   cugggagaag gcuguuuacu cu 22
<210> 482
   <211> 22
   <212> RNA
   <213> Homo sapiens
<400> 482
   cugggagagg guuguuuacu cc 22
<210> 483
   <211> 23
   <212> RNA
   <213> Homo sapiens
<400> 483
   uguaaacauc cuacacucuc age 23
<210> 484
   <211> 22
   <212> RNA
   <213> Homo sapiens
<400> 484
   cugggaggug gauguuuacu uc 22
<210> 485
   <211> 22
   <212> RNA
   <213> Homo sapiens
<400> 485
   uguaaacauc cuacacucag cu 22
<210> 486
   <211> 22
   <212> RNA
   <213> Homo sapiens
<400> 486
   cuuucagucg gauguuugca gc 22
<210> 487
   <211> 22
   <212> RNA
   <213> Homo sapiens
<400> 487
   uguaaacauc cucgacugga ag 22
<210> 488
   <211> 17
   <212> RNA
   <213> Homo sapiens
<400> 488
   uaauugcuuc cauguuu 17
<210> 489
   <211> 17
   <212> RNA
   <213> Homo sapiens
<400> 489
   uaagugcuuc caugcuu 17
<210> 490
   <211> 22
   <212> RNA
   <213> Homo sapiens
<400> 490
   acuuuaacau ggaggcacuu gc 22
<210> 491
   <211> 23
   <212> RNA
   <213> Homo sapiens
<400> 491
   uaagugcuuc cauguuugag ugu 23
<210> 492
   <211> 22
   <212> RNA
   <213> Homo sapiens
<400> 492
   uuuaacaugg ggguaccugc ug 22
<210> 493
   <211> 23
   <212> RNA
   <213> Homo sapiens
<400> 493
   uaagugcuuc cauguuucag ugg 23
<210> 494
   <211> 22
   <212> RNA
   <213> Homo sapiens
<400> 494
   acuuuaacau ggaagugcuu uc 22
<210> 495
   <211> 23
   <212> RNA
   <213> Homo sapiens
<400> 495
   uaagugcuuc cauguuuuag uag 23
<210> 496
   <211> 23
   <212> RNA
   <213> Homo sapiens
<400> 496
   acuuaaacgu ggauguacuu gcu 23
<210> 497
   <211> 23
   <212> RNA
   <213> Homo sapiens
<400> 497
   uaagugcuuc cauguuuugg uga 23
<210> 498
   <211> 23
   <212> RNA
   <213> Homo sapiens
<400> 498
   cagugcaaug auauugucaa age 23
<210> 499
   <211> 23
   <212> RNA
   <213> Homo sapiens
<400> 499
   cagugcaaua guauugucaa agc 23
<210> 500
   <211> 22
   <212> RNA
   <213> Homo sapiens
<400> 500
   uauacaaggg cagacucucu cu 22
<210> 501
   <211> 22
   <212> RNA
   <213> Homo sapiens
<400> 501
   ugaccgauuu cuccuggugu uc 22
<210> 502
   <211> 22
   <212> RNA
   <213> Homo sapiens
<400> 502
   uagcaccauu ugaaaucggu ua 22
<210> 503
   <211> 22
   <212> RNA
   <213> Homo sapiens
<400> 503
   cugguuucac augguggcuu ag 22
<210> 504
   <211> 24
   <212> RNA
   <213> Homo sapiens
<400> 504
   gcugguuuca uauggugguu uaga 24
<210> 505
   <211> 23
   <212> RNA
   <213> Homo sapiens
<400> 505
   uagcaccauu ugaaaucagu guu 23
<210> 506
   <211> 22
   <212> RNA
   <213> Homo sapiens
<400> 506
   acugauuucu uuugguguuc ag 22
<210> 507
   <211> 22
   <212> RNA
   <213> Homo sapiens
<400> 507
   uagcaccauc ugaaaucggu ua 22
<210> 508
   <211> 22
   <212> RNA
   <213> Homo sapiens
<400> 508
   ugguuuaccg ucccacauac au 22
<210> 509
   <211> 22
   <212> RNA
   <213> Homo sapiens
<400> 509
   uaugugggau gguaaaccgc uu 22
<210> 510
   <211> 24
   <212> RNA
   <213> Homo sapiens
<400> 510
   agcagaagca gggagguucu ccca 24
<210> 511
   <211> 21
   <212> RNA
   <213> Homo sapiens
<400> 511
   auguaugugu gcaugugcau g 21
<210> 512
   <211> 21
   <212> RNA
   <213> Homo sapiens
<400> 512
   agggcccccc cucaauccug u 21
<210> 513
   <211> 22
   <212> RNA
   <213> Homo sapiens
<400> 513
   gaggguuggg uggaggcucu cc 22
<210> 514
   <211> 22
   <212> RNA
   <213> Homo sapiens
<400> 514
   aaggagcuca cagucuauug ag 22
<210> 515
   <211> 22
   <212> RNA
   <213> Homo sapiens
<400> 515
   cacuagauug ugagcuccug ga 22
<210> 516
   <211> 22
   <212> RNA
   <213> Homo sapiens
<400> 516
   agagcuuagc ugauugguga ac 22
<210> 517
   <211> 21
   <212> RNA
   <213> Homo sapiens
<400> 517
   uucacagugg cuaaguucug c 21
<210> 518
   <211> 22
   <212> RNA
   <213> Homo sapiens
<400> 518
   agggcuuagc ugcuugugag ca 22
<210> 519
   <211> 21
   <212> RNA
   <213> Homo sapiens
<400> 519
   uucacagugg cuaaguuccg c 21
<210> 520
   <211> 22
   <212> RNA
   <213> Homo sapiens
<400> 520
   ccuguucucc auuacuuggc uc 22
<210> 521
   <211> 21
   <212> RNA
   <213> Homo sapiens
<400> 521
   uucaaguaau ucaggauagg u 21
<210> 522
   <211> 22
   <212> RNA
   <213> Homo sapiens
<400> 522
   ccuauucuug auuacuuguu uc 22
<210> 523
   <211> 22
   <212> RNA
   <213> Homo sapiens
<400> 523
   ccuauucuug guuacuugca cg 22
<210> 524
   <211> 22
   <212> RNA
   <213> Homo sapiens
<400> 524
   uucaaguaau ccaggauagg cu 22
<210> 525
   <211> 21
   <212> RNA
   <213> Homo sapiens
<400> 525
   aggcggagac uugggcaauu g 21
<210> 526
   <211> 22
   <212> RNA
   <213> Homo sapiens
<400> 526
   cauugcacuu gucucggucu ga 22
<210> 527
   <211> 22
   <212> RNA
   <213> Homo sapiens
<400> 527
   ugccuacuga gcugaaacac ag 22
<210> 528
   <211> 22
   <212> RNA
   <213> Homo sapiens
<400> 528
   ugccuacuga gcugauauca gu 22
<210> 529
   <211> 22
   <212> RNA
   <213> Homo sapiens
<400> 529
   uggcucaguu cagcaggaac ag 22
<210> 530
   <211> 22
   <212> RNA
   <213> Homo sapiens
<400> 530
   uggguuccug gcaugcugau uu 22
<210> 531
   <211> 21
   <212> RNA
   <213> Homo sapiens
<400> 531
   aucacauugc cagggauuac c 21
<210> 532
   <211> 22
   <212> RNA
   <213> Homo sapiens
<400> 532
   gggguuccug gggaugggau uu 22
<210> 533
   <211> 21
   <212> RNA
   <213> Homo sapiens
<400> 533
   aucacauugc cagggauuuc c 21
<210> 534
   <211> 22
   <212> RNA
   <213> Homo sapiens
<400> 534
   gagagcagug uguguugccu gg 22
<210> 535
   <211> 21
   <212> RNA
   <213> Homo sapiens
<400> 535
   ugacagcgcc cugccuggcu c 21
<210> 536
   <211> 22
   <212> RNA
   <213> Homo sapiens
<400> 536
   ucugcaagug ucagaggcga gg 22
<210> 537
   <211> 23
   <212> RNA
   <213> Homo sapiens
<400> 537
   aaaauggugc ccuagugacu aca 23
<210> 538
   <211> 21
   <212> RNA
   <213> Homo sapiens
<400> 538
   caagucacua gugguuccgu u 21
<210> 539
   <211> 22
   <212> RNA
   <213> Homo sapiens
<400> 539
   cguguauuug acaagcugag uu 22
<210> 540
   <211> 22
   <212> RNA
   <213> Homo sapiens
<400> 540
   ugucaguuug ucaaauaccc ca 22
<210> 541
   <211> 22
   <212> RNA
   <213> Homo sapiens
<400> 541
   cucaguagcc aguguagauc cu 22
<210> 542
   <211> 21
   <212> RNA
   <213> Homo sapiens
<400> 542
   agcuacaucu ggcuacuggg u 21
<210> 543
   <211> 22
   <212> RNA
   <213> Homo sapiens
<400> 543
   accuggcaua caauguagau uu 22
<210> 544
   <211> 23
   <212> RNA
   <213> Homo sapiens
<400> 544
   agcuacauug ucugcugggu uuc 23
<210> 545
   <211> 22
   <212> RNA
   <213> Homo sapiens
<400> 545
   acacagggcu guugugaaga cu 22
<210> 546
   <211> 21
   <212> RNA
   <213> Homo sapiens
<400> 546
   ccaccaccgu gucugacacu u 21
<210> 547
   <211> 21
   <212> RNA
   <213> Homo sapiens
<400> 547
   ccacaccgua ucugacacuu u 21
<210> 548
   <211> 22
   <212> RNA
   <213> Homo sapiens
<400> 548
   aguucuucag uggcaagcuu ua 22
<210> 549
   <211> 22
   <212> RNA
   <213> Homo sapiens
<400> 549
   aagcugccag uugaagaacu gu 22
<210> 550
   <211> 21
   <212> RNA
   <213> Homo sapiens
<400> 550
   ugauugucca aacgcaauuc u 21
<210> 551
   <211> 22
   <212> RNA
   <213> Homo sapiens
<400> 551
   agaauugugg cuggacaucu gu 22
<210> 552
   <211> 22
   <212> RNA
   <213> Homo sapiens
<400> 552
   agaguugagu cuggacgucc cg 22
<210> 553
   <211> 22
   <212> RNA
   <213> Homo sapiens
<400> 553
   caugguucug ucaagcaccg cg 22
<210> 554
   <211> 22
   <212> RNA
   <213> Homo sapiens
<400> 554
   augguuccgu caagcaccau gg 22
<210> 555
   <211> 21
   <212> RNA
   <213> Homo sapiens
<400> 555
   uugugcuuga ucuaaccaug u 21
<210> 556
   <211> 23
   <212> RNA
   <213> Homo sapiens
<400> 556
   uacugcauca ggaacugauu gga 23
<210> 557
   <211> 22
   <212> RNA
   <213> Homo sapiens
<400> 557
   aaaucucugc aggcaaaugu ga 22
<210> 558
   <211> 22
   <212> RNA
   <213> Homo sapiens
<400> 558
   uaaucucagc uggcaacugu ga 22
<210> 559
   <211> 21
   <212> RNA
   <213> Homo sapiens
<400> 559
   augaccuaug aauugacaga c 21
<210> 560
   <211> 22
   <212> RNA
   <213> Homo sapiens
<400> 560
   ugccugucua cacuugcugu gc 22
<210> 561
   <211> 22
   <212> RNA
   <213> Homo sapiens
<400> 561
   acagcaggca cagacaggca gu 22
<210> 562
   <211> 21
   <212> RNA
   <213> Homo sapiens
<400> 562
   uaacagucuc cagucacggc c 21
<210> 563
   <211> 21
   <212> RNA
   <213> Homo sapiens
<400> 563
   uguucucuuu gccaaggaca g 21
<210> 564
   <211> 21
   <212> RNA
   <213> Homo sapiens
<400> 564
   ccucccaugc caagaacucc c 21
<210> 565
   <211> 21
   <212> RNA
   <213> Homo sapiens
<400> 565
   gguucuuagc auaggagguc u 21
<210> 566
   <211> 22
   <212> RNA
   <213> Homo sapiens
<400> 566
   caucagaauu cauggaggcu ag 22
<210> 567
   <211> 22
   <212> RNA
   <213> Homo sapiens
<400> 567
   agcuuccaug acuccugaug ga 22
<210> 568
   <211> 21
   <212> RNA
   <213> Homo sapiens
<400> 568
   cgagccucaa gcaagggacu u 21
<210> 569
   <211> 22
   <212> RNA
   <213> Homo sapiens
<400> 569
   uagucccuuc cuugaagcgg uc 22
<210> 570
   <211> 21
   <212> RNA
   <213> Homo sapiens
<400> 570
   auuugugcuu ggcucuguca c 21
<210> 571
   <211> 22
   <212> RNA
   <213> Homo sapiens
<400> 571
   uuggggaaac ggccgcugag ug 22
<210> 572
   <211> 22
   <212> RNA
   <213> Homo sapiens
<400> 572
   uucccuuugu cauccuucgc cu 22
<210> 573
   <211> 22
   <212> RNA
   <213> Homo sapiens
<400> 573
   cugugcgugu gacagcggcu ga 22
<210> 574
   <211> 21
   <212> RNA
   <213> Homo sapiens
<400> 574
   caacaccagu cgaugggcug u 21
<210> 575
   <211> 22
   <212> RNA
   <213> Homo sapiens
<400> 575
   uagcuuauca gacugauguu ga 22
<210> 576
   <211> 22
   <212> RNA
   <213> Homo sapiens
<400> 576
   acuguaguau gggcacuucc ag 22
<210> 577
   <211> 23
   <212> RNA
   <213> Homo sapiens
<400> 577
   caaagugcuc auagugcagg uag 23
<210> 578
   <211> 22
   <212> RNA
   <213> Homo sapiens
<400> 578
   acugcauuau gagcacuuaa ag 22
<210> 579
   <211> 23
   <212> RNA
   <213> Homo sapiens
<400> 579
   uaaagugcuu auagugcagg uag 23
<210> 580
   <211> 22
   <212> RNA
   <213> Homo sapiens
<400> 580
   auaagacgaa caaaagguuu gu 22
<210> 581
   <211> 22
   <212> RNA
   <213> Homo sapiens
<400> 581
   auaagacgag caaaaagcuu gu 22
<210> 582
   <211> 22
   <212> RNA
   <213> Homo sapiens
<400> 582
   uggaauguaa ggaagugugu gg 22
<210> 583
   <211> 23
   <212> RNA
   <213> Homo sapiens
<400> 583
   cuguaauaua aauuuaauuu auu 23
<210> 584
   <211> 23
   <212> RNA
   <213> Homo sapiens
<400> 584
   guguuaauua aaccucuauu uac 23
<210> 585
   <211> 20
   <212> RNA
   <213> Homo sapiens
<400> 585
   uguuuugaua acaguaaugu 20
<210> 586
   <211> 21
   <212> RNA
   <213> Homo sapiens
<400> 586
   gauuucagug gagugaaguu c 21
<210> 587
   <211> 22
   <212> RNA
   <213> Homo sapiens
<400> 587
   uccuucauuc caccggaguc ug 22
<210> 588
   <211> 22
   <212> RNA
   <213> Homo sapiens
<400> 588
   uucccuuugu cauccuaugc cu 22
<210> 589
   <211> 22
   <212> RNA
   <213> Homo sapiens
<400> 589
   gugaaauguu uaggaccacu ag 22
<210> 590
   <211> 22
   <212> RNA
   <213> Homo sapiens
<400> 590
   uuccuaugca uauacuucuu ug 22
<210> 591
   <211> 20
   <212> RNA
   <213> Homo sapiens
<400> 591
   agagguauag ggcaugggaa 20
<210> 592
   <211> 22
   <212> RNA
   <213> Homo sapiens
<400> 592
   cgucuuaccc agcaguguuu gg 22
<210> 593
   <211> 23
   <212> RNA
   <213> Homo sapiens
<400> 593
   uaauacugcc ggguaaugau gga 23
<210> 594
   <211> 22
   <212> RNA
   <213> Homo sapiens
<400> 594
   caucuuacug ggcagcauug ga 22
<210> 595
   <211> 22
   <212> RNA
   <213> Homo sapiens
<400> 595
   uaauacugcc ugguaaugau ga 22
<210> 596
   <211> 22
   <212> RNA
   <213> Homo sapiens
<400> 596
   caucuuaccg gacagugcug ga 22
<210> 597
   <211> 22
   <212> RNA
   <213> Homo sapiens
<400> 597
   uaacacuguc ugguaacgau gu 22
<210> 598
   <211> 22
   <212> RNA
   <213> Homo sapiens
<400> 598
   aguuuugcag guuugcauuu ca 22
<210> 599
   <211> 23
   <212> RNA
   <213> Homo sapiens
<400> 599
   aguuuugcag guuugcaucc age 23
<210> 600
   <211> 23
   <212> RNA
   <213> Homo sapiens
<400> 600
   ugugcaaauc caugcaaaac uga 23
<210> 601
   <211> 22
   <212> RNA
   <213> Homo sapiens
<400> 601
   aguuuugcau aguugcacua ca 22
<210> 602
   <211> 23
   <212> RNA
   <213> Homo sapiens
<400> 602
   ugugcaaauc uaugcaaaac uga 23
<210> 603
   <211> 23
   <212> RNA
   <213> Homo sapiens
<400> 603
   cccaguguuu agacuaucug uuc 23
<210> 604
   <211> 22
   <212> RNA
   <213> Homo sapiens
<400> 604
   acaguagucu gcacauuggu ua 22
<210> 605
   <211> 23
   <212> RNA
   <213> Homo sapiens
<400> 605
   cccaguguuc agacuaccug uuc 23
<210> 606
   <211> 22
   <212> RNA
   <213> Homo sapiens
<400> 606
   acaguagucu gcacauuggu ua 22
<210> 607
   <211> 22
   <212> RNA
   <213> Homo sapiens
<400> 607
   gguccagagg ggagauaggu uc 22
<210> 608
   <211> 22
   <212> RNA
   <213> Homo sapiens
<400> 608
   cucccacugc uucacuugac ua 22
<210> 609
   <211> 21
   <212> RNA
   <213> Homo sapiens
<400> 609
   gguuuggucc uagccuuucu a 21
<210> 610
   <211> 22
   <212> RNA
   <213> Homo sapiens
<400> 610
   gauuagggug cuuagcuguu aa 22
<210> 611
   <211> 20
   <212> RNA
   <213> Homo sapiens
<400> 611
   ccuccugccc uccuugcugu 20
<210> 612
   <211> 25
   <212> RNA
   <213> Homo sapiens
<400> 612
   cccccacaac cgcgcuugac uagcu 25
<210> 613
   <211> 23
   <212> RNA
   <213> Homo sapiens
<400> 613
   ugguuguagu ccgugcgaga aua 23
<210> 614
   <211> 19
   <212> RNA
   <213> Homo sapiens
<400> 614
   accgugcaaa gguagcaua 19
<210> 615
   <211> 22
   <212> RNA
   <213> Homo sapiens
<400> 615
   ucaggccagg cacaguggcu ca 22
<210> 616
   <211> 22
   <212> RNA
   <213> Homo sapiens
<400> 616
   uucaccaccu ucuccaccca gc 22
<210> 617
   <211> 22
   <212> RNA
   <213> Homo sapiens
<400> 617
   ucgacagcac gacacugccu uc 22
<210> 618
   <211> 22
   <212> RNA
   <213> Homo sapiens
<400> 618
   uagguaguuu ccuguuguug gg 22
<210> 619
   <211> 22
   <212> RNA
   <213> Homo sapiens
<400> 619
   cggcaacaag aaacugccug ag 22
<210> 620
   <211> 22
   <212> RNA
   <213> Homo sapiens
<400> 620
   uagguaguuu cauguuguug gg 22
<210> 621
   <211> 22
   <212> RNA
   <213> Homo sapiens
<400> 621
   ccaauauugg cugugcugcu cc 22
<210> 622
   <211> 21
   <212> RNA
   <213> Homo sapiens
<400> 622
   uagcagcaca gaaauauugg c 21
<210> 623
   <211> 22
   <212> RNA
   <213> Homo sapiens
<400> 623
   ccaguggggc ugcuguuauc ug 22
<210> 624
   <211> 22
   <212> RNA
   <213> Homo sapiens
<400> 624
   uguaacagca acuccaugug ga 22
<210> 625
   <211> 22
   <212> RNA
   <213> Homo sapiens
<400> 625
   cgggguuuug agggcgagau ga 22
<210> 626
   <211> 22
   <212> RNA
   <213> Homo sapiens
<400> 626
   aacuggcccu caaagucccg cu 22
<210> 627
   <211> 22
   <212> RNA
   <213> Homo sapiens
<400> 627
   ugggucuuug cgggcgagau ga 22
<210> 628
   <211> 22
   <212> RNA
   <213> Homo sapiens
<400> 628
   aacuggccua caaaguccca gu 22
<210> 629
   <211> 22
   <212> RNA
   <213> Homo sapiens
<400> 629
   cugccaauuc cauaggucac ag 22
<210> 630
   <211> 21
   <212> RNA
   <213> Homo sapiens
<400> 630
   cugaccuaug aauugacagc c 21
<210> 631
   <211> 22
   <212> RNA
   <213> Homo sapiens
<400> 631
   accuugccuu gcugcccggg cc 22
<210> 632
   <211> 20
   <212> RNA
   <213> Homo sapiens
<400> 632
   ccccagggcg acgcggcggg 20
<210> 633
   <211> 22
   <212> RNA
   <213> Homo sapiens
<400> 633
   ggaggggucc cgcacuggga gg 22
<210> 634
   <211> 22
   <212> RNA
   <213> Homo sapiens
<400> 634
   cccugugccc ggcccacuuc ug 22
<210> 635
   <211> 22
   <212> RNA
   <213> Homo sapiens
<400> 635
   ucugcccccu ccgcugcugc ca 22
<210> 636
   <211> 22
   <212> RNA
   <213> Homo sapiens
<400> 636
   uacccagagc augcagugug aa 22
<210> 637
   <211> 20
   <212> RNA
   <213> Homo sapiens
<400> 637
   caccaggcau uguggucucc 20
<210> 638
   <211> 23
   <212> RNA
   <213> Homo sapiens
<400> 638
   ugaguaccgc caugucuguu ggg 23
<210> 639
   <211> 21
   <212> RNA
   <213> Homo sapiens
<400> 639
   ccaguccugu gccugccgcc u 21
<210> 640
   <211> 22
   <212> RNA
   <213> Homo sapiens
<400> 640
   gcugcgcuug gauuucgucc cc 22
<210> 641
   <211> 23
   <212> RNA
   <213> Homo sapiens
<400> 641
   caacggaauc ccaaaagcag cug 23
<210> 642
   <211> 21
   <212> RNA
   <213> Homo sapiens
<400> 642
   ugauauguuu gauauugggu u 21
<210> 643
   <211> 21
   <212> RNA
   <213> Homo sapiens
<400> 643
   ugagugccgg ugccugcccu g 21
<210> 644
   <211> 22
   <212> RNA
   <213> Homo sapiens
<400> 644
   cgcaggggcc gggugcucac cg 22
<210> 645
   <211> 21
   <212> RNA
   <213> Homo sapiens
<400> 645
   cggcggggac ggcgauuggu c 21
<210> 646
   <211> 22
   <212> RNA
   <213> Homo sapiens
<400> 646
   ugauauguuu gauauauuag gu 22
<210> 647
   <211> 22
   <212> RNA
   <213> Homo sapiens
<400> 647
   ugcccuaaau gccccuucug gc 22
<210> 648
   <211> 23
   <212> RNA
   <213> Homo sapiens
<400> 648
   uaaggugcau cuagugcagu uag 23
<210> 649
   <211> 23
   <212> RNA
   <213> Homo sapiens
<400> 649
   acugcccuaa gugcuccuuc ugg 23
<210> 650
   <211> 23
   <212> RNA
   <213> Homo sapiens
<400> 650
   uaaggugcau cuagugcaga uag 23
<210> 651
   <211> 21
   <212> RNA
   <213> Homo sapiens
<400> 651
   caucccuugc augguggagg g 21
<210> 652
   <211> 21
   <212> RNA
   <213> Homo sapiens
<400> 652
   cucccacaug caggguuugc a 21
<210> 653
   <211> 22
   <212> RNA
   <213> Homo sapiens
<400> 653
   ggcuacaaca caggacccgg gc 22
<210> 654
   <211> 22
   <212> RNA
   <213> Homo sapiens
<400> 654
   ucgugucuug uguugcagcc gg 22
<210> 655
   <211> 22
   <212> RNA
   <213> Homo sapiens
<400> 655
   gcccaaaggu gaauuuuuug gg 22
<210> 656
   <211> 22
   <212> RNA
   <213> Homo sapiens
<400> 656
   caaagaauuc uccuuuuggg cu 22
<210> 657
   <211> 22
   <212> RNA
   <213> Homo sapiens
<400> 657
   aggggcuggc uuuccucugg uc 22
<210> 658
   <211> 22
   <212> RNA
   <213> Homo sapiens
<400> 658
   uggagagaaa ggcaguuccu ga 22
<210> 659
   <211> 22
   <212> RNA
   <213> Homo sapiens
<400> 659
   uggacggaga acugauaagg gu 22
<210> 660
   <211> 22
   <212> RNA
   <213> Homo sapiens
<400> 660
   gugaauuacc gaagggccau aa 22
<210> 661
   <211> 22
   <212> RNA
   <213> Homo sapiens
<400> 661
   uauggcacug guagaauuca cu 22
<210> 662
   <211> 18
   <212> RNA
   <213> Homo sapiens
<400> 662
   ugaggcagua gauugaau 18
<210> 663
   <211> 27
   <212> RNA
   <213> Homo sapiens
<400> 663
   auugaucauc gacacuucga acgcaau 27
<210> 664
   <211> 18
   <212> RNA
   <213> Homo sapiens
<400> 664
   uccagugccc uccucucc 18
<210> 665
   <211> 21
   <212> RNA
   <213> Homo sapiens
<400> 665
   ugguucuaga cuugccaacu a 21
<210> 666
   <211> 24
   <212> RNA
   <213> Homo sapiens
<400> 666
   uuuggcaaug guagaacuca cacu 24
<210> 667
   <211> 23
   <212> RNA
   <213> Homo sapiens
<400> 667
   aacauucauu guugucggug ggu 23
<210> 668
   <211> 22
   <212> RNA
   <213> Homo sapiens
<400> 668
   aaccaucgac cguugagugg ac 22
<210> 669
   <211> 22
   <212> RNA
   <213> Homo sapiens
<400> 669
   aacauucaac cugucgguga gu 22
<210> 670
   <211> 23
   <212> RNA
   <213> Homo sapiens
<400> 670
   aacauucauu gcugucggug ggu 23
<210> 671
   <211> 22
   <212> RNA
   <213> Homo sapiens
<400> 671
   accacugacc guugacugua cc 22
<210> 672
   <211> 22
   <212> RNA
   <213> Homo sapiens
<400> 672
   accaucgacc guugauugua cc 22
<210> 673
   <211> 23
   <212> RNA
   <213> Homo sapiens
<400> 673
   aacauucaac gcugucggug agu 23
<210> 674
   <211> 22
   <212> RNA
   <213> Homo sapiens
<400> 674
   acugcaguga aggcacuugu ag 22
<210> 675
   <211> 23
   <212> RNA
   <213> Homo sapiens
<400> 675
   caaagugcuu acagugcagg uag 23
<210> 676
   <211> 22
   <212> RNA
   <213> Homo sapiens
<400> 676
   ccaauauuac ugugcugcuu ua 22
<210> 677
   <211> 22
   <212> RNA
   <213> Homo sapiens
<400> 677
   ccaguauuaa cugugcugcu ga 22
<210> 678
   <211> 22
   <212> RNA
   <213> Homo sapiens
<400> 678
   uagcagcacg uaaauauugg cg 22
<210> 679
   <211> 22
   <212> RNA
   <213> Homo sapiens
<400> 679
   cgaaucauua uuugcugcuc ua 22
<210> 680
   <211> 22
   <212> RNA
   <213> Homo sapiens
<400> 680
   uagcagcaca ucaugguuua ca 22
<210> 681
   <211> 22
   <212> RNA
   <213> Homo sapiens
<400> 681
   caggccauau ugugcugccu ca 22
<210> 682
   <211> 22
   <212> RNA
   <213> Homo sapiens
<400> 682
   uagcagcaca uaaugguuug ug 22
<210> 683
   <211> 22
   <212> RNA
   <213> Homo sapiens
<400> 683
   cuccuacaua uuagcauuaa ca 22
<210> 684
   <211> 23
   <212> RNA
   <213> Homo sapiens
<400> 684
   uuaaugcuaa ucgugauagg ggu 23
<210> 685
   <211> 22
   <212> RNA
   <213> Homo sapiens
<400> 685
   aaucauacac gguugaccua uu 22
<210> 686
   <211> 22
   <212> RNA
   <213> Homo sapiens
<400> 686
   uagguuaucc guguugccuu cg 22
<210> 687
   <211> 21
   <212> RNA
   <213> Homo sapiens
<400> 687
   uccugcgcgu cccagaugcc c 21
<210> 688
   <211> 23
   <212> RNA
   <213> Homo sapiens
<400> 688
   cggcccgggc ugcugcuguu ccu 23
<210> 689
   <211> 22
   <212> RNA
   <213> Homo sapiens
<400> 689
   aaaaccgucu aguuacaguu gu 22
<210> 690
   <211> 22
   <212> RNA
   <213> Homo sapiens
<400> 690
   uugcauaguc acaaaaguga uc 22
<210> 691
   <211> 21
   <212> RNA
   <213> Homo sapiens
<400> 691
   ucagugcaug acagaacuug g 21
<210> 692
   <211> 21
   <212> RNA
   <213> Homo sapiens
<400> 692
   ucgaggagcu cacagucuag u 21
<210> 693
   <211> 21
   <212> RNA
   <213> Homo sapiens
<400> 693
   cuagacugaa gcuccuugag g 21
<210> 694
   <211> 22
   <212> RNA
   <213> Homo sapiens
<400> 694
   cugguacagg ccugggggac ag 22
<210> 695
   <211> 22
   <212> RNA
   <213> Homo sapiens
<400> 695
   ucucccaacc cuuguaccag ug 22
<210> 696
   <211> 21
   <212> RNA
   <213> Homo sapiens
<400> 696
   agggagggac gggggcugug c 21
<210> 697
   <211> 23
   <212> RNA
   <213> Homo sapiens
<400> 697
   ucuggcuccg ugucuucacu ccc 23
<210> 698
   <211> 22
   <212> RNA
   <213> Homo sapiens
<400> 698
   aaguucuguu auacacucag gc 22
<210> 699
   <211> 22
   <212> RNA
   <213> Homo sapiens
<400> 699
   ucagugcauc acagaacuuu gu 22
<210> 700
   <211> 22
   <212> RNA
   <213> Homo sapiens
<400> 700
   aaaguucuga gacacuccga cu 22
<210> 701
   <211> 22
   <212> RNA
   <213> Homo sapiens
<400> 701
   ucagugcacu acagaacuuu gu 22
<210> 702
   <211> 22
   <212> RNA
   <213> Homo sapiens
<400> 702
   gugugcggaa augcuucugc ua 22
<210> 703
   <211> 22
   <212> RNA
   <213> Homo sapiens
<400> 703
   gcccgcgugu ggagccaggu gu 22
<210> 704
   <211> 21
   <212> RNA
   <213> Homo sapiens
<400> 704
   gcccuccgcc cgugcacccc g 21
<210> 705
   <211> 20
   <212> RNA
   <213> Homo sapiens
<400> 705
   guguguggaa augcuucugc 20
<210> 706
   <211> 22
   <212> RNA
   <213> Homo sapiens
<400> 706
   ugagaacuga auuccauagg cu 22
<210> 707
   <211> 22
   <212> RNA
   <213> Homo sapiens
<400> 707
   ugcccugugg acucaguucu gg 22
<210> 708
   <211> 22
   <212> RNA
   <213> Homo sapiens
<400> 708
   ccucugaaau ucaguucuuc ag 22
<210> 709
   <211> 22
   <212> RNA
   <213> Homo sapiens
<400> 709
   ugagaacuga auuccauggg uu 22
<210> 710
   <211> 22
   <212> RNA
   <213> Homo sapiens
<400> 710
   cucggcgcgg ggcgcgggcu cc 22
<210> 711
   <211> 21
   <212> RNA
   <213> Homo sapiens
<400> 711
   cuccguuugc cuguuucgcu g 21
<210> 712
   <211> 22
   <212> RNA
   <213> Homo sapiens
<400> 712
   ggauuccugg aaauacuguu cu 22
<210> 713
   <211> 23
   <212> RNA
   <213> Homo sapiens
<400> 713
   guccaguuuu cccaggaauc ccu 23
<210> 714
   <211> 22
   <212> RNA
   <213> Homo sapiens
<400> 714
   ggauaucauc auauacugua ag 22
<210> 715
   <211> 20
   <212> RNA
   <213> Homo sapiens
<400> 715
   uacaguauag augauguacu 20
<210> 716
   <211> 22
   <212> RNA
   <213> Homo sapiens
<400> 716
   ggugcagugc ugcaucucug gu 22
<210> 717
   <211> 21
   <212> RNA
   <213> Homo sapiens
<400> 717
   ugagaugaag cacuguagcu c 21
<210> 718
   <211> 21
   <212> RNA
   <213> Homo sapiens
<400> 718
   cauaaaguag aaagcacuac u 21
<210> 719
   <211> 23
   <212> RNA
   <213> Homo sapiens
<400> 719
   uguaguguuu ccuacuuuau gga 23
<210> 720
   <211> 22
   <212> RNA
   <213> Homo sapiens
<400> 720
   caucuuccag uacaguguug ga 22
<210> 721
   <211> 22
   <212> RNA
   <213> Homo sapiens
<400> 721
   uaacacuguc ugguaaagau gg 22
<210> 722
   <211> 22
   <212> RNA
   <213> Homo sapiens
<400> 722
   cagugguuuu acccuauggu ag 22
<210> 723
   <211> 21
   <212> RNA
   <213> Homo sapiens
<400> 723
   uaccacaggg uagaaccacg g 21
<210> 724
   <211> 22
   <212> RNA
   <213> Homo sapiens
<400> 724
   ucuacagugc acgugucucc ag 22
<210> 725
   <211> 22
   <212> RNA
   <213> Homo sapiens
<400> 725
   ggagacgcgg cccuguugga gu 22
<210> 726
   <211> 22
   <212> RNA
   <213> Homo sapiens
<400> 726
   gcuauuucac gacaccaggg uu 22
<210> 727
   <211> 22
   <212> RNA
   <213> Homo sapiens
<400> 727
   gcuacuucac aacaccaggg cc 22
<210> 728
   <211> 23
   <212> RNA
   <213> Homo sapiens
<400> 728
   agcugguguu gugaaucagg ccg 23
<210> 729
   <211> 23
   <212> RNA
   <213> Homo sapiens
<400> 729
   uuauugcuua agaauacgcg uag 23
<210> 730
   <211> 22
   <212> RNA
   <213> Homo sapiens
<400> 730
   caucaucguc ucaaaugagu cu 22
<210> 731
   <211> 23
   <212> RNA
   <213> Homo sapiens
<400> 731
   acuccauuug uuuugaugau gga 23
<210> 732
   <211> 22
   <212> RNA
   <213> Homo sapiens
<400> 732
   auguagggcu aaaagccaug gg 22
<210> 733
   <211> 23
   <212> RNA
   <213> Homo sapiens
<400> 733
   uauggcuuuu cauuccuaug uga 23
<210> 734
   <211> 22
   <212> RNA
   <213> Homo sapiens
<400> 734
   uauagggauu ggagccgugg cg 22
<210> 735
   <211> 23
   <212> RNA
   <213> Homo sapiens
<400> 735
   uauggcuuuu uauuccuaug uga 23
<210> 736
   <211> 22
   <212> RNA
   <213> Homo sapiens
<400> 736
   ugugacuggu ugaccagagg gg 22
<210> 737
   <211> 22
   <212> RNA
   <213> Homo sapiens
<400> 737
   uuuggucccc uucaaccagc ua 22
<210> 738
   <211> 22
   <212> RNA
   <213> Homo sapiens
<400> 738
   uuuggucccc uucaaccagc ug 22
<210> 739
   <211> 24
   <212> RNA
   <213> Homo sapiens
<400> 739
   ccagacagaa uucuaugcac uuuc 24
<210> 740
   <211> 22
   <212> RNA
   <213> Homo sapiens
<400> 740
   ucaaaacuga ggggcauuuu cu 22
<210> 741
   <211> 19
   <212> RNA
   <213> Homo sapiens
<400> 741
   gaugaugcug cugaugcug 19
<210> 742
   <211> 18
   <212> RNA
   <213> Homo sapiens
<400> 742
   cagggaggug aaugugau 18
<210> 743
   <211> 22
   <212> RNA
   <213> Homo sapiens
<400> 743
   accguggcuu ucgauuguua cu 22
<210> 744
   <211> 22
   <212> RNA
   <213> Homo sapiens
<400> 744
   uaacagucua cagccauggu cg 22
<210> 745
   <211> 21
   <212> RNA
   <213> Homo sapiens
<400> 745
   acucuuuccc uguugcacua c 21
<210> 746
   <211> 22
   <212> RNA
   <213> Homo sapiens
<400> 746
   cagugcaaug augaaagggc au 22
<210> 747
   <211> 22
   <212> RNA
   <213> Homo sapiens
<400> 747
   uucacauugu gcuacugucu gc 22
<210> 748
   <211> 22
   <212> RNA
   <213> Homo sapiens
<400> 748
   cagugcaaug uuaaaagggc au 22
<210> 749
   <211> 18
   <212> RNA
   <213> Homo sapiens
<400> 749
   gcaugggugg uucagugg 18
<210> 750
   <211> 22
   <212> RNA
   <213> Homo sapiens
<400> 750
   acucggcgug gcgucggucg ug 22
<210> 751
   <211> 18
   <212> RNA
   <213> Homo sapiens
<400> 751
   acguuggcuc ugguggug 18
<210> 752
   <211> 22
   <212> RNA
   <213> Homo sapiens
<400> 752
   uuuucaacuc uaaugggaga ga 22
<210> 753
   <211> 22
   <212> RNA
   <213> Homo sapiens
<400> 753
   uuugaggcua cagugagaug ug 22
<210> 754
   <211> 22
   <212> RNA
   <213> Homo sapiens
<400> 754
   uuuagagacg gggucuugcu cu 22
<210> 755
   <211> 21
   <212> RNA
   <213> Homo sapiens
<400> 755
   uugggacaua cuuaugcuaa a 21
<210> 756
   <211> 24
   <212> RNA
   <213> Homo sapiens
<400> 756
   uugcagcugc cugggaguga cuuc 24
<210> 757
   <211> 22
   <212> RNA
   <213> Homo sapiens
<400> 757
   uucuggaauu cugugugagg ga 22
<210> 758
   <211> 22
   <212> RNA
   <213> Homo sapiens
<400> 758
   uucauucggc uguccagaug ua 22
<210> 759
   <211> 17
   <212> RNA
   <213> Homo sapiens
<400> 759
   uucaaguaau ucaggug 17
<210> 760
   <211> 22
   <212> RNA
   <213> Homo sapiens
<400> 760
   uuagggcccu ggcuccaucu cc 22
<210> 761
   <211> 21
   <212> RNA
   <213> Homo sapiens
<400> 761
   cuuuuugcgg ucugggcuug c 21
<210> 762
   <211> 21
   <212> RNA
   <213> Homo sapiens
<400> 762
   uuaggccgca gaucugggug a 21
<210> 763
   <211> 22
   <212> RNA
   <213> Homo sapiens
<400> 763
   ugugagguug gcauuguugu cu 22
<210> 764
   <211> 22
   <212> RNA
   <213> Homo sapiens
<400> 764
   aagcccuuac cccaaaaagc au 22
<210> 765
   <211> 22
   <212> RNA
   <213> Homo sapiens
<400> 765
   uggguggucu ggagauuugu gc 22
<210> 766
   <211> 25
   <212> RNA
   <213> Homo sapiens
<400> 766
   ugggaacggg uuccggcaga cgcug 25
<210> 767
   <211> 24
   <212> RNA
   <213> Homo sapiens
<400> 767
   uggcccugac ugaagaccag cagu 24
<210> 768
   <211> 19
   <212> RNA
   <213> Homo sapiens
<400> 768
   uggauuuuug gaucaggga 19
<210> 769
   <211> 22
   <212> RNA
   <213> Homo sapiens
<400> 769
   aagcccuuac cccaaaaagu au 22
<210> 770
   <211> 23
   <212> RNA
   <213> Homo sapiens
<400> 770
   uggaguccag gaaucugcau uuu 23
<210> 771
   <211> 21
   <212> RNA
   <213> Homo sapiens
<400> 771
   uggacugccc ugaucuggag a 21
<210> 772
   <211> 22
   <212> RNA
   <213> Homo sapiens
<400> 772
   ugcuggauca gugguucgag uc 22
<210> 773
   <211> 21
   <212> RNA
   <213> Homo sapiens
<400> 773
   ugcaggacca agaugagccc u 21
<210> 774
   <211> 22
   <212> RNA
   <213> Homo sapiens
<400> 774
   ucugggcaac aaagugagac cu 22
<210> 775
   <211> 22
   <212> RNA
   <213> Homo sapiens
<400> 775
   ucuauacaga cccuggcuuu uc 22
<210> 776
   <211> 22
   <212> RNA
   <213> Homo sapiens
<400> 776
   ucuacaaagg aaagcgcuuu cu 22
<210> 777
   <211> 20
   <212> RNA
   <213> Homo sapiens
<400> 777
   ucguuugccu uuuucugcuu 20
<210> 778
   <211> 17
   <212> RNA
   <213> Homo sapiens
<400> 778
   ucgccuccuc cucuccc 17
<210> 779
   <211> 17
   <212> RNA
   <213> Homo sapiens
<400> 779
   ucccaccgcu gccaccc 17
<210> 780
   <211> 21
   <212> RNA
   <213> Homo sapiens
<400> 780
   ucacagugaa ccggucucuu u 21
<210> 781
   <211> 17
   <212> RNA
   <213> Homo sapiens
<400> 781
   ucauauugcu ucuuucu 17
<210> 782
   <211> 22
   <212> RNA
   <213> Homo sapiens
<400> 782
   uaguacugug cauaucaucu au 22
<210> 783
   <211> 22
   <212> RNA
   <213> Homo sapiens
<400> 783
   uacguagaua uauauguauu uu 22
<210> 784
   <211> 20
   <212> RNA
   <213> Homo sapiens
<400> 784
   uaaagagccc uguggagaca 20
<210> 785
   <211> 22
   <212> RNA
   <213> Homo sapiens
<400> 785
   cugaagcuca gagggcucug au 22
<210> 786
   <211> 17
   <212> RNA
   <213> Homo sapiens
<400> 786
   gugggggaga ggcuguc 17
<210> 787
   <211> 17
   <212> RNA
   <213> Homo sapiens
<400> 787
   ucccuguucg ggcgcca 17
<210> 788
   <211> 18
   <212> RNA
   <213> Homo sapiens
<400> 788
   gucccuguuc aggcgcca 18
<210> 789
   <211> 22
   <212> RNA
   <213> Homo sapiens
<400> 789
   ucggauccgu cugagcuugg cu 22
<210> 790
   <211> 25
   <212> RNA
   <213> Homo sapiens
<400> 790
   gggcgacaaa gcaagacucu uucuu 25
<210> 791
   <211> 26
   <212> RNA
   <213> Homo sapiens
<400> 791
   gaugaugaug gcagcaaauu cugaaa 26
<210> 792
   <211> 22
   <212> RNA
   <213> Homo sapiens
<400> 792
   cuuggcaccu agcaagcacu ca 22
<210> 793
   <211> 23
   <212> RNA
   <213> Homo sapiens
<400> 793
   cuggagauau ggaagagcug ugu 23
<210> 794
   <211> 22
   <212> RNA
   <213> Homo sapiens
<400> 794
   cuggacugag ccgugcuacu gg 22
<210> 795
   <211> 18
   <212> RNA
   <213> Homo sapiens
<400> 795
   cgggcguggu gguggggg 18
<210> 796
   <211> 21
   <212> RNA
   <213> Homo sapiens
<400> 796
   ccuguugaag uguaaucccc a 21
<210> 797
   <211> 23
   <212> RNA
   <213> Homo sapiens
<400> 797
   ccucagggcu guagaacagg gcu 23
<210> 798
   <211> 22
   <212> RNA
   <213> Homo sapiens
<400> 798
   caggaugugg ucaaguguug uu 22
<210> 799
   <211> 23
   <212> RNA
   <213> Homo sapiens
<400> 799
   caagucuuau uugagcaccu guu 23
<210> 800
   <211> 22
   <212> RNA
   <213> Homo sapiens
<400> 800
   augguacccu ggcauacuga gu 22
<210> 801
   <211> 22
   <212> RNA
   <213> Homo sapiens
<400> 801
   augggugaau uuguagaagg au 22
<210> 802
   <211> 19
   <212> RNA
   <213> Homo sapiens
<400> 802
   auggauaagg cuuuggcuu 19
<210> 803
   <211> 18
   <212> RNA
   <213> Homo sapiens
<400> 803
   aucccaccuc ugccacca 18
<210> 804
   <211> 21
   <212> RNA
   <213> Homo sapiens
<400> 804
   cauuauuacu uuugguacgc g 21
<210> 805
   <211> 22
   <212> RNA
   <213> Homo sapiens
<400> 805
   ucguaccgug aguaauaaug cg 22
<210> 806
   <211> 22
   <212> RNA
   <213> Homo sapiens
<400> 806
   ucacaaguca ggcucuuggg ac 22
<210> 807
   <211> 22
   <212> RNA
   <213> Homo sapiens
<400> 807
   acggguuagg cucuugggag cu 22
<210> 808
   <211> 22
   <212> RNA
   <213> Homo sapiens
<400> 808
   ucccugagac ccuaacuugu ga 22
<210> 809
   <211> 24
   <212> RNA
   <213> Homo sapiens
<400> 809
   ucccugagac ccuuuaaccu guga 24
<210> 810
   <211> 22
   <212> RNA
   <213> Homo sapiens
<400> 810
   acaggugagg uucuugggag cc 22
<210> 811
   <211> 21
   <212> RNA
   <213> Homo sapiens
<400> 811
   auauaugaug acuuagcuuu u 21
<210> 812
   <211> 21
   <212> RNA
   <213> Homo sapiens
<400> 812
   aguuaggauu aggucgugga a 21
<210> 813
   <211> 21
   <212> RNA
   <213> Homo sapiens
<400> 813
   agugaaugau ggguucugac c 21
<210> 814
   <211> 22
   <212> RNA
   <213> Homo sapiens
<400> 814
   aggcauugac uucucacuag cu 22
<210> 815
   <211> 22
   <212> RNA
   <213> Homo sapiens
<400> 815
   cggaugagca aagaaagugg uu 22
<210> 816
   <211> 23
   <212> RNA
   <213> Homo sapiens
<400> 816
   aggaugagca aagaaaguag auu 23
<210> 817
   <211> 24
   <212> RNA
   <213> Homo sapiens
<400> 817
   agccuggaag cuggagccug cagu 24
<210> 818
   <211> 21
   <212> RNA
   <213> Homo sapiens
<400> 818
   agagaagaag aucagccugc a 21
<210> 819
   <211> 22
   <212> RNA
   <213> Homo sapiens
<400> 819
   agaaggaaau ugaauucauu ua 22
<210> 820
   <211> 21
   <212> RNA
   <213> Homo sapiens
<400> 820
   acucuagcug ccaaaggcgc u 21
<210> 821
   <211> 21
   <212> RNA
   <213> Homo sapiens
<400> 821
   acggugcugg auguggccuu u 21
<210> 822
   <211> 22
   <212> RNA
   <213> Homo sapiens
<400> 822
   acgcccuucc cccccuucuu ca 22
<210> 823
   <211> 27
   <212> RNA
   <213> Homo sapiens
<400> 823
   accuucuugu auaagcacug ugcuaaa 27
<210> 824
   <211> 22
   <212> RNA
   <213> Homo sapiens
<400> 824
   acccgucccg uucguccccg ga 22
<210> 825
   <211> 19
   <212> RNA
   <213> Homo sapiens
<400> 825
   aauggauuuu uggagcagg 19
<210> 826
   <211> 21
   <212> RNA
   <213> Homo sapiens
<400> 826
   aagugaucua aaggccuaca u 21
<210> 827
   <211> 26
   <212> RNA
   <213> Homo sapiens
<400> 827
   aaguaguugg uuuguaugag augguu 26
<210> 828
   <211> 22
   <212> RNA
   <213> Homo sapiens
<400> 828
   aacuggauca auuauaggag ug 22
<210> 829
   <211> 22
   <212> RNA
   <213> Homo sapiens
<400> 829
   cguguucaca gcggaccuug au 22
<210> 830
   <211> 20
   <212> RNA
   <213> Homo sapiens
<400> 830
   uaaggcacgc ggugaaugcc 20
<210> 831
   <211> 20
   <212> RNA
   <213> Homo sapiens
<400> 831
   cuuccucguc ugucugcccc 20
<210> 832
   <211> 21
   <212> RNA
   <213> Homo sapiens
<400> 832
   uccuucugcu ccguccccca g 21
<210> 833
   <211> 22
   <212> RNA
   <213> Homo sapiens
<400> 833
   ccucuucccc uugucucucc ag 22
<210> 834
   <211> 22
   <212> RNA
   <213> Homo sapiens
<400> 834
   ucggccugac cacccacccc ac 22
<210> 835
   <211> 20
   <212> RNA
   <213> Homo sapiens
<400> 835
   ugagcccugu ccucccgcag 20
<210> 836
   <211> 20
   <212> RNA
   <213> Homo sapiens
<400> 836
   gugucugggc ggacagcugc 20
<210> 837
   <211> 23
   <212> RNA
   <213> Homo sapiens
<400> 837
   cucucaccac ugcccuccca cag 23
<210> 838
   <211> 21
   <212> RNA
   <213> Homo sapiens
<400> 838
   gugggcgggg gcaggugugu g 21
<210> 839
   <211> 20
   <212> RNA
   <213> Homo sapiens
<400> 839
   ucacaccugc cucgcccccc 20
<210> 840
   <211> 20
   <212> RNA
   <213> Homo sapiens
<400> 840
   cgugccaccc uuuuccccag 20
<210> 841
   <211> 26
   <212> RNA
   <213> Homo sapiens
<400> 841
   gugagggcau gcaggccugg augggg 26
<210> 842
   <211> 22
   <212> RNA
   <213> Homo sapiens
<400> 842
   ucaccagccc uguguucccu ag 22
<210> 843
   <211> 22
   <212> RNA
   <213> Homo sapiens
<400> 843
   guggguacgg cccagugggg gg 22
<210> 844
   <211> 22
   <212> RNA
   <213> Homo sapiens
<400> 844
   ugagccccug ugccgccccc ag 22
<210> 845
   <211> 19
   <212> RNA
   <213> Homo sapiens
<400> 845
   gugaggacuc gggaggugg 19
<210> 846
   <211> 21
   <212> RNA
   <213> Homo sapiens
<400> 846
   ccccaccucc ucucuccuca g 21
<210> 847
   <211> 22
   <212> RNA
   <213> Homo sapiens
<400> 847
   aacgccauua ucacacuaaa ua 22
<210> 848
   <211> 22
   <212> RNA
   <213> Homo sapiens
<400> 848
   uggaguguga caaugguguu ug 22
<210> 849
   <211> 20
   <212> RNA
   <213> Homo sapiens
<400> 849
   ucacuguuca gacaggcgga 20
<210> 850
   <211> 21
   <212> RNA
   <213> Homo sapiens
<400> 850
   uggcagggag gcugggaggg g 21
<210> 851
   <211> 18
   <212> RNA
   <213> Homo sapiens
<400> 851
   ucagcuggcc cucauuuc 18
<210> 852
   <211> 21
   <212> RNA
   <213> Homo sapiens
<400> 852
   uguucaugua gauguuuaag c 21
<210> 853
   <211> 20
   <212> RNA
   <213> Homo sapiens
<400> 853
   ucugcagggu uugcuuugag 20
<210> 854
   <211> 21
   <212> RNA
   <213> Homo sapiens
<400> 854
   ucguggccug gucuccauua u 21
<210> 855
   <211> 21
   <212> RNA
   <213> Homo sapiens
<400> 855
   cccggagcca ggaugcagcu c 21
<210> 856
   <211> 21
   <212> RNA
   <213> Homo sapiens
<400> 856
   gugccagcug caguggggga g 21
<210> 857
   <211> 24
   <212> RNA
   <213> Homo sapiens
<400> 857
   agccugauua aacacaugcu cuga 24
<210> 858
   <211> 22
   <212> RNA
   <213> Homo sapiens
<400> 858
   cuccugagcc auucugagcc uc 22
<210> 859
   <211> 21
   <212> RNA
   <213> Homo sapiens
<400> 859
   uaggacacau ggucuacuuc u 21
<210> 860
   <211> 21
   <212> RNA
   <213> Homo sapiens
<400> 860
   agaggauacc cuuuguaugu u 21
<210> 861
   <211> 23
   <212> RNA
   <213> Homo sapiens
<400> 861
   ccugcagcga cuugauggcu ucc 23
<210> 862
   <211> 27
   <212> RNA
   <213> Homo sapiens
<400> 862
   cacuguaggu gauggugaga gugggca 27
<210> 863
   <211> 23
   <212> RNA
   <213> Homo sapiens
<400> 863
   gagggucuug ggagggaugu gac 23
<210> 864
   <211> 21
   <212> RNA
   <213> Homo sapiens
<400> 864
   ccgucgccgc cacccgagcc g 21
<210> 865
   <211> 22
   <212> RNA
   <213> Homo sapiens
<400> 865
   uuuccggcuc gcgugggugu gu 22
<210> 866
   <211> 21
   <212> RNA
   <213> Homo sapiens
<400> 866
   aagcauucuu ucauugguug g 21
<210> 867
   <211> 21
   <212> RNA
   <213> Homo sapiens
<400> 867
   uugcucacug uucuucccua g 21
<210> 868
   <211> 22
   <212> RNA
   <213> Homo sapiens
<400> 868
   acagauucga uucuagggga au 22
<210> 869
   <211> 23
   <212> RNA
   <213> Homo sapiens
<400> 869
   uacccuguag aaccgaauuu gug 23
<210> 870
   <211> 22
   <212> RNA
   <213> Homo sapiens
<400> 870
   caaauucgua ucuaggggaa ua 22
<210> 871
   <211> 23
   <212> RNA
   <213> Homo sapiens
<400> 871
   uacccuguag auccgaauuu gug 23
<210> 872
   <211> 23
   <212> RNA
   <213> Homo sapiens
<400> 872
   agcagcauug uacagggcua uca 23
<210> 873
   <211> 22
   <212> RNA
   <213> Homo sapiens
<400> 873
   ccgcacugug gguacuugcu gc 22
<210> 874
   <211> 21
   <212> RNA
   <213> Homo sapiens
<400> 874
   uaaagugcug acagugcaga u 21
<210> 875
   <211> 22
   <212> RNA
   <213> Homo sapiens
<400> 875
   cugcaaugua agcacuucuu ac 22
<210> 876
   <211> 23
   <212> RNA
   <213> Homo sapiens
<400> 876
   aaaagugcuu acagugcagg uag 23
<210> 877
   <211> 22
   <212> RNA
   <213> Homo sapiens
<400> 877
   acggauguuu gagcaugugc ua 22
<210> 878
   <211> 23
   <212> RNA
   <213> Homo sapiens
<400> 878
   ucaaaugcuc agacuccugu ggu 23
<210> 879
   <211> 23
   <212> RNA
   <213> Homo sapiens
<400> 879
   ucauagcccu guacaaugcu gcu 23
<210> 880
   <211> 23
   <212> RNA
   <213> Homo sapiens
<400> 880
   agcuucuuua cagugcugcc uug 23
<210> 881
   <211> 23
   <212> RNA
   <213> Homo sapiens
<400> 881
   agcagcauug uacagggcua uga 23
<210> 882
   <211> 22
   <212> RNA
   <213> Homo sapiens
<400> 882
   caguuaucac agugcugaug cu 22
<210> 883
   <211> 21
   <212> RNA
   <213> Homo sapiens
<400> 883
   uacaguacug ugauaacuga a 21
<210> 884
   <211> 22
   <212> RNA
   <213> Homo sapiens
<400> 884
   caagcuugua ucuauaggua ug 22
<210> 885
   <211> 22
   <212> RNA
   <213> Homo sapiens
<400> 885
   aacccguaga uccgaacuug ug 22
<210> 886
   <211> 22
   <212> RNA
   <213> Homo sapiens
<400> 886
   uggaauguaa agaaguaugu au 22
<210> 887
   <211> 22
   <212> RNA
   <213> Homo sapiens
<400> 887
   cugcgcaagc uacugccuug cu 22
<210> 888
   <211> 22
   <212> RNA
   <213> Homo sapiens
<400> 888
   ugagguagua guuugugcug uu 22
<210> 889
   <211> 21
   <212> RNA
   <213> Homo sapiens
<400> 889
   cuguacaggc cacugccuug c 21
<210> 890
   <211> 22
   <212> RNA
   <213> Homo sapiens
<400> 890
   ugagguagua guuuguacag uu 22
<210> 891
   <211> 22
   <212> RNA
   <213> Homo sapiens
<400> 891
   cuauacaguc uacugucuuu cc 22
<210> 892
   <211> 22
   <212> RNA
   <213> Homo sapiens
<400> 892
   cuauacaauc uauugccuuc cc 22
<210> 893
   <211> 22
   <212> RNA
   <213> Homo sapiens
<400> 893
   ugagguagua gauuguauag uu 22
<210> 894
   <211> 22
   <212> RNA
   <213> Homo sapiens
<400> 894
   cuauacggcc uccuagcuuu cc 22
<210> 895
   <211> 22
   <212> RNA
   <213> Homo sapiens
<400> 895
   ugagguagga gguuguauag uu 22
<210> 896
   <211> 22
   <212> RNA
   <213> Homo sapiens
<400> 896
   cuauacgacc ugcugccuuu cu 22
<210> 897
   <211> 22
   <212> RNA
   <213> Homo sapiens
<400> 897
   agagguagua gguugcauag uu 22
<210> 898
   <211> 22
   <212> RNA
   <213> Homo sapiens
<400> 898
   uagaguuaca cccugggagu ua 22
<210> 899
   <211> 22
   <212> RNA
   <213> Homo sapiens
<400> 899
   ugagguagua gguuguaugg uu 22
<210> 900
   <211> 22
   <212> RNA
   <213> Homo sapiens
<400> 900
   cuauacaacc uacugccuuc cc 22
<210> 901
   <211> 22
   <212> RNA
   <213> Homo sapiens
<400> 901
   ugagguagua gguugugugg uu 22
<210> 902
   <211> 22
   <212> RNA
   <213> Homo sapiens
<400> 902
   cuguacagcc uccuagcuuu cc 22
<210> 903
   <211> 21
   <212> RNA
   <213> Homo sapiens
<400> 903
   cuauacaauc uacugucuuu c 21
<210> 904
   <211> 22
   <212> RNA
   <213> Homo sapiens
<400> 904
   ugagguagua gguuguauag uu 22
<210> 905
   <211> 22
   <212> RNA
   <213> Homo sapiens
<400> 905
   uaauacugcc ugguaaugau ga 22
<210> 906
   <211> 21
   <212> RNA
   <213> Homo sapiens
<400> 906
   cucuccucuc cuaaccucgc u 21
<210> 907
   <211> 23
   <212> RNA
   <213> Homo sapiens
<400> 907
   agucgagagu gggagaagag cgg 23
<210> 908
   <211> 19
   <212> RNA
   <213> Homo sapiens
<400> 908
   aaaaccgucu aguuacagu 19
<210> 909
   <211> 23
   <212> RNA
   <213> Homo sapiens
<400> 909
   cucagugaug aaaacuuugu cca 23
<210> 910
   <211> 21
   <212> RNA
   <213> Homo sapiens
<400> 910
   guugccuuuu uguucccaug c 21
<210> 911
   <211> 21
   <212> RNA
   <213> Homo sapiens
<400> 911
   uaggcaccaa aaagcaacaa c 21
<210> 912
   <211> 21
   <212> RNA
   <213> Homo sapiens
<400> 912
   gcugcaccgg agacugggua a 21
<210> 913
   <211> 21
   <212> RNA
   <213> Homo sapiens
<400> 913
   uacccagucu ccggugcagc c 21
<210> 914
   <211> 24
   <212> RNA
   <213> Homo sapiens
<400> 914
   uuccucugau gacuuccugu uagu 24
<210> 915
   <211> 21
   <212> RNA
   <213> Homo sapiens
<400> 915
   uggaacugag gaucugagga a 21
<210> 916
   <211> 21
   <212> RNA
   <213> Homo sapiens
<400> 916
   aguggcaaag ucuuuccaua u 21
<210> 917
   <211> 22
   <212> RNA
   <213> Homo sapiens
<400> 917
   uuagcucagc gguuacuucg ac 22
<210> 918
   <211> 21
   <212> RNA
   <213> Homo sapiens
<400> 918
   caagcaaccu gucuggguug u 21
<210> 919
   <211> 19
   <212> RNA
   <213> Homo sapiens
<400> 919
   uaacgcauaa uauggacau 19
<210> 920
   <211> 21
   <212> RNA
   <213> Homo sapiens
<400> 920
   auguccauau uauggguuag u 21
<210> 921
   <211> 21
   <212> RNA
   <213> Homo sapiens
<400> 921
   caguugcuag uugcacuccu c 21
<210> 922
   <211> 21
   <212> RNA
   <213> Homo sapiens
<400> 922
   aggcagugua uugcuagcgg c 21
<210> 923
   <211> 21
   <212> RNA
   <213> Homo sapiens
<400> 923
   aguucuugcc ugguuucucu a 21
<210> 924
   <211> 18
   <212> RNA
   <213> Homo sapiens
<400> 924
   ucugcauugc cagggauu 18
<210> 925
   <211> 19
   <212> RNA
   <213> Homo sapiens
<400> 925
   uagcuuuaga gacugagag 19
<210> 926
   <211> 23
   <212> RNA
   <213> Homo sapiens
<400> 926
   ugagacaggc uuaugcugcu auc 23
<210> 927
   <211> 21
   <212> RNA
   <213> Homo sapiens
<400> 927
   agcagcauga accugucuca c 21
<210> 928
   <211> 21
   <212> RNA
   <213> Homo sapiens
<400> 928
   ccggcggcag ggguggcacc g 21
<210> 929
   <211> 20
   <212> RNA
   <213> Homo sapiens
<400> 929
   gccuuaggag aaaguuucug 20
<210> 930
   <211> 19
   <212> RNA
   <213> Homo sapiens
<400> 930
   uccuaaggca gucccugga 19
<210> 931
   <211> 21
   <212> RNA
   <213> Homo sapiens
<400> 931
   aaggucgccc ucaaggugac c 21
<210> 932
   <211> 21
   <212> RNA
   <213> Homo sapiens
<400> 932
   augccuggga guugcgaucu g 21
<210> 933
   <211> 21
   <212> RNA
   <213> Homo sapiens
<400> 933
   uggaguuaaa gacuuuuucu c 21
<210> 934
   <211> 24
   <212> RNA
   <213> Homo sapiens
<400> 934
   cagagaauag uuuaaauuag aauc 24
<210> 935
   <211> 23
   <212> RNA
   <213> Homo sapiens
<400> 935
   ugcaacuuac ugagggcuuu gaa 23
<210> 936
   <211> 21
   <212> RNA
   <213> Homo sapiens
<400> 936
   ugggguuuug caguccuuag c 21
<210> 937
   <211> 21
   <212> RNA
   <213> Homo sapiens
<400> 937
   agacacuaua cgagucauau a 21
<210> 938
   <211> 21
   <212> RNA
   <213> Homo sapiens
<400> 938
   auaggacuca uauagugcca g 21
<210> 939
   <211> 22
   <212> RNA
   <213> Homo sapiens
<400> 939
   auccccagau acaauggaca au 22
<210> 940
   <211> 23
   <212> RNA
   <213> Homo sapiens
<400> 940
   uucaccuguu agccugucca gag 23
<210> 941
   <211> 23
   <212> RNA
   <213> Homo sapiens
<400> 941
   ugacagcgcc cugccuggcu cgg 23
<210> 942
   <211> 23
   <212> RNA
   <213> Homo sapiens
<400> 942
   agcgcgggcu gagcgcugcc agu 23
<210> 943
   <211> 22
   <212> RNA
   <213> Homo sapiens
<400> 943
   uauacuacau auaauauaug ua 22
<210> 944
   <211> 22
   <212> RNA
   <213> Homo sapiens
<400> 944
   uauacuacau auaauauaug ua 22
<210> 945
   <211> 23
   <212> RNA
   <213> Homo sapiens
<400> 945
   uagcaccauc ugaaaucggu uau 23
<210> 946
   <211> 22
   <212> RNA
   <213> Homo sapiens
<400> 946
   gugcaaaagu caucacgguu uu 22
<210> 947
   <211> 21
   <212> RNA
   <213> Homo sapiens
<400> 947
   accgcgauga cuuuugcauc a 21
<210> 948
   <211> 23
   <212> RNA
   <213> Homo sapiens
<400> 948
   ucaccgcggu cuuuuccucc cac 23
<210> 949
   <211> 21
   <212> RNA
   <213> Homo sapiens
<400> 949
   uuggggaaac ggccgcugag u 21
<210> 950
   <211> 21
   <212> RNA
   <213> Homo sapiens
<400> 950
   uccucccaug ccaagaacuc c 21
<210> 951
   <211> 21
   <212> RNA
   <213> Homo sapiens
<400> 951
   gguucuuagc auaggagguc u 21
<210> 952
   <211> 22
   <212> RNA
   <213> Homo sapiens
<400> 952
   cugcgugucc cuaggugagg gg 22
<210> 953
   <211> 22
   <212> RNA
   <213> Homo sapiens
<400> 953
   ggcacagggc gaguggaaag aa 22
<210> 954
   <211> 22
   <212> RNA
   <213> Homo sapiens
<400> 954
   ucacuaccug acaauacagu au 22
<210> 955
   <211> 22
   <212> RNA
   <213> Homo sapiens
<400> 955
   ugcuguauug ucagguagug au 22
<210> 956
   <211> 24
   <212> RNA
   <213> Homo sapiens
<400> 956
   uggaggugau gaacugucug agcc 24
<210> 957
   <211> 21
   <212> RNA
   <213> Homo sapiens
<400> 957
   ugcguguccc gccuguuccc u 21
<210> 958
   <211> 24
   <212> RNA
   <213> Homo sapiens
<400> 958
   ugugcugauu gucacguucu gauu 24
<210> 959
   <211> 24
   <212> RNA
   <213> Homo sapiens
<400> 959
   acagaugaug aacuuauuga cggg 24
<210> 960
   <211> 23
   <212> RNA
   <213> Homo sapiens
<400> 960
   accuuccucu ccaugggucu uuc 23
<210> 961
   <211> 22
   <212> RNA
   <213> Homo sapiens
<400> 961
   agacccauug aggagaaggu uc 22
<210> 962
   <211> 21
   <212> RNA
   <213> Homo sapiens
<400> 962
   uucucaagag ggaggcaauc a 21

## Description

### Background of the invention

MicroRNAs (miRNA) are a recently discovered class of small non-coding RNAs (17-14 nucleotides). Due to their function as regulators of gene expression they play a critical role both in physiological and in pathological processes, such as cancer (Calin and Croce 2006; Esquela-Kerscher and Slack 2006; Zhang, Pan et al. 2007; Sassen, Miska et al. 2008).

There is increasing evidence that miRNAs are not only found in tissues but also in human blood both as free circulating nucleic acids and in mononuclear cells. A recent proof-of-principle study demonstrated miRNA expression pattern in pooled blood sera and pooled blood cells, both in healthy individuals and in cancer patients including patients with lung cancer (Chen, Ba et al. 2008). In addition, a remarkable stability of miRNAs in human sera was recently demonstrated (Chen, Ba et al. 2008; Gilad, Meiri et al. 2008). These findings make miRNA a potential tool for diagnostics for various types of diseases based on blood analysis.

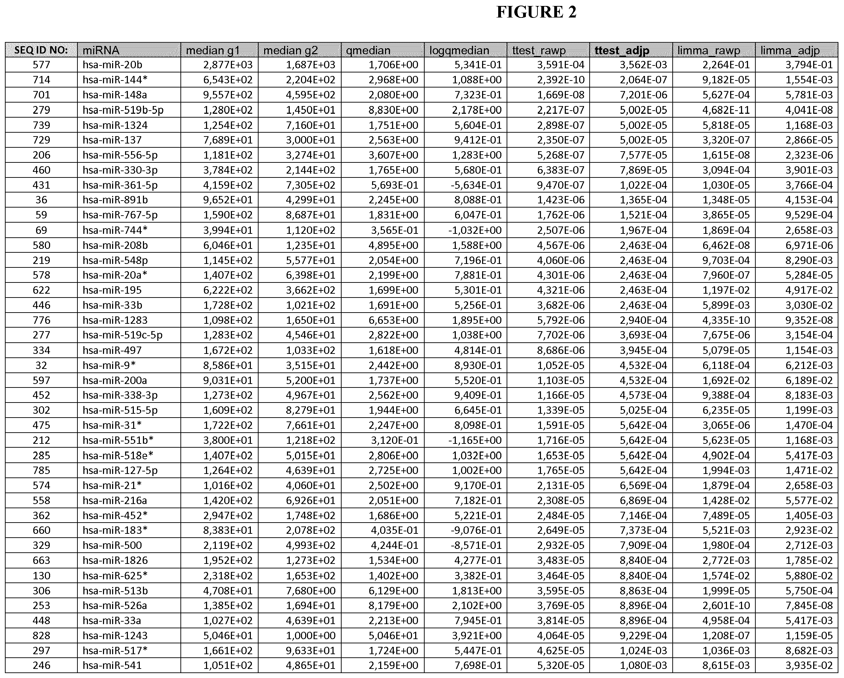

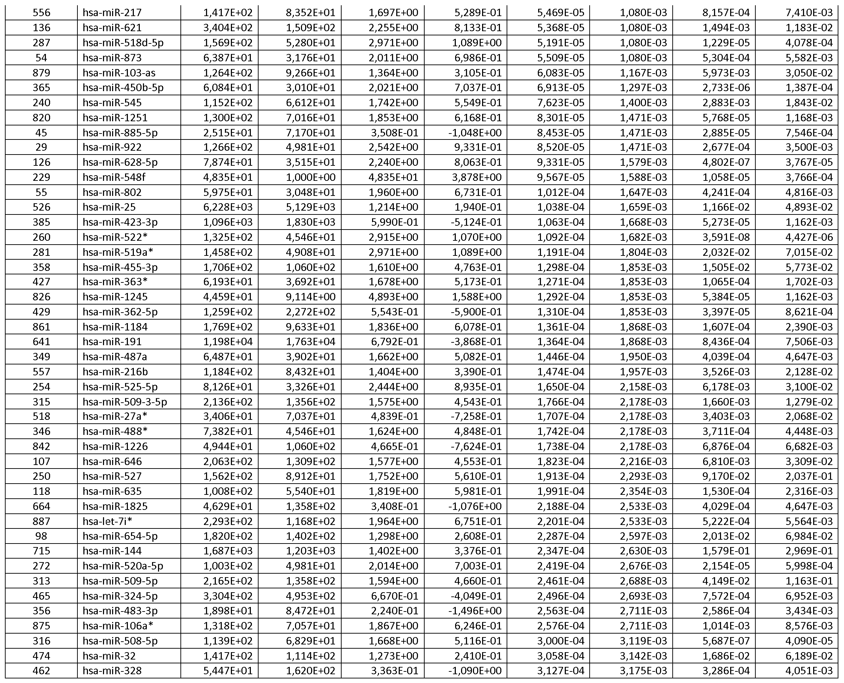

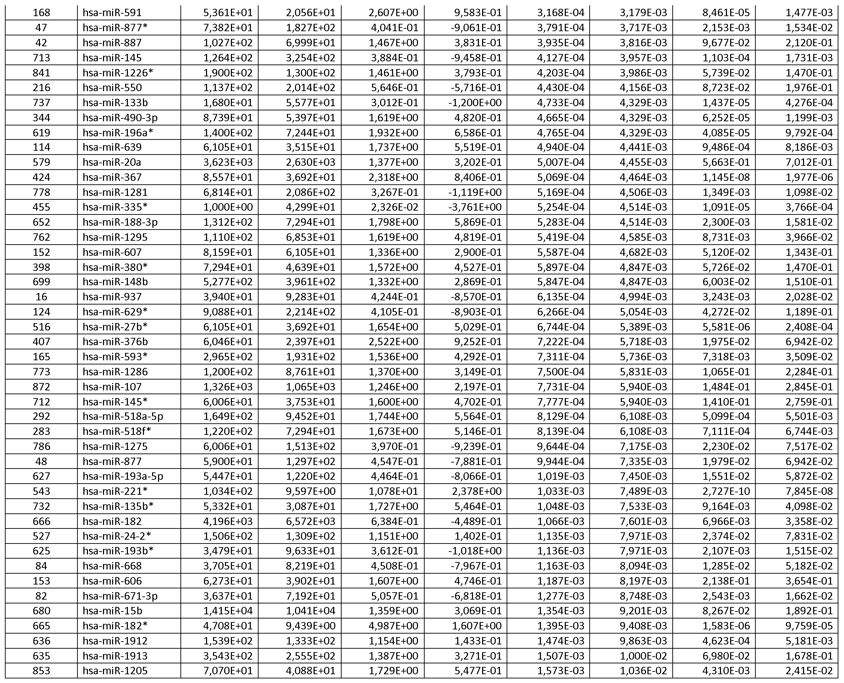

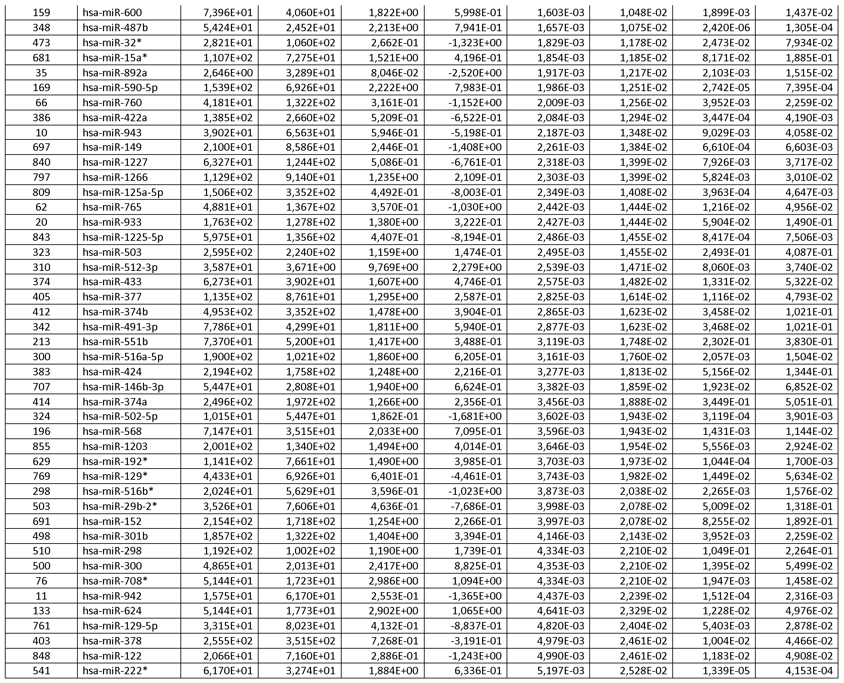

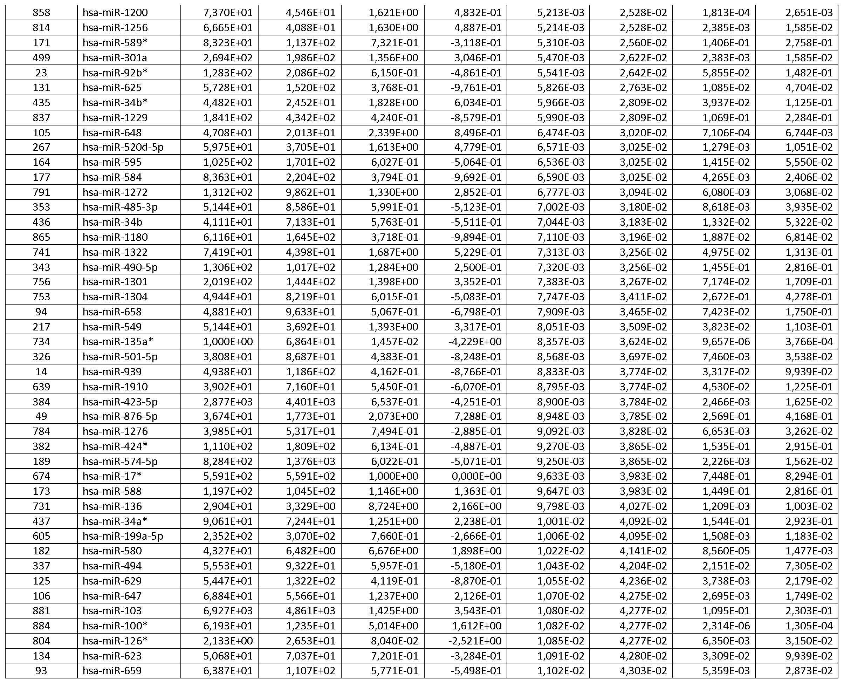

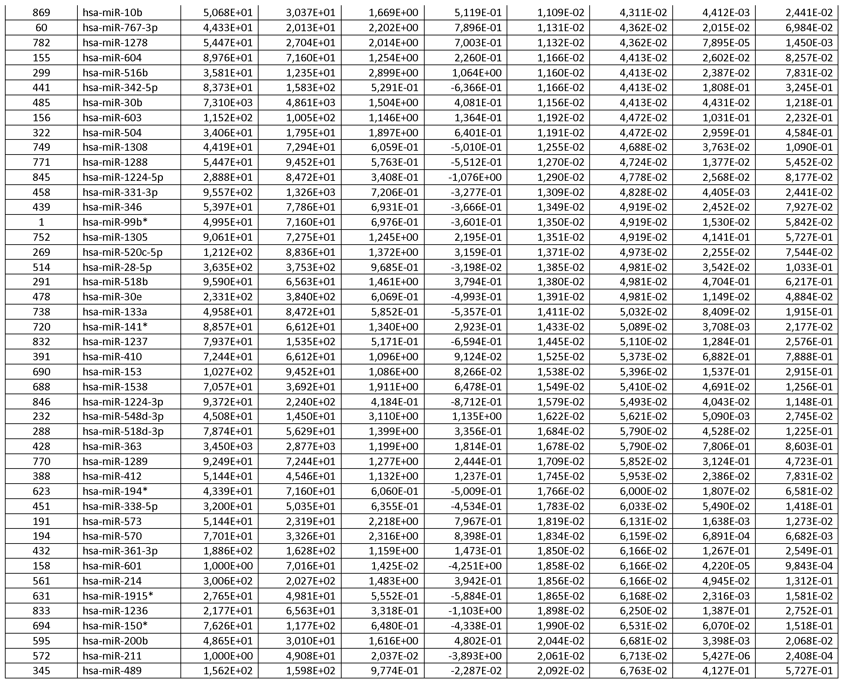

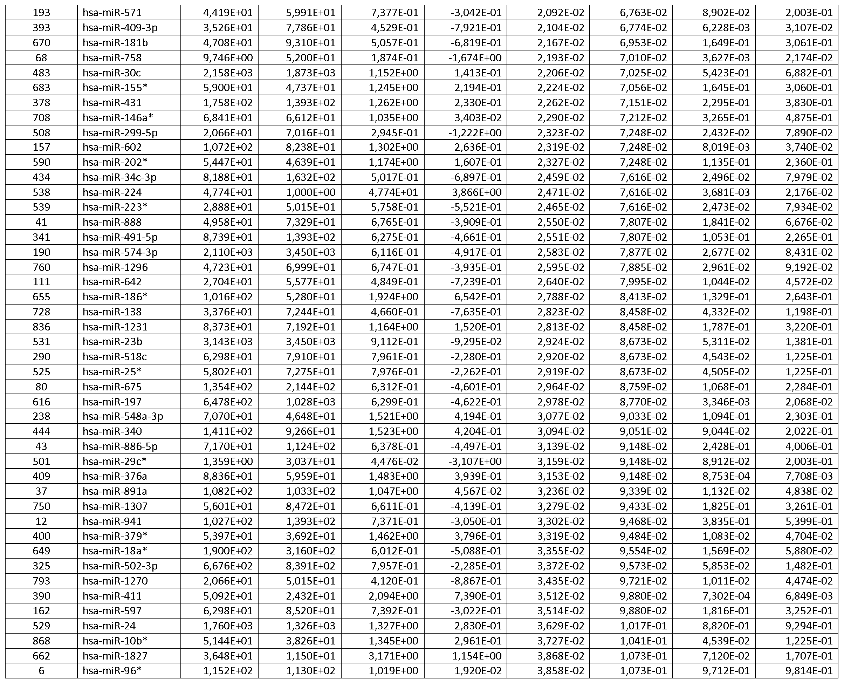

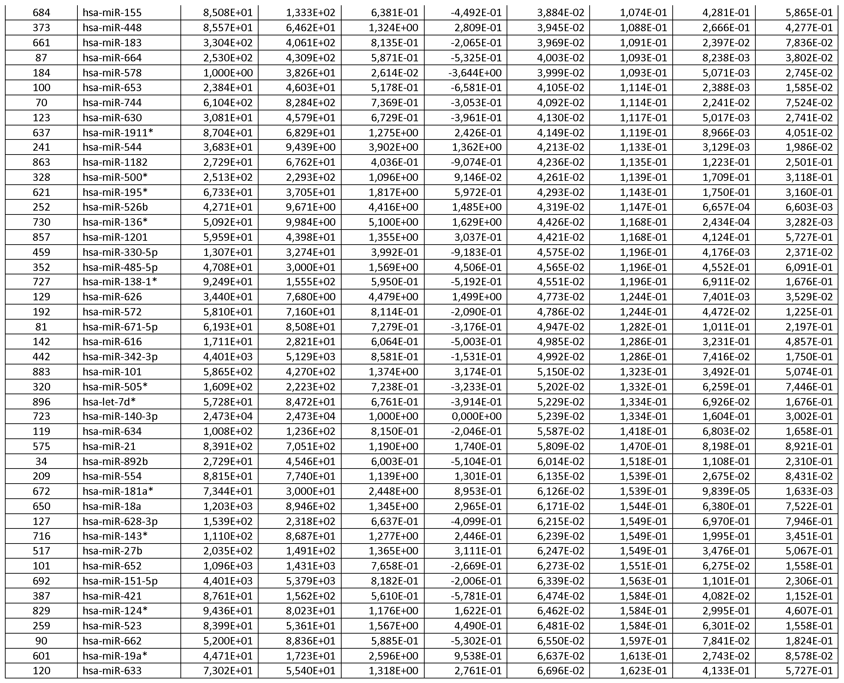

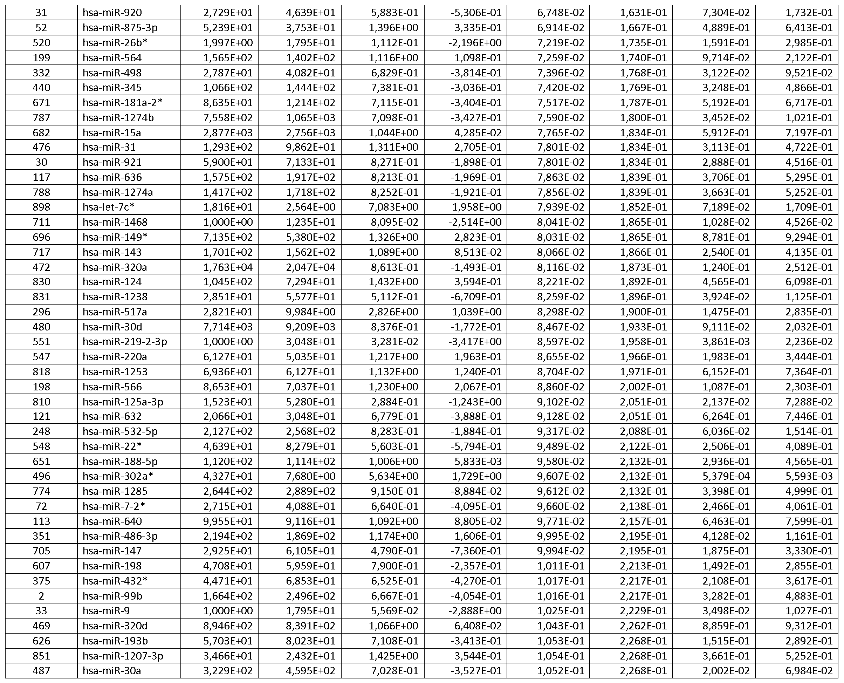

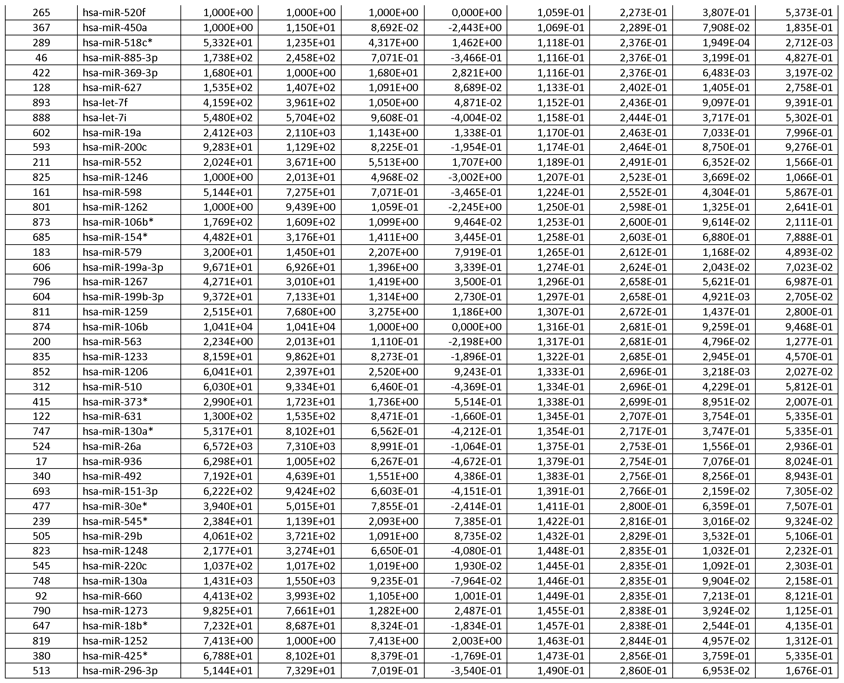

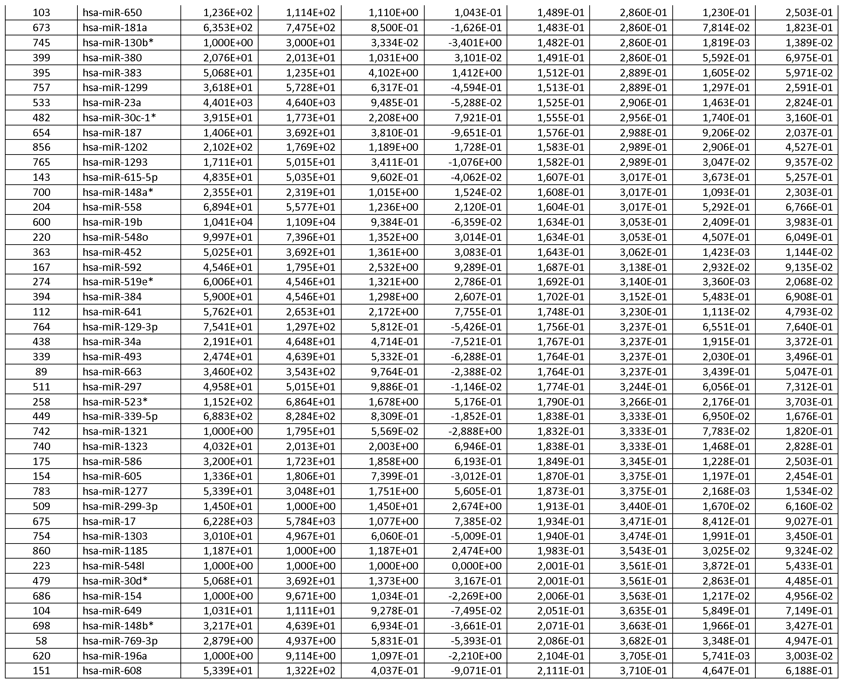

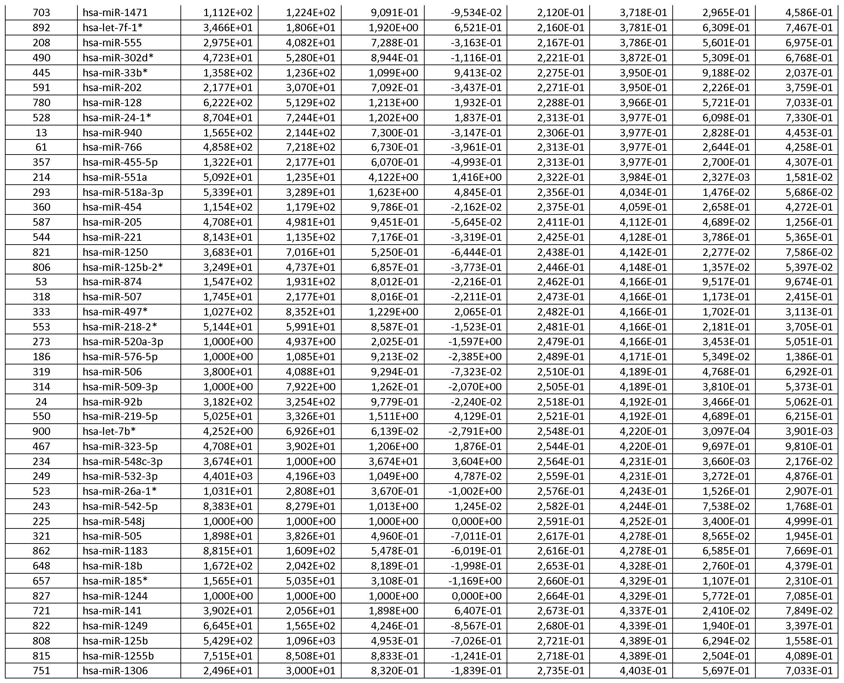

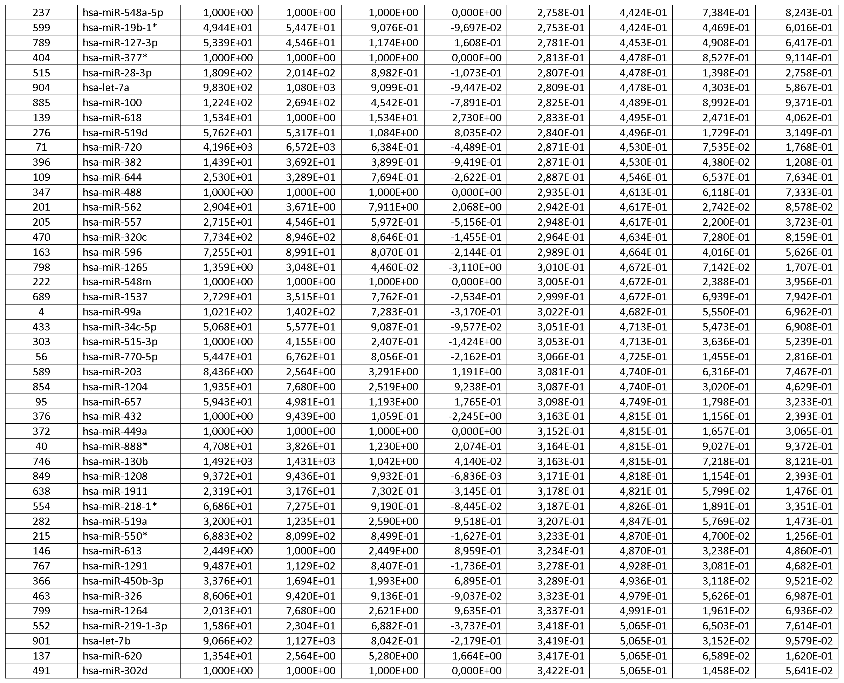

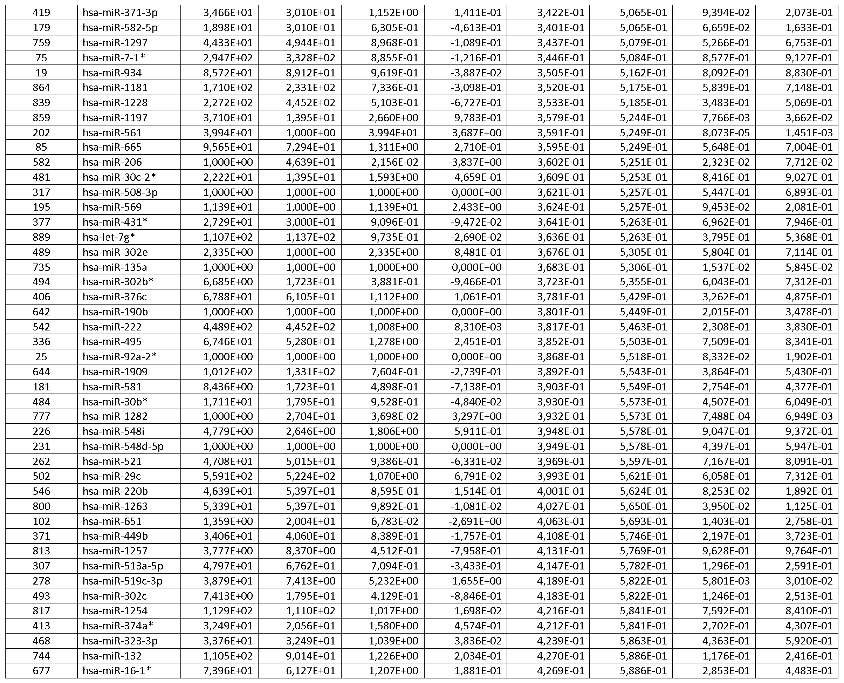

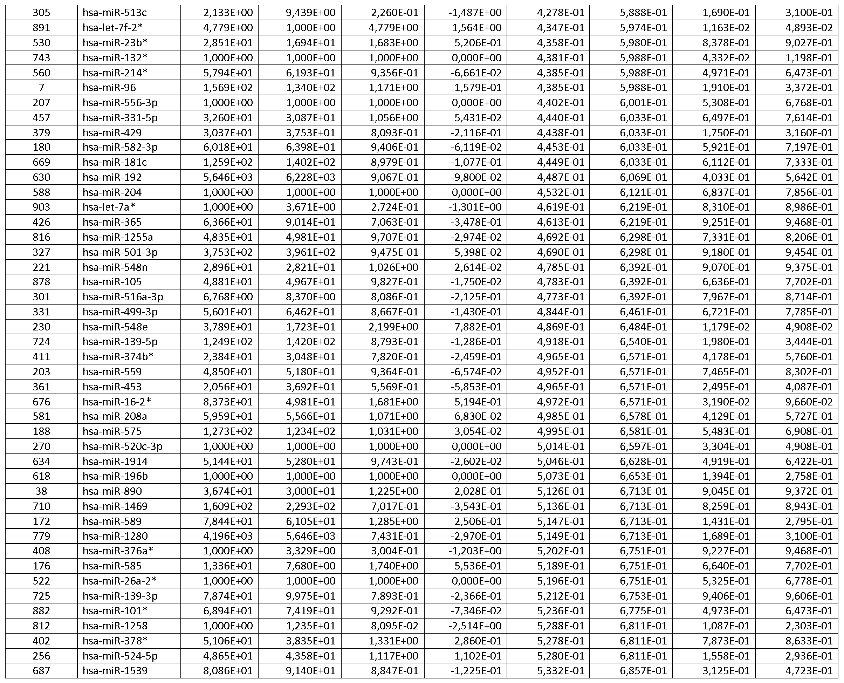

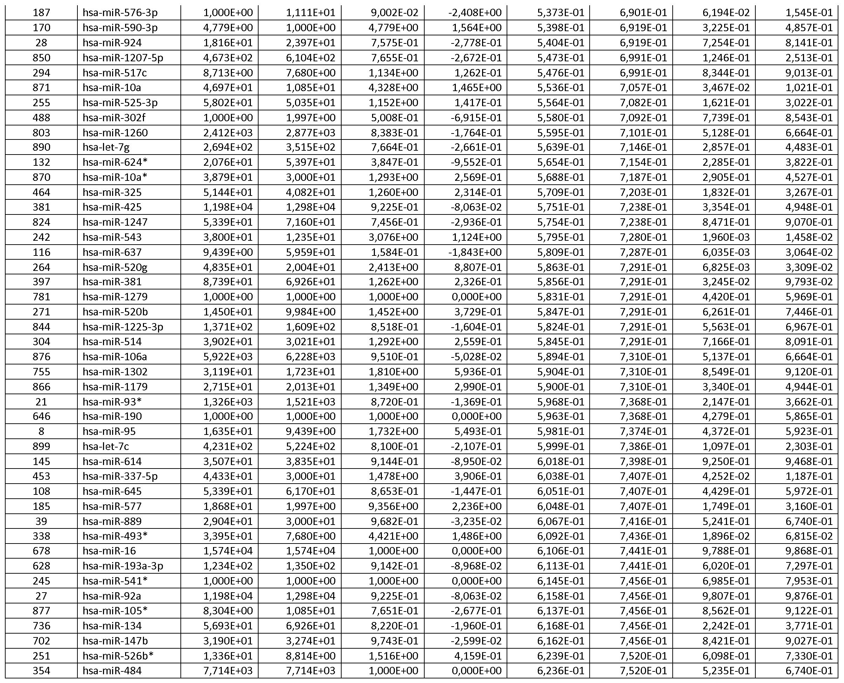

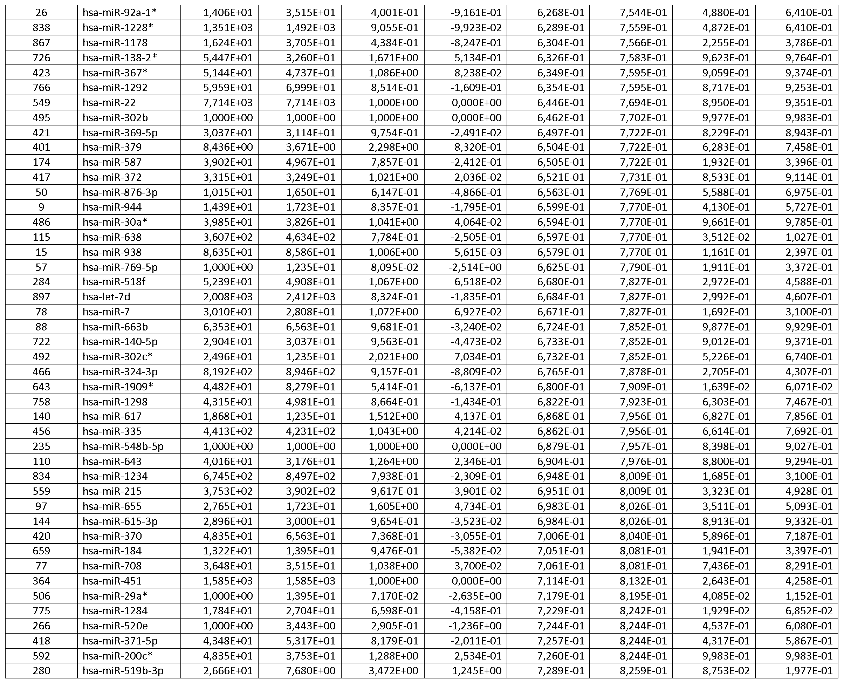

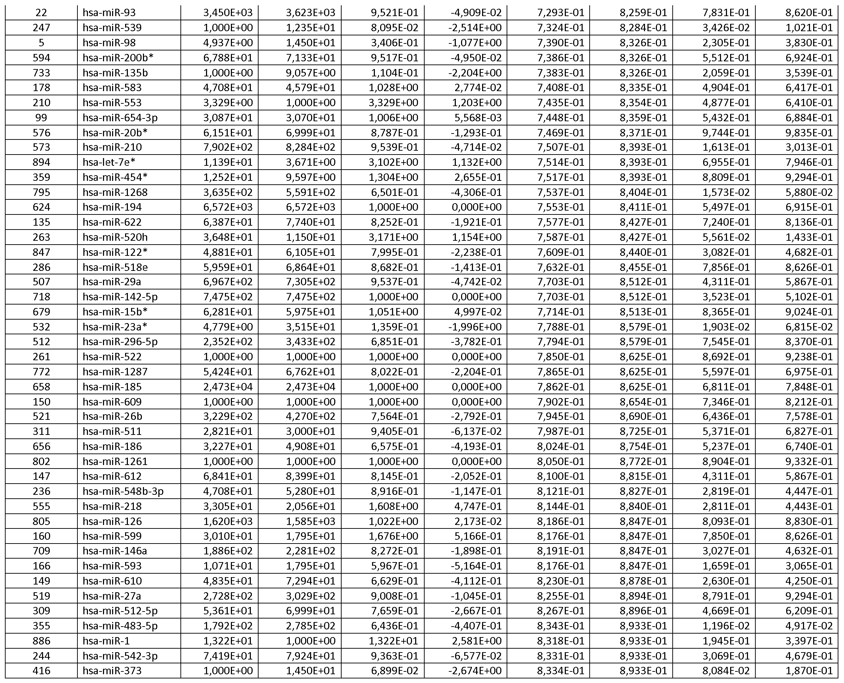

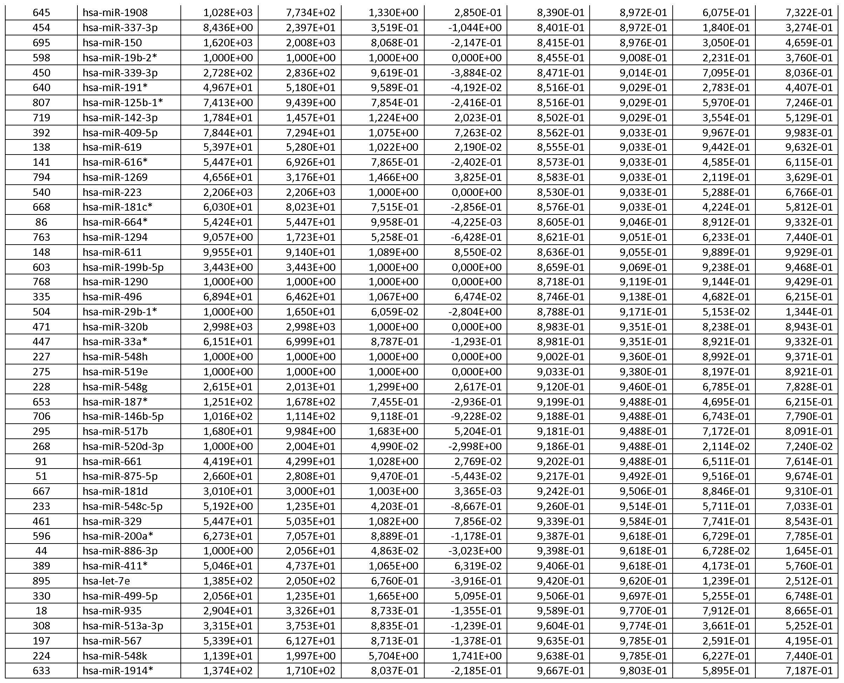

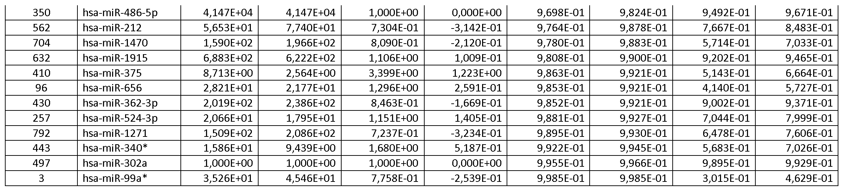

Thus, although various markers have been proposed to indicate specific types of disorders such as prostate cancer, there is still a need for more efficient and effective methods and compositions for the diagnosis of diseases. As the closest prior art one could consider WO2009108860 disclosing a method for diagnosing prostate cancer by means of expression profiling of microRNA species, the latter comprising inter alia - c.f. Figure 2 - microRNA species that have also been identified in the underlying application as differentially regulated. WO2009108860 does not rely upon the extraction of the miRNAs from cellular fractions of blood comprising erythrocytes, leukocytes and thrombocytes but instead uses non-cellular blood samples, namely serum or plasma blood samples as well as tumor tissue.

### Summary of the invention

The present invention provides novel methods for diagnosing diseases based on the determination of specific miRNAs that have altered expression levels in disease states compared to healthy controls or altered expression levels in a condition 1 (biological state or health state 1) compared to a condition 2 (biological state or health state 2). The disease is particularly selected from prostate cancer and the sample is a blood cell sample comprising erythrocytes, leukocytes and thrombocytes.

### Definitions

### miRNA

microRNAs (miRNA or µRNA) are single-stranded RNA molecules of ∼21-23 nucleotides in length, which regulate gene expression. miRNAs are encoded by genes from whose DNA they are transcribed but miRNAs are not translated into protein (i.e. they are non-coding RNAs). The genes encoding miRNAs are much longer than the processed mature miRNA molecule; miRNAs are first transcribed as primary transcripts or pri-miRNA with a cap and poly-A tail and processed to short, 70-nucleotide stem-loop structures known as pre-miRNA in the cell nucleus. This processing is performed in animals by a protein complex known as the Microprocessor complex, consisting of the nuclease Drosha and the double-stranded RNA binding protein Pasha. These pre-miRNAs are then processed to mature miRNAs in the cytoplasm by interaction with the endonuclease Dicer, which also initiates the formation of the RNA-induced silencing complex (RISC). When Dicer cleaves the pre-miRNA stem-loop, two complementary short RNA molecules are formed, but only one is integrated into the RISC. This strand is known as the guide strand and is selected by the argonaute protein, the catalytically active RNase in the RISC, on the basis of the stability of the 5' end. The remaining strand, known as the miRNA*, anti-guide or passenger strand, is degraded as a RISC substrate. Therefore the miRNA*s are derived from the same hairpin structure like the "normal" miRNAs. So if the "normal" miRNA is then later called the "mature miRNA" or "guided strand", the miRNA* is the passenger strand.

### miRNA* (see also above "miRNA")

The miRNA*s, also known as the anti-guide or passenger strand, are mostly complementary to the guide strand, but there are usually single-stranded overhangs on each end, there is usually one or a few mispairs and there are sometimes extra or missing bases causing single-stranded "bubbles.The miRNA*s are likely to act in a regulatory fashion as the miRNAs.

It is understood that according to the present invention the term "miRNA" also includes the term "miRNA*".

### miRBase

A well established repository of validated miRNAs is the miRBase.

The miRBase (www.mirbase.org) is a searchable database of published miRNA sequences and annotation. Each entry in the miRBase Sequence database represents a predicted hairpin portion of a miRNA transcript (termed mir in the database), with information on the location and sequence of the mature miRNA sequence (termed miR). Both hairpin and mature sequences are available for searching and browsing, and entries can also be retrieved by name, keyword, references and annotation. All sequence and annotation data are also available for download.

### miRNA-(expression) profile or miRNA fingerprint

A miRNA-Profile represents the collection of expression levels of a plurality of miRNAs, therefore it is a quantitative measure of individual miRNA expression levels. Hereby, each miRNA is represented by a numerical value. The higher the value of an individual miRNA the higher is the expression level of this miRNA. A miRNA-profile is obtained from the RNA of a biological sample. The are various technologies to determine a miRNA-Profile, e.g. microarrays, RT-PCR, Next Generation Sequencing. As a starting material for analysis, RNA or total-RNA or any fraction thereof can be used. The plurality of miRNAs that are determined by a miRNA-profile can range from a selection of one up to all known miRNAs.

### Pre-determined set of miRNAs or miRNA signature

The pre-determined set of miRNAs or miRNA signature is understood in the present invention as a fixed defined set of miRNAs which is able to differentiate between a condition 1 and another condition 2. e.g. when condition 1 is lung cancer and condition 2 is normal control, the corresponding pre-determined set of miRNAs is able to differentiate between a samples derived from a lung cancer patient or a normal control patient. Alternatively, condition 1 is lung cancer and condition 2 is multiple sclerosis, the corresponding pre-determined set of miRNAs is able to differentiate between a lung cancer patient and a multiple sclerosis patient. In order to be able to perform the sample analysis it is required that, e.g. on the matrix that will be used to determine a miRNA profile, these fixed defined set of miRNAs have to be represented by capture probes that are defined by the pre-determined set of miRNAs. For example, when the predetermined set of miRNAs for diagnosing lung cancer from healthy controls consists of 25 miRNAs, probes capable for detecting these 25 miRNAs have to be implemented for performing the diagnostic analysis.

### Common miRNA Signature Profile

A common miRNA signature profile is understood in the present invention as a non-fixed defined set of miRNAs or non-coding RNAs which is able to differentiate between a condition 1 and another condition 2. The common miRNA or non-coding RNA signature profile is calculated "on-the-fly" from a plurality of miRNA-profiles that are stored, e.g. in database. The common miRNA signature profile which is able to differentiate between a condition 1 and another condition 2 is changing as soon as an new profile is added to the database which is relevant to either to state of health 1 or another condition 2. In this respect it is different from a predetermined set of miRNAs (see above). Furthermore, the basis for generating the common miRNA signature profile- hence the miRNA profiles stored in the database - is generated from capture probes, e.g. on a matrix that is representing as much as possible different capture probes for detecting as much as possible, ideally all known, miRNAs.

### Non-coding RNA

A non-coding RNA (ncRNA) is a functional RNA molecule that is not translated into a protein. Less-frequently used synonyms are non-protein-coding RNA (npcRNA), non-messenger RNA (nmRNA), small non-messenger RNA (snmRNA), functional RNA (fRNA). The term small RNA (sRNA) is often used for bacterial ncRNAs. The DNA sequence from which a non-coding RNA is transcribed as the end product is often called an RNA gene or non-coding RNA gene.

Non-coding RNA genes include highly abundant and functionally important RNAs such as transfer RNA (tRNA) and ribosomal RNA (rRNA), as well as RNAs such as snoRNAs, microRNAs, siRNAs and piRNAs and the long ncRNAs that include examples such as Xist and HOTAIR (see here for a more complete list of ncRNAs). The number of ncRNAs encoded within the human genome is unknown, however recent transcriptomic and bioinformatic studies suggest the existence of thousands of ncRNAs. Since most of the newly identified ncRNAs have not been validated for their function, it is possible that many are non-functional.

### Condition

A condition (biological state or health state or state of health) is understood in the present invention as status of a subject that can be described by physical, mental or social criteria. It includes as well so-called "healthy" and "diseased" conditions, therefore it is not limited to the WHO definition of health as "a state of complete physical, mental, and social well-being and not merely the absence of disease or infirmity." but includes disease and infirmity. For the definition of diseases comprised, e.g. by the conditions of the present invention, it is referred to the international classification of diseases (ICD) of the WHO (http://www.who.int/classifications/icd/en /index.html). When 2 or more conditions are compared according to the present invention, it is understood that this is possible for all conditions that can be defined and is not limited to a comparison of a disease versus healthy and extends to multi-way comparisons. Examples for comparison are, but not limited to:
pairwise comparisons :
▪ lung cancer vs. healthy control, pancreatic cancer vs. healthy control
▪ lung cancer vs. pancreatic cancer, lung cancer vs. multiple sclerosis
▪ lung cancer WHO grade 1 vs. lung cancer WHO grade 2
▪ lung cancer WHO grade 1 metastasing vs. lung cancer WHO grade 1 non-metastasing
▪ Morbus Crohn vs. collitis
▪ Pancreatic cancer vs. pancreatitis
multi-way comparisons :
▪ Lung cancer vs. pancreatic cancer vs. multiple sclerosis
▪ Pancreas cancer vs. pancreatitis vs. lung cancer WHO grade 1 non-metastasing

### Prostate Cancer

Prostate cancer is a form of cancer that develops in the prostate, a gland in the male reproductive system. The cancer cells may metastasize (spread) from the prostate to other parts of the body, particularly the bones and lymph nodes. Prostate cancer may cause pain, difficulty in urinating, problems during sexual intercourse, or erectile dysfunction. Other symptoms can potentially develop during later stages of the disease.

Rates of detection of prostate cancers vary widely across the world, with South and East Asia detecting less frequently than in Europe, and especially the United States.Prostate cancer tends to develop in men over the age of fifty and although it is one of the most prevalent types of cancer in men, many never have symptoms, undergo no therapy, and eventually die of other causes. This is because cancer of the prostate is, in most cases, slow-growing, symptom free and men with the condition often die of causes unrelated to the prostate cancer, such as heart/circulatory disease, pneumonia, other unconnected cancers, or old age. Many factors, including genetics and diet, have been implicated in the development of prostate cancer. The presence of prostate cancer may be indicated by symptoms, physical examination, prostate specific antigen (PSA), or biopsy. There is controversy about the accuracy of the PSA test and the value of screening. Suspected prostate cancer is typically confirmed by taking a biopsy of the prostate and examining it under a microscope. Further tests, such as CT scans and bone scans, may be performed to determine whether prostate cancer has spread.

Treatment options for prostate cancer with intent to cure are primarily surgery, radiation therapy, and proton therapy. Other treatments, such as hormonal therapy, chemotherapy, cryosurgery, and high intensity focused ultrasound (HIFU) also exist, depending on the clinical scenario and desired outcome.
The age and underlying health of the man, the extent of metastasis, appearance under the microscope, and response of the cancer to initial treatment are important in determining the outcome of the disease. The decision whether or not to treat localized prostate cancer (a tumour that is contained within the prostate) with curative intent is a patient trade-off between the expected beneficial and harmful effects in terms of patient survival and quality of life.

A "biological sample" in terms of the invention means a sample of biological tissue or fluid. Examples of biological samples are sections of tissues, blood, blood fractions, plasma, serum, urine or samples from other peripheral sources, or cell cultures, cell colonies of even single cells, or a collection of single cells. Furthermore, also pools or mixture of the above mentioned samples may be employed. A biological sample may be provided by removing a sample of cells from a subject, but can also be provided by using a previously isolated sample. For example, a tissue sample can be removed from a subject suspected of having a disease by conventional biopsy techniques. In a preferred embodiment, a blood sample is taken from the subject. In one embodiment, the blood or tissue sample is obtained from the subject prior to initiation of radiotherapy, chemotherapy or other therapeutic treatment. According to the invention, the biological sample preferably is a blood or a serum sample. Further, it is also preferred to use blood cells comprising erythrocytes, leukocytes and thrombocytes.

A biological sample from a patient means a sample from a subject suspected to be affected by a disease. As used herein, the term "subject" refers to any mammal, including both human and other mammals. Preferably, the methods of the present invention are applied to human subjects.

Subject-matter of the invention is a method for diagnosing a disease, comprising the steps
(a) determining an expression profile of a predetermined set of miRNAs in a biological sample from a patient (or subject); and
(b) comparing said expression profile to a reference expression profile,
wherein the comparison of said determined expression profile to said reference expression profile allows for the diagnosis of the disease.

In step (a) of the above method of the invention, an expression profile of a predetermined set of miRNAs is determined. The determination may be carried out by any convenient means for determining nucleic acids. For expression profiling, qualitative, semiquantitative and preferably quantitative detection methods can be used. A variety of techniques are well known to those of skill in the art. In particular, the determination may comprise nucleic acid hybridization and/or nucleic acid amplification steps.

Nucleic acid hybridization may for example be performed using a solid phase nucleic acid biochip array, in particular a microarray, or *in situ* hybridization. The miRNA microarray technology affords the analysis of a complex biological sample for all expressed miRNAs. Nucleotides with complementarity to the corresponding miRNAs are spotted on coated carriers or are fabricated by *in-situ* synthesis methods on a carrier. Preferably, miRNAs isolated from the sample of interest are not labelled, e.g. before hybridization of the miRNAs to the complementary sequences on the carrier and the resulting signal indicating the occurrence of a distinct miRNA is generated by incorporation of a detectable label (e.g. biotin, fluorescent dye) by means of an enzyme reaction.

According to another embodiment of the invention, miRNAs isolated from the sample of interest are labelled, e.g. fluorescently labelled, so that upon hybridization of the miRNAs to the complementary sequences on the carrier the resulting signal indicates the occurrence of a distinct miRNA.
On one miRNA microarray, preferably at least the whole predetermined set of miRNAs can be analyzed.

Further, quantitative real-time polymerase chain reaction (RT-PCR) can be used to detect miRNAs even at very low abundance.

Alternative methods for obtaining expression profiles may also contain sequencing, next generation sequencing or mass spectroscopy.

The predetermined set of miRNAs in step (a) of the above method of the invention depends on the disease to be diagnosed. The inventors found out that single miRNA biomarkers lack sufficient accuracy, specificity and sensitivity, and therefore it is preferred to analyze more complex miRNA expression patterns, so-called miRNA signatures. The predetermined set of miRNAs comprises one or more, preferably a larger number of miRNAs (miRNA signatures) that are differentially regulated in samples of a patient affected by a particular disease compared to healthy controls. Alternatively, the disease can also be compared to any other defined condition (e.g. another disease).

The expression profile determined in the above step (a) is subsequently compared to a reference expression profile or to a plurality of reference profiles in the above step (b). The reference expression profile is the expression profile of the same set of miRNAs in a biological sample originating from the same source as the biological sample from a patient but obtained from a healthy subject. Preferably, both the reference expression profile and the expression profile of the above step (a) are determined in a blood or serum sample or in a sample of erythrocytes, leukocytes and/or thrombocytes. It is understood that the reference expression profile is not necessarily obtained from a single healthy subject but may be an average expression profile of a plurality of healthy subjects. It is preferred to use a reference expression profile obtained from a person of the same gender, and a similar age as the patient.

The above method of the invention is suitable for diagnosing any diseases for which a differential expression of miRNAs compared to healthy controls or other diseases exists. In particular, the method may be used for diagnosing cancer including bladder cancer, brain cancer, breast cancer, colon cancer, endometrium cancer, gastrointestinal stromal cancer, glioma, head- and neck cancer, kidney cancer, leukemia, liver cancer, lung cancer, lymph node cancer, melanoma, meninges cancer, ovarian cancer, pancreas cancer, prostate cancer, sarcoma, stomach cancer, testicular cancer, thyroid cancer, thymus cancer and Wilms' tumour or COPD. The diagnosis may comprise determining type, rate and/or stage of cancer. The course of the disease and the success of therapy such as chemotherapy may be monitored. The method of the invention provides a prognosis on the survivor rate and enables to determine a patient's response to drugs.

In addition to cancer, also different types of diseases may be diagnosed by means of the above method of the invention, if the disease state is correlated with a differential expression of miRNAs compared to a healthy control. For example the disease may be Alzheimer's disease, multiple sclerosis, melanoma, Morbus Crohn and cardiovascular diseases. The inventors found out that also these diseases are correlated with a specific expression profile of miRNAs.

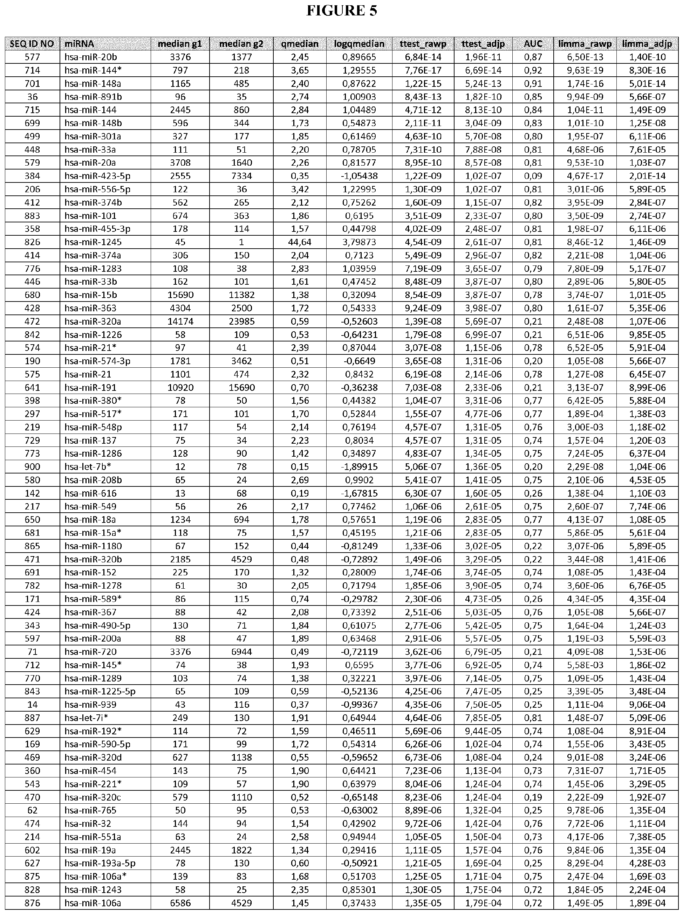

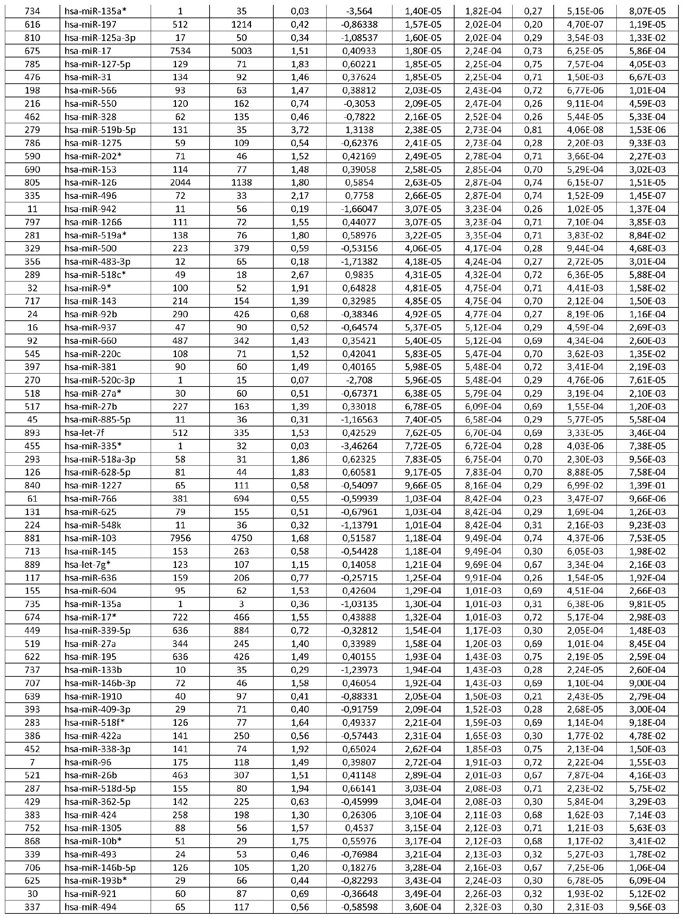

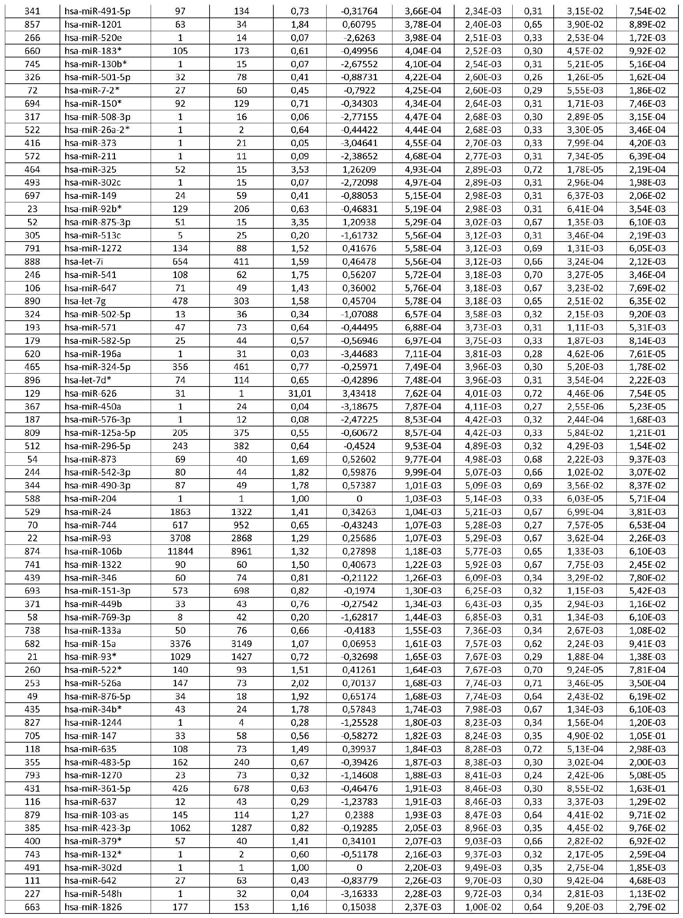

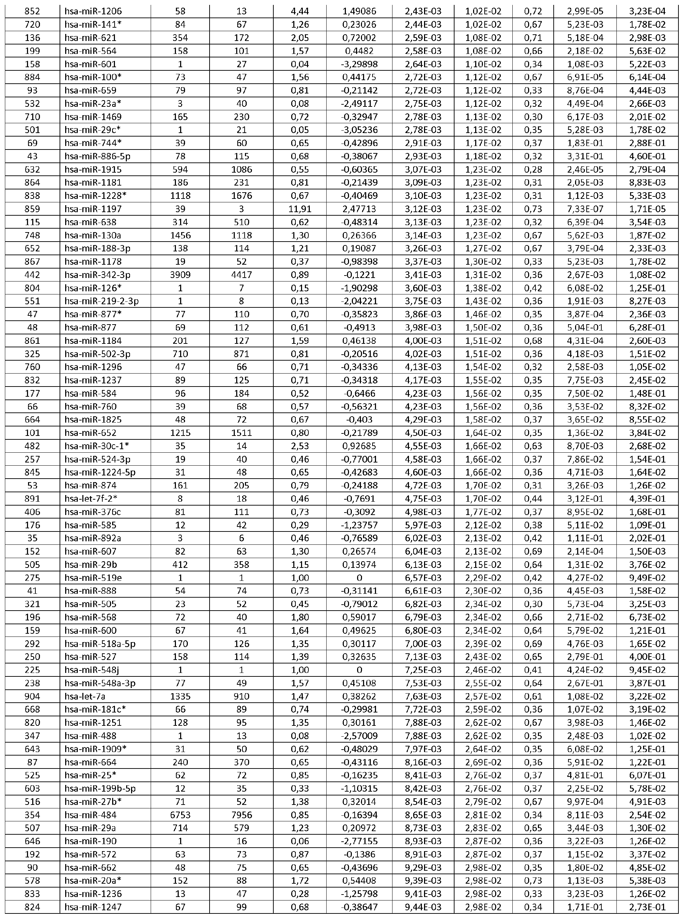

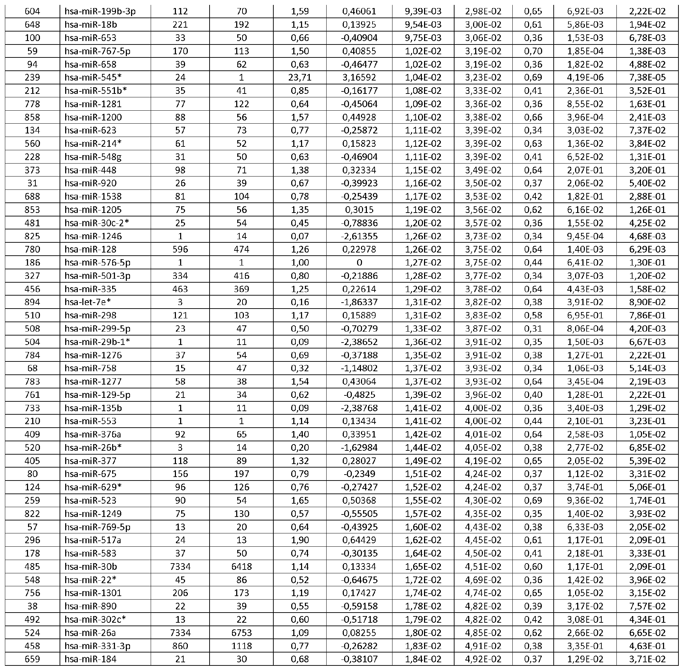

The inventors succeeded in developing a generally applicable approach to arrive at miRNA signatures that are correlated with a particular disease. In more detail, the following steps are accomplished:
1. miRNAs are extracted from a biological sample of a patient, preferably a blood or serum or urine sample or a sample comprising erythrocytes, leukocytes or thrombocytes, using suitable kits / purification methods. From these samples preferably the RNA-fraction is used for analysis.
2. The respective samples are measured using experimental techniques. These techniques include but are not restricted to:
   - Array based approaches
   - Real time quantitative polymerase chain reaction
   - Sequencing
   - Next Generation Sequencing
   - Mass Spectroscopy
3. Mathematical approaches are applied to gather information on the value and the redundancy of single biomarkers. These methods include, but are not restricted to:
   - basic mathematic approaches (e.g. Fold Quotients, Signal to Noise ratios, Correlation)
   - statistical methods as hypothesis tests (e.g. t-test, Wilcoxon-Mann-Whitney test), the Area under the Receiver operator Characteristics Curve
   - Information Theory approaches, (e.g. the Mutual Information, Cross-entropy)
   - Probability theory (e.g. joint and conditional probabilities)
   - Combinations and modifications of the previously mentioned examples
4. The information collected in 3) are used to estimate for each biomarker the diagnostic content or value. Usually, however, this diagnostic value of only one biomarker is too small to get a highly accurate diagnosis with accuracy rates, specificities and sensitivities beyond the 90% barrier.
   Please note that the diagnostic content for our miRNAs can be found in the tables in Figures 2 and 5. These tables includes the miRNAs with the sequences, and the significance value as computed by a t-test and further statistical measures.
5. Thus statistical learning / machine learning / bioinformatics / computational approaches are applied to define subsets of biomarkers that are tailored for the detection of diseases. These techniques includes but are not restricted to
   - Wrapper subset selection techniques (e.g. forward step-wise, backward step-wise, combinatorial approaches, optimization approaches)
   - Filter subset selection methods (e.g. the methods mentioned in 3)
   - Principal Component Analysis
   - Combinations and modifications of such methods (e.g. hybrid approaches)
6. The diagnostic content of each detected set can be estimated by mathematical and/or computational techniques to define the diagnostic information content of subsets.
7. The subsets, detected in step 5, which may range from only a small number (at least two) to all measured biomarkers is then used to carry out a diagnosis. To this end, statistical learning / machine learning / bioinformatics / computational approaches are applied that include but are not restricted to any type of supervised or unsupervised analysis:
   - Classification techniques (e.g. naive Bayes, Linear Discriminant Analysis, Quadratic Discriminant Analysis Neural Nets, Tree based approaches, Support Vector Machines, Nearest Neighbour Approaches)
   - Regression techniques (e.g. linear Regression, Multiple Regression, logistic regression, probit regression, ordinal logistic regression ordinal Probit-Regression, Poisson Regression, negative binomial Regression, multinomial logistic Regression, truncated regression)
   - Clustering techniques (e.g. k-means clustering, hierarchical clustering, PCA)
   - Adaptations, extensions, and combinations of the previously mentioned approaches

The inventors surprisingly found out that the described approach yields in miRNA signatures that provide high diagnostic accuracy, specificity and sensitivity in the determination of diseases.

According to a preferred embodiment of the invention, the disease to be determined is prostate cancer.

The inventors succeeded in determining miRNAs that are differentially regulated in samples from prostate cancer patients as compared to healthy controls. A complete overview of all miRNAs that are found to be differentially regulated in blood samples of prostate cancer patients is provided in the tables shown in Figure 2 and 5.

In the tables shown in Figures 2 and 5, the miRNAs that are found to be differentially regulated are sorted in the order of their t-test significance. Another method for assessing the significance is to compute the Mutual information (MI) (Shannon, 1984) which is an adequate measure to estimate the overall diagnostic information content of single biomarkers (Keller, Ludwig et al., 2006). According to the invention mutual information is considered as the reduction in uncertainty about the class labels "0" for controls and "1" for tumour samples due to the knowledge of the miRNA expression. The higher the value of the MI of a miRNA, the higher is the diagnostic content of the respective miRNA.

### Diagnosis of Prostate Cancer

According to a preferred embodiment of the invention, the disease to be determined is prostate cancer. Surprisingly, the inventors found out that miRNAs are differentially regulated in samples from prostate cancer patients as compared to healthy controls. A complete overview of all miRNAs that are found to be differentially regulated in blood samples of prostate cancer patients is provided in the table shown in Figure 2 and Figure 5. In Figure 2 in total, 241 miRNAs were found to be significantly deregulated (t-test significance <0.05) in blood cells of prostate cancer patients as compared to the healthy controls.

Preferably, the predetermined set of miRNAs for the diagnosis of prostate cancer comprises one or more nucleic acids selected from the deregulated miRNAs presented in the tables in Figure 2 or Figure 5.

The predetermined set of miRNAs should preferably comprise at least 1, preferably at least 7, 10 ,15 ,20, 25, 30, 35, 40, 50, 75 or 100 of the indicated nucleic acids. It is particularly preferred to include the 100, 75, 50, 40, 35, 30, 25, 20, 15, 10 or at least 7 of the first mentioned miRNAs according to their order in the tables in Figure 2 or Figure 5.

Thus, preferably the predetermined set of miRNAs for the diagnosis of prostate cancer comprises one or more nucleic acids selected from the 241 most deregulated miRNAs.

Preferably, the predetermined set of miRNAs comprises at least 7, preferably at least 10, 15, 20, 25, 30, 35, 40, 50, 75, 100 or all of the above-indicated nucleic acids.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195, hsa-miR-33b, hsa-miR-1283, hsa-miR-519c-5p, hsa-miR-497, hsa-miR-9*.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195, hsa-miR-33b, hsa-miR-1283, hsa-miR-519c-5p, hsa-miR-497, hsa-miR-9*, hsa-miR-200a, hsa-miR-338-3p, hsa-miR-515-5p, hsa-miR-31*, hsa-miR-551b*.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195, hsa-miR-33b, hsa-miR-1283, hsa-miR-519c-5p, hsa-miR-497, hsa-miR-9*, hsa-miR-200a, hsa-miR-338-3p, hsa-miR-515-5p, hsa-miR-31*, hsa-miR-551b*, hsa-miR-518e*, hsa-miR-127-5p, hsa-miR-21*, hsa-miR-216a, hsa-miR-452*.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195, hsa-miR-33b, hsa-miR-1283, hsa-miR-519c-5p, hsa-miR-497, hsa-miR-9*, hsa-miR-200a, hsa-miR-338-3p, hsa-miR-515-5p, hsa-miR-31*, hsa-miR-551b*, hsa-miR-518e*, hsa-miR-127-5p, hsa-miR-21*, hsa-miR-216a, hsa-miR-452*, hsa-miR-183*, hsa-miR-500, hsa-miR-1826, hsa-miR-625*, hsa-miR-513b.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195, hsa-miR-33b, hsa-miR-1283, hsa-miR-519c-5p, hsa-miR-497, hsa-miR-9*, hsa-miR-200a, hsa-miR-338-3p, hsa-miR-515-5p, hsa-miR-31*, hsa-miR-551b*, hsa-miR-518e*, hsa-miR-127-5p, hsa-miR-21*, hsa-miR-216a, hsa-miR-452*, hsa-miR-183*, hsa-miR-500, hsa-miR-1826, hsa-miR-625*, hsa-miR-513b, hsa-miR-526a, hsa-miR-33a, hsa-miR-1243, hsa-miR-517*, hsa-miR-541.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195, hsa-miR-33b, hsa-miR-1283, hsa-miR-519c-5p, hsa-miR-497, hsa-miR-9*, hsa-miR-200a, hsa-miR-338-3p, hsa-miR-515-5p, hsa-miR-31*, hsa-miR-551b*, hsa-miR-518e*, hsa-miR-127-5p, hsa-miR-21*, hsa-miR-216a, hsa-miR-452*, hsa-miR-183*, hsa-miR-500, hsa-miR-1826, hsa-miR-625*, hsa-miR-513b, hsa-miR-526a, hsa-miR-33a, hsa-miR-1243, hsa-miR-517*, hsa-miR-541, hsa-miR-217, hsa-miR-621, hsa-miR-518d-5p, hsa-miR-873, hsa-miR-103-as, hsa-miR-450b-5p, hsa-miR-545, hsa-miR-1251, hsa-miR-885-5p, hsa-miR-922.

In a further embodiment the predetermined set of miRNAs for the diagnosis of prostate cancer comprises one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195, hsa-miR-33b, hsa-miR-1283, hsa-miR-519c-5p, hsa-miR-497, hsa-miR-9*, hsa-miR-200a, hsa-miR-338-3p, hsa-miR-515-5p, hsa-miR-31*, hsa-miR-551b*, hsa-miR-518e*, hsa-miR-127-5p, hsa-miR-21*, hsa-miR-216a, hsa-miR-452*, hsa-miR-183*, hsa-miR-500, hsa-miR-1826, hsa-miR-625*, hsa-miR-513b, hsa-miR-526a, hsa-miR-33a, hsa-miR-1243, hsa-miR-517*, hsa-miR-541, hsa-miR-217, hsa-miR-621, hsa-miR-518d-5p, hsa-miR-873, hsa-miR-103-as, hsa-miR-450b-5p, hsa-miR-545, hsa-miR-1251, hsa-miR-885-5p, hsa-miR-922, hsa-miR-628-5p, hsa-miR-548f, hsa-miR-802, hsa-miR-25, hsa-miR-423-3p, hsa-miR-522*, hsa-miR-519a*, hsa-miR-455-3p, hsa-miR-1245, hsa-miR-362-5p, hsa-miR-1184, hsa-miR-191, hsa-miR-487a, hsa-miR-216b, hsa-miR-525-5p, hsa-miR-509-3-5p, hsa-miR-27a*, hsa-miR-488*, hsa-miR-1226, hsa-miR-646, hsa-miR-527, hsa-miR-635, hsa-miR-1825, hsa-let-7i*.

Most preferably, the predetermined set of miRNAs comprises those miRNAs that were most significantly deregulated.

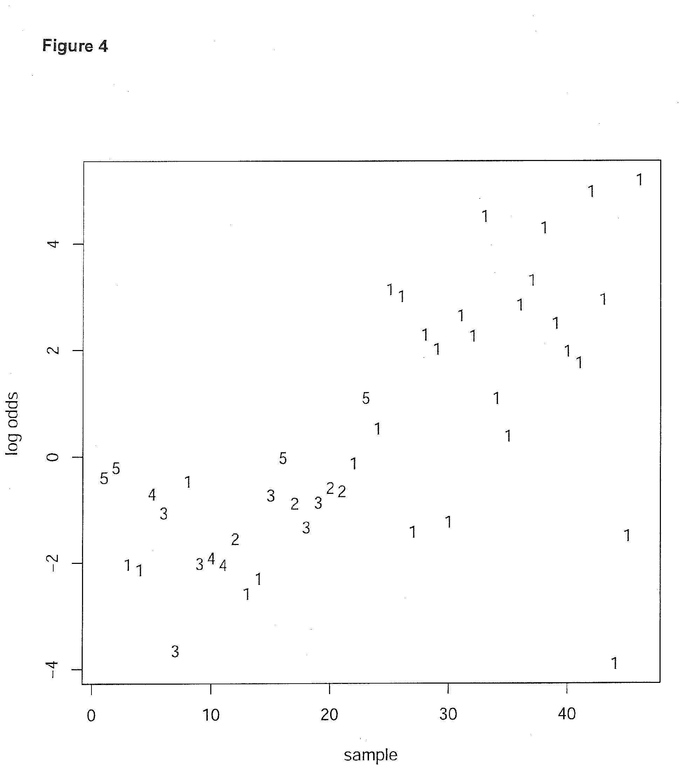

In a further embodiment, the measured miRNA profiles were classified using statistical learning approaches in order to compute accuracy, specificity, and sensitivity for the diagnosis of prostate cancer (see Figure 4). The miRNAs that performed best for the diagnosis of prostate cancer according to their accuracy, specificity, and sensitivity are the 270 miRNAs shown in Table in Figure 2 (entries No. 1-270) leading to an accuracy 82.8 %, a specificity of 87.5 % and a sensitivity of 71.9 %.

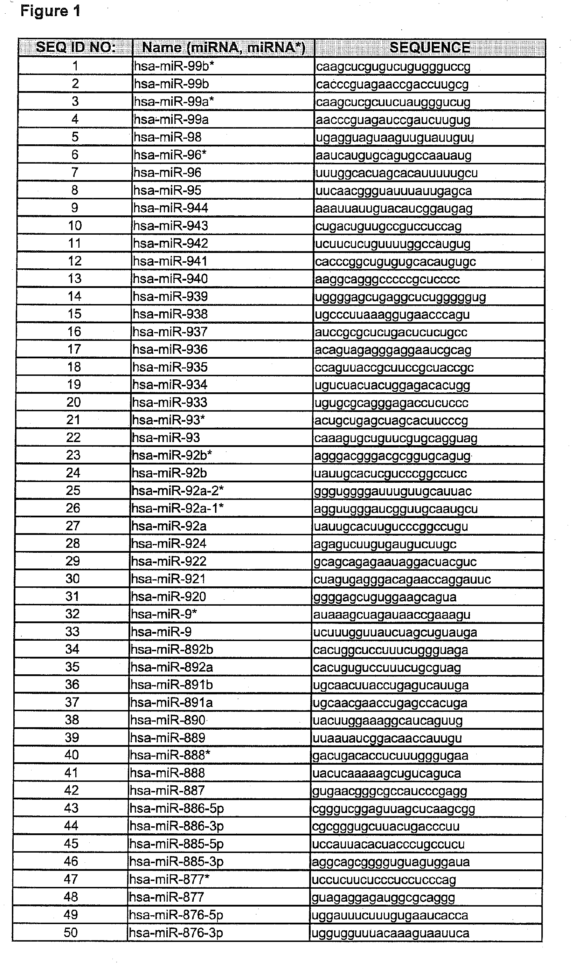

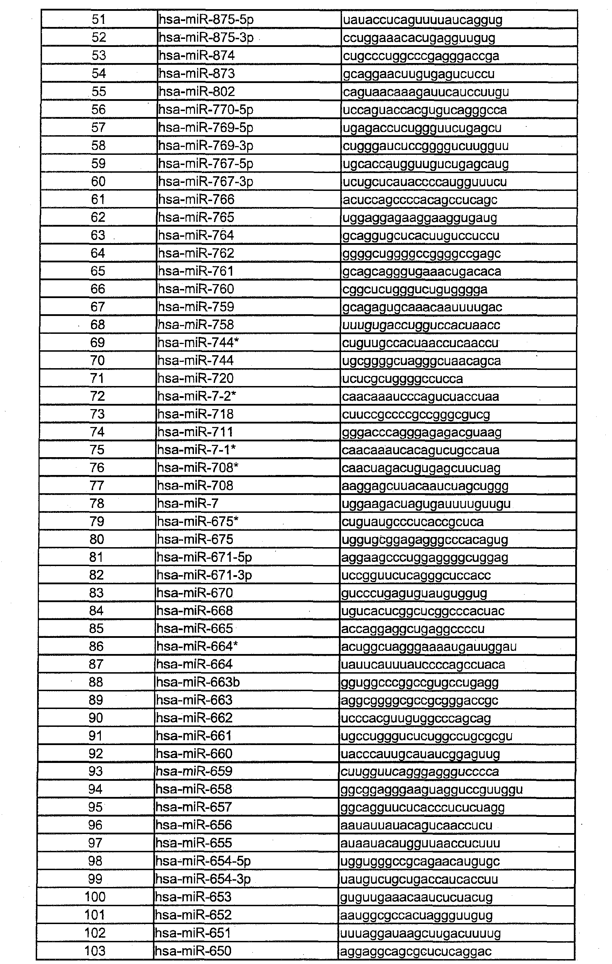

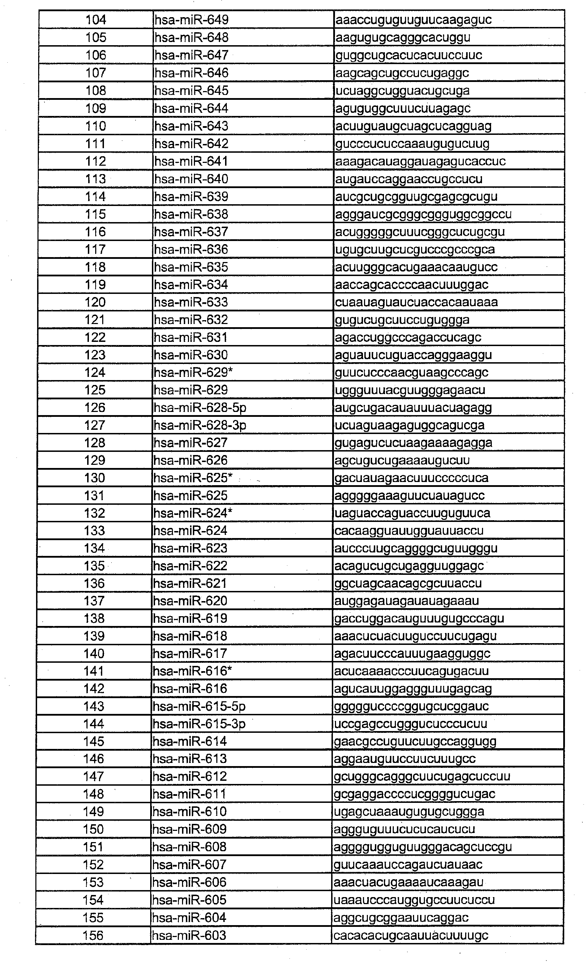

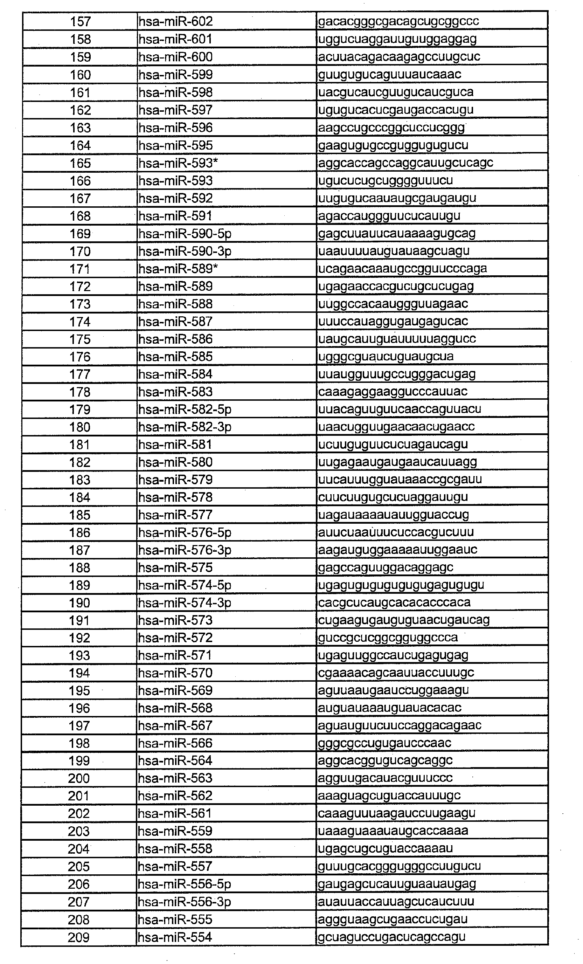

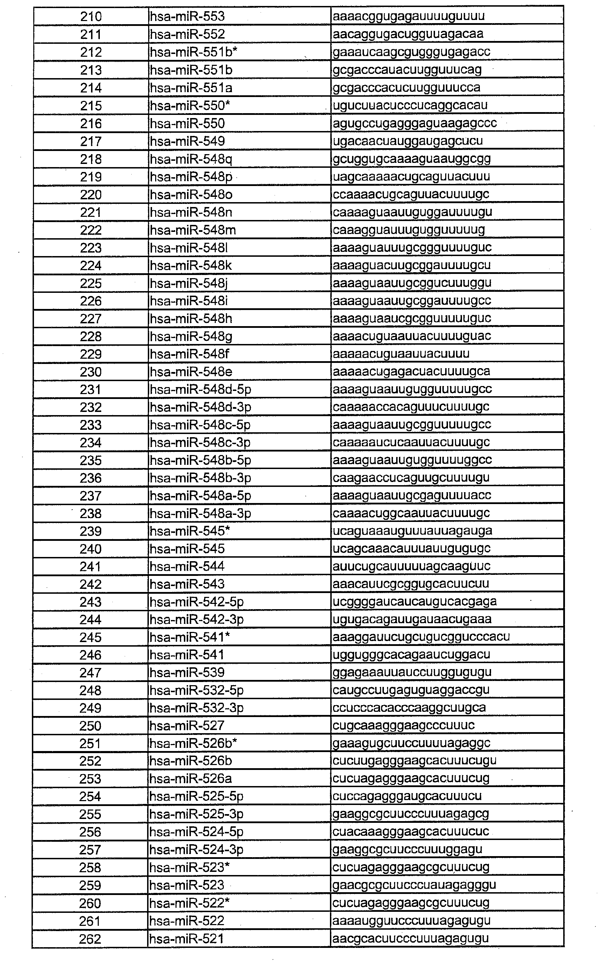

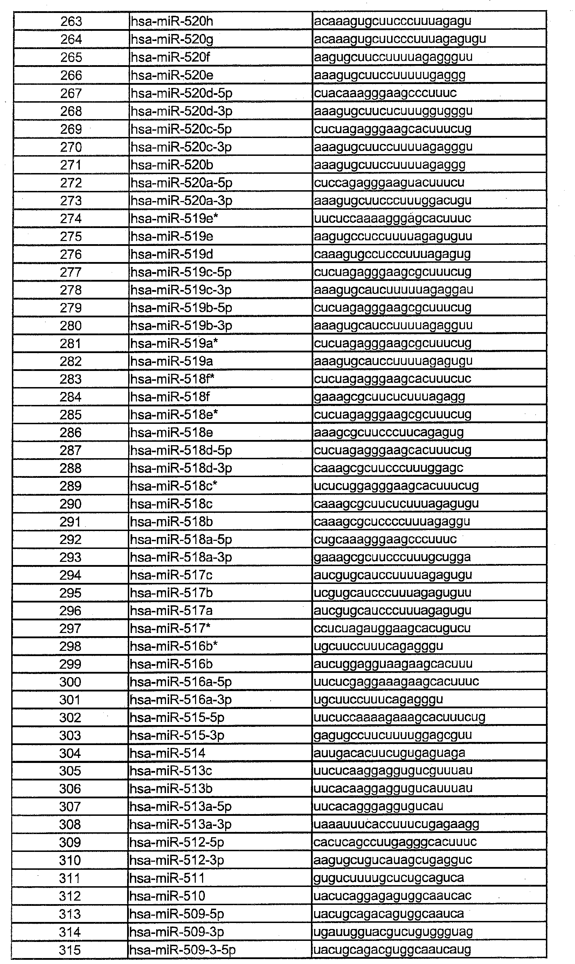

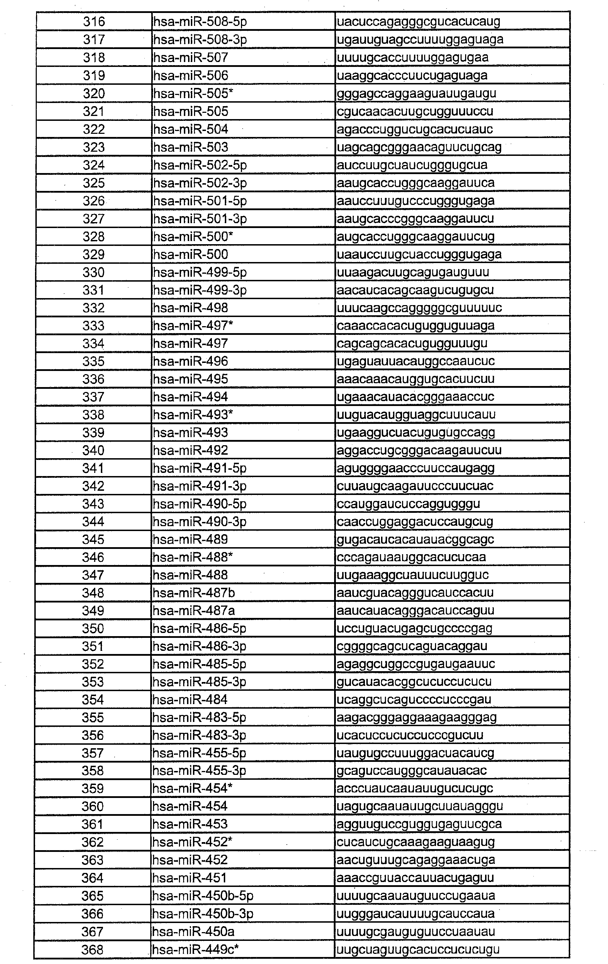

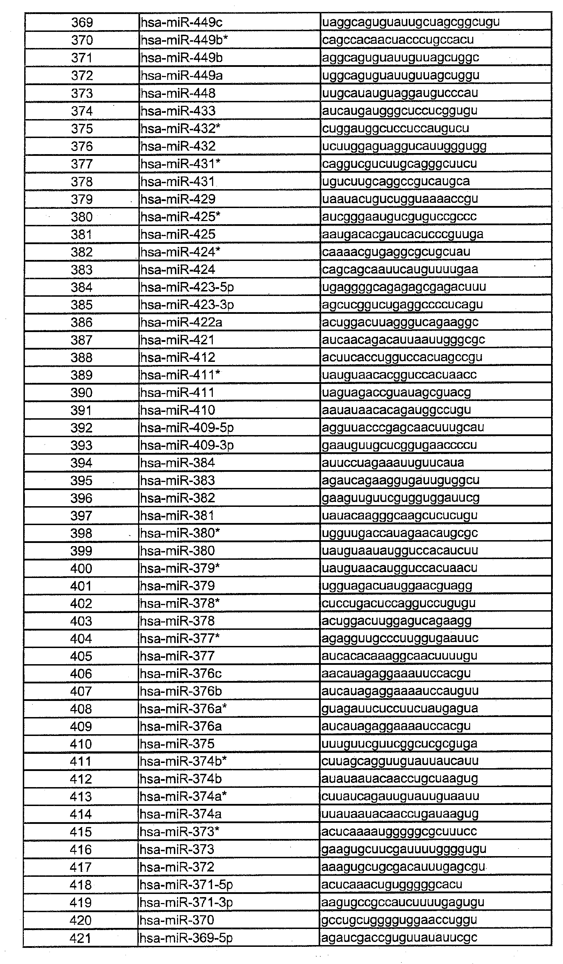

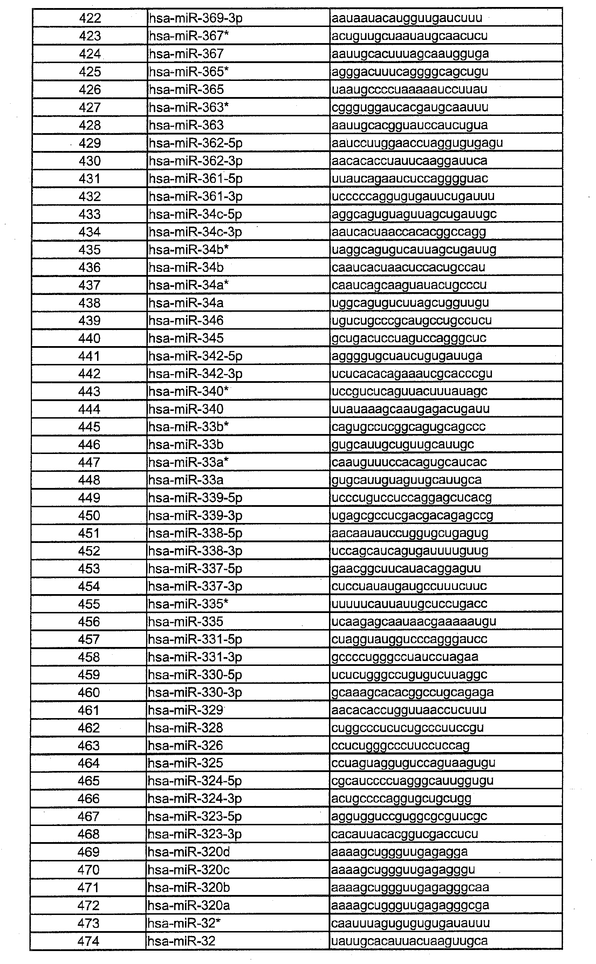

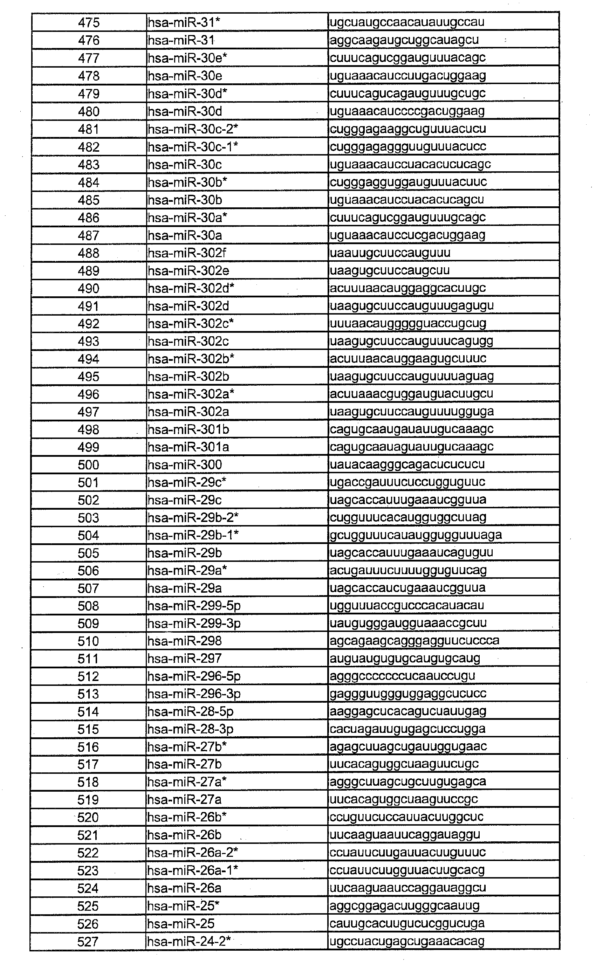

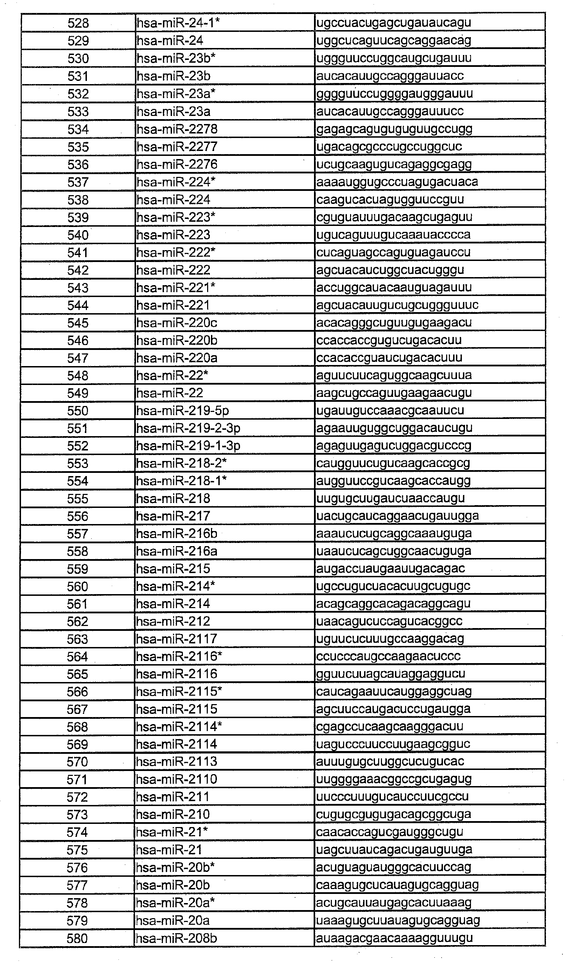

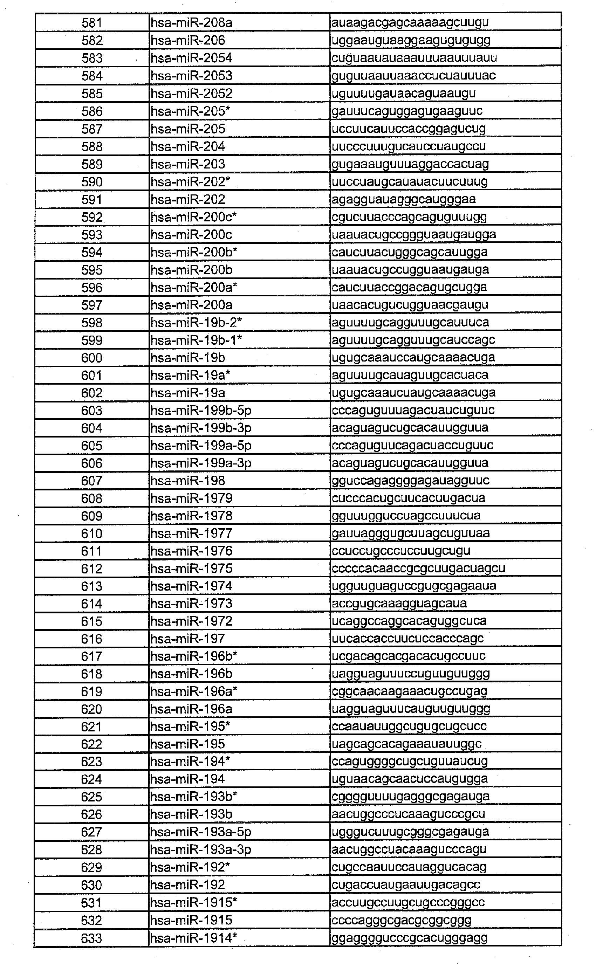

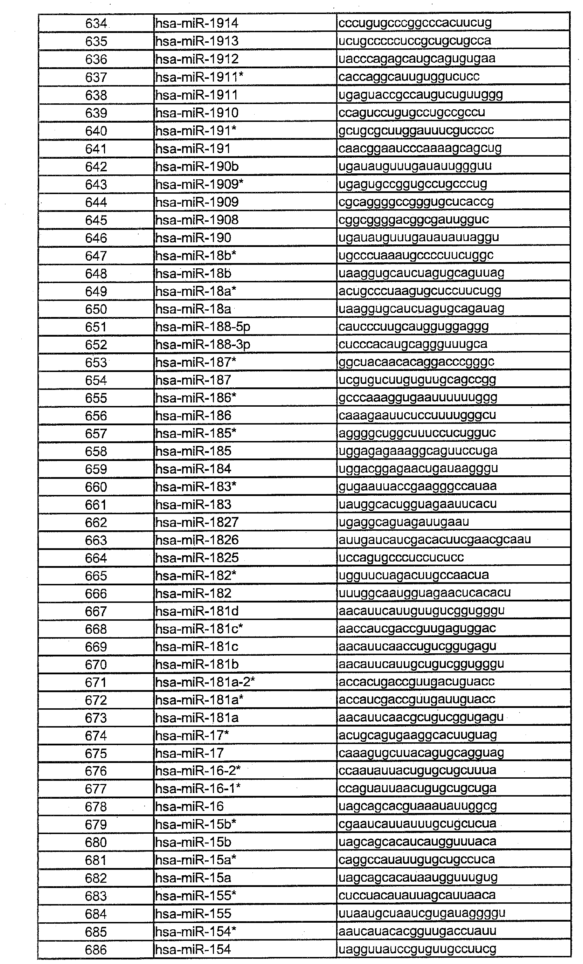

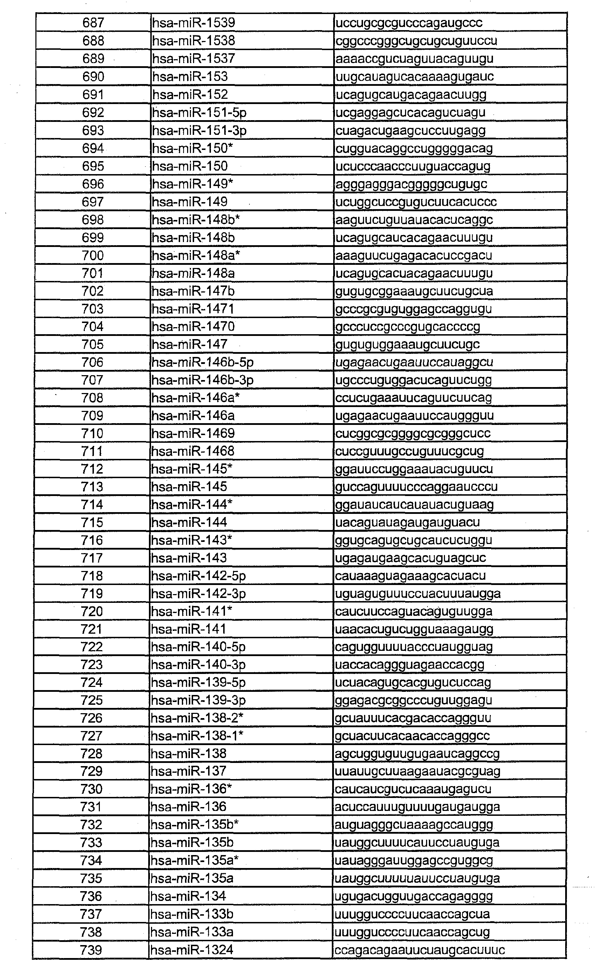

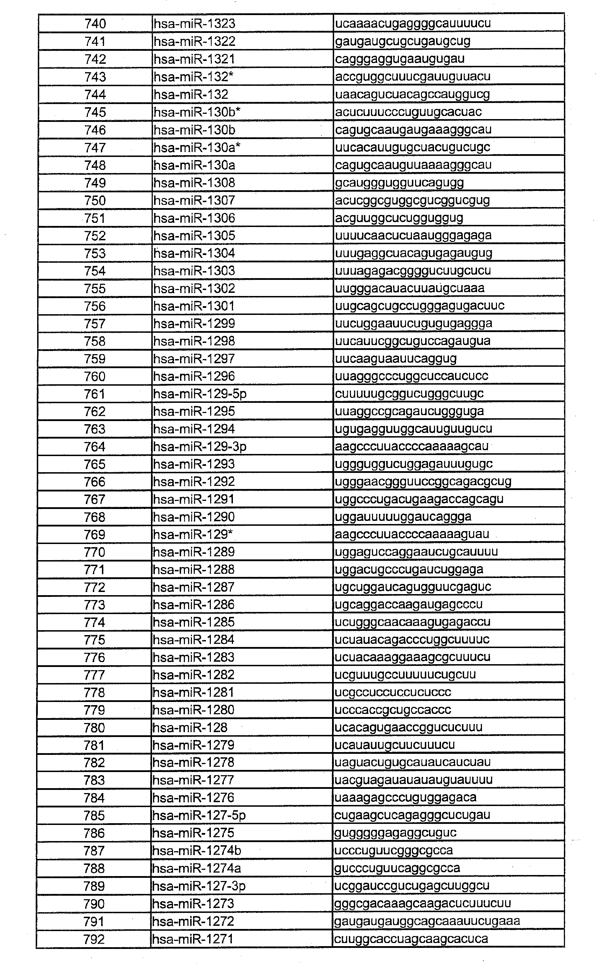

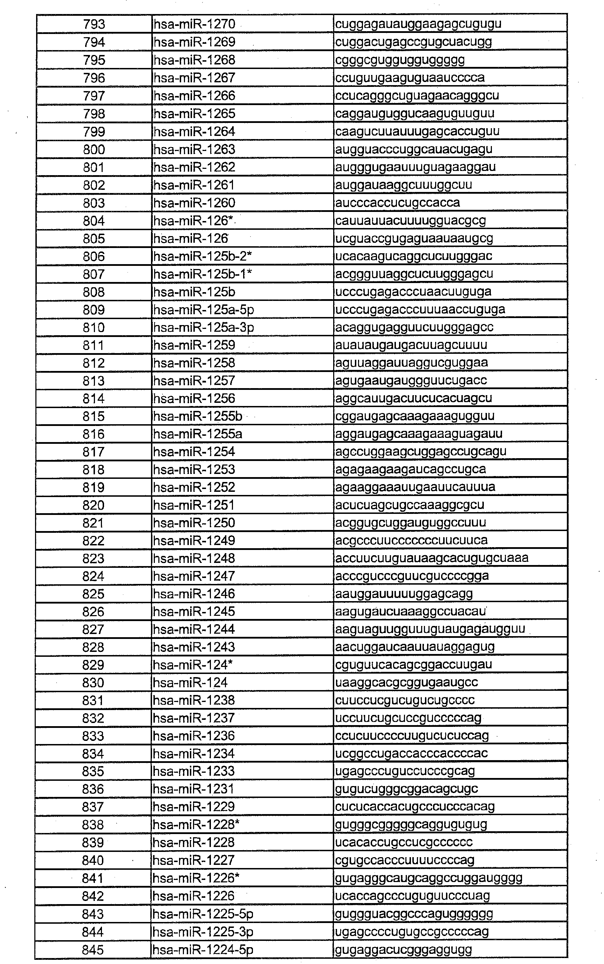

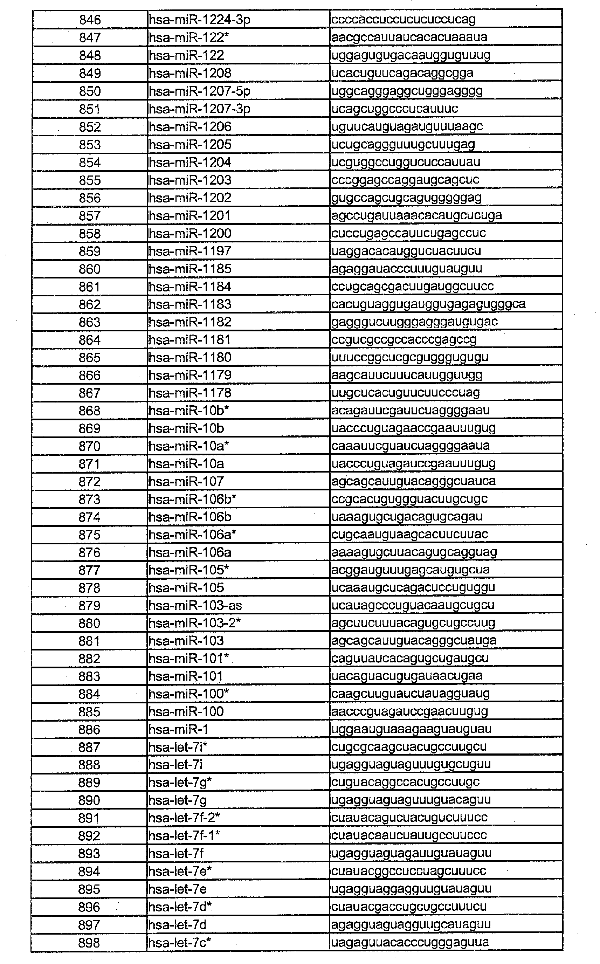

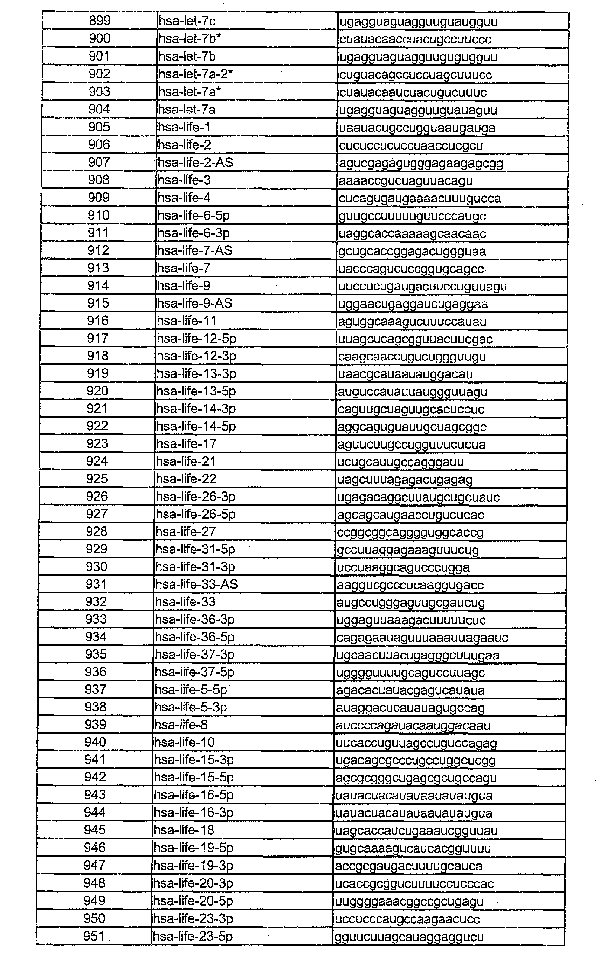

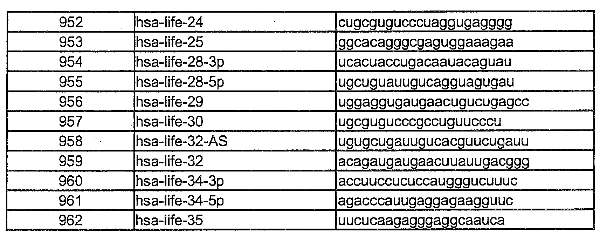

The predetermined set of miRNAs for the diagnosis of prostate cancer should preferably comprise at least 7, preferably at least 10, 15, 20, 25, 30, 35, 40, 50, 75, 100, preferably all of the known miRNAs, preferably all of the 863 (see Figure 1, representing the current status of all known miRNAs in the version 12, 13, and 14 of the miRBase repository (www.mirbase.org).

Another embodiment of the present invention is a kit for diagnosing a disease, comprising means for determining an expression profile of a predetermined set of miRNAs in a biological sample, in particular in a blood and/or serum sample. Preferably, one or more reference expression profiles are also provided which show the expression profile of the same set of miRNAs in the same type of biological sample, in particular in a blood and/or serum sample, obtained from one or more healthy subjects. A comparison to said reference expression profile(s) allows for the diagnosis of the disease.

Another preferred embodiment of the present invention is a kit for diagnosing prostate cancer, comprising means for determining the expression profile of one or more miRNAs presented in the table in Figure 2, preferably one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195, hsa-miR-33b, hsa-miR-1283, hsa-miR-519c-5p, hsa-miR-497, hsa-miR-9*, hsa-miR-200a, hsa-miR-338-3p, hsa-miR-515-5p, hsa-miR-31*, hsa-miR-551b*, hsa-miR-518e*, hsa-miR-127-5p, hsa-miR-21*, hsa-miR-216a, hsa-miR-452*, hsa-miR-183*, hsa-miR-500, hsa-miR-1826, hsa-miR-625*, hsa-miR-513b, hsa-miR-526a, hsa-miR-33a, hsa-miR-1243, hsa-miR-517*, hsa-miR-541, hsa-miR-217, hsa-miR-621, hsa-miR-518d-5p, hsa-miR-873, hsa-miR-103-as, hsa-miR-450b-5p, hsa-miR-545, hsa-miR-1251, hsa-miR-885-5p, hsa-miR-922.

In a preferred embodiment the kit comprises means for determining at least 7, preferably at least 10, 15, 20, 25, 30, 35, 40, 50, 75, 100 or all of the indicated miRNAs. It is particularly preferred to include means for determining the 100, 75, 50, 45 ,40 ,35, 30, 25, 20, 15, 10 or at least 7 first mentioned miRNAs in the order of their diagnostic significance as represented by their order in the table in Figure 2. The kit for diagnosing prostate cancer is particularly suitable for diagnosing prostate cancer in a blood and/or serum sample or in a sample comprising erythrocytes, leukocytes and/or thrombocytes.

The means for determining a predetermined set of miRNAs may for example comprise a microarray comprising miRNA-specific oligonucleotide probes. In a preferred embodiment, the microarray comprises miRNA-specific oligonucleotide probes for the detection of miRNAs. Depending on the intended use of the microarray in the diagnosis or prognosis of a particular disease, probes for detecting different miRNAs may be included.

A microarray intended for use in the diagnosis of prostate cancer preferably comprises miRNA specific oligonucleotide probes for one or more miRNAs presented in the table in Figure 2, preferably for one or more miRNAs selected from the group consisting of hsa-miR-144*, hsa-miR-148a, hsa-miR-519b-5p, hsa-miR-1324, hsa-miR-137, hsa-miR-556-5p, hsa-miR-330-3p, hsa-miR-361-5p, hsa-miR-891b, hsa-miR-767-5p, hsa-miR-744*, hsa-miR-208b, hsa-miR-548p, hsa-miR-20a*, hsa-miR-195, hsa-miR-33b, hsa-miR-1283, hsa-miR-519c-5p, hsa-miR-497, hsa-miR-9*, hsa-miR-200a, hsa-miR-338-3p, hsa-miR-515-5p, hsa-miR-31*, hsa-miR-551b*, hsa-miR-518e*, hsa-miR-127-5p, hsa-miR-21*, hsa-miR-216a, hsa-miR-452*, hsa-miR-183*, hsa-miR-500, hsa-miR-1826, hsa-miR-625*, hsa-miR-513b, hsa-miR-526a, hsa-miR-33a, hsa-miR-1243, hsa-miR-517*, hsa-miR-541, hsa-miR-217, hsa-miR-621, hsa-miR-518d-5p, hsa-miR-873, hsa-miR-103-as, hsa-miR-450b-5p, hsa-miR-545, hsa-miR-1251, hsa-miR-885-5p, hsa-miR-922.

In a preferred embodiment the microarray comprises oligonucleotide probes for determining at least 7, preferably at least 10, 15, 20, 25, 30, 35, 40, 50, 75, 100 or all of the indicated miRNAs. It is particularly preferred to include oligonucleotide probes for determining the most significant miRNAs, which is represented by their order in the table depicted in Figure 2.

The microarray can comprise oligonucleotide probes obtained from known or predicted miRNA sequences. The array may contain different oligonucleotide probes for each miRNA, for example one containing the active mature sequence and another being specific for the precursor of the miRNA. The array may also contain controls such as one or more sequences differing from the human orthologs by only a few bases, which can serve as controls for hybridization stringency conditions. It is also possible to include viral miRNAs or putative miRNAs as predicted from bioinformatic tools. Further, it is possible to include appropriate controls for non-specific hybridization on the microarray.
In summary, the present invention is composed of the following items:

So far, miRNAs have been extensively studied in tissue material. It has been found that miRNAs are expressed in a highly tissue-specific manner. Disease-specific expression of miRNAs have been reported in many human cancers employing primarily tissue material as the miRNA source. In this context miRNAs expression profiles were found to be useful in identifying the tissue of origin for cancers of unknown primary origin. Since recently it is known that miRNAs are not only present in tissues but also in other body fluid samples, including human blood. Nevertheless, the mechanism why miRNAs are found in body fluids, especially in blood, or their function in these body fluids is not understood yet.

Various miRNA biomarkers found in tissue material have been proposed to be correlated with certain diseases, e.g. cancer. However, there is still a need for novel miRNAs as biomarkers for the detection and/or prediction of these and other types of diseases. Especially desirable are non-invasive biomarkers, that allow for quick, easy and cost-effective diagnosis/prognosis which cause only minimal stress for the patient eliminating the need for surgical intervention.

Particularly, the potential role of miRNAs as non-invasive biomarkers for the diagnosis and/or prognosis of prostate cancer has not been systematically evaluated yet. In addition, many of the miRNA biomarkers presently available for diagnosing and/or prognosing of diseases have shortcomings such as reduced sensitivity, not sufficient specificity or do not allow timely diagnosis or represent invasive biomarkers. Accordingly, there is still a need for novel and efficient miRNAs or sets of miRNAs as markers, effective methods and kits for the non-invasive diagnosis and/or prognosis of diseases such as prostate cancer.

The inventors of the present invention assessed for the first time the expression of miRNAs on a whole-genome level in subjects with prostate cancer as non-invasive biomarkers from body fluids, preferably in blood. They surprisingly found that miRNAs are significantly dysregulated in blood of prostate cancer subjects in comparison to healthy controls and thus, miRNAs are appropriated non-invasive biomarkers for diagnosing and/or prognosing of prostate cancer. This finding is surprising, since there is nearly no overlap of the miRNA biomarkers found in blood and the miRNA biomarkers found in tissue material representing the origin of the disease. The inventors of the present invention surprisingly found miRNA biomarkers in body fluids, especially in blood, that have not been found to be correlated to prostate cancer when tissues material was used for this kind of analysis. Therefore, the inventors of the invention identified for the first time miRNAs as non-invasive surrogate biomarkers for diagnosis and/or prognosis of prostate cancer. The inventors of the present invention identified single miRNAs which predict prostate cancer with high specificity, sensitivity and accuracy. The inventors of the present invention also pursued a multiple biomarker strategy, thus implementing sets of miRNA biomarkers for diagnosing and/or prognosing of prostate cancer leading to added specificity, sensitivity, accuracy and predictive power, thereby circumventing the limitations of single biomarker. In detail, by using a machine learning algorithms, they identified unique sets of miRNAs (miRNA signatures) that allow for non-invasive diagnosis of prostate cancer with even higher power, indicating that sets of miRNAs (miRNA signatures) derived from a body fluid sample, such as blood from a subject (e.g. human) can be used as novel non-invasive biomarkers.

The inventors of the present invention surprisingly found that miRNAs are significantly dysregulated in body fluid samples such as blood of prostate cancer subjects in comparison to a cohort of controls (healthy subjects) and thus, miRNAs are appropriated biomarkers for diagnosing and/or prognosing of prostate cancer in a non-invasive fashion. Furthermore, the predetermined sets of miRNAs of the present invention lead to high performance in diagnosing and/or prognosing of prostate cancer, thus expose very high specificity, sensitivity and accuracy. They succeeded in determining the miRNAs that are differentially regulated in body fluid samples from patients having prostate cancer compared to a cohort of controls (healthy subjects) (see experimental section for experimental details). Additionally, the inventors of the present invention performed hypothesis tests (e.g. t-test, limma-test) or other measurements (e.g. AUC, mutual information) on the expression level of the found miRNAs, in all controls (healthy subjects) and subjects suffering from prostate cancer. These tests resulted in a significance value (p-value) for each miRNA. This p-value is a measure for the diagnostic power of each of these single miRNAs to discriminate, for example, between the two clinical conditions: controls (healthy subjects), i.e. not suffering from prostate cancer, or diseased, i.e. suffering from prostate cancer. Since a manifold of tests are carried out, one for each miRNA, the p-values may be too optimistic and, thus, over-estimate the actual discriminatory power. Hence, the p-values are corrected for multiple testing by the Benjamini Hochberg approach.

The term "body fluid sample", as used in the context of the present invention, refers to liquids originating from the body of a subject. Said body fluid samples include, but are not limited to, blood, urine, sputum, breast milk, cerebrospinal fluid, cerumen (earwax), endolymph, perilymph, gastric juice, mucus, peritoneal fluid, pleural fluid, saliva, sebum (skin oil), semen, sweat, tears, vaginal secretion, vomit including components or fractions thereof. Said body fluid samples may be mixed or pooled, e.g. a body fluid sample may be a mixture of blood and urine samples or blood and tissue material. A "body fluid sample" may be provided by removing a body liquid from a subject, but may also be provided by using previously isolated sample material. Preferably, the body fluid sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml. In the context of the present invention said "body fluid sample" allows for a non-invasive diagnosis/and or prognosis of a subject.

The term "blood sample", as used in the context of the present invention, refers to a blood sample originating from a subject. The "blood sample" may be derived by removing blood from a subject by conventional blood collecting techniques, but may also be provided by using previously isolated and/or stored blood samples. For example a blood sample may be whole blood, plasma, serum, PBMC (peripheral blood mononuclear cells), blood cellular fractions including red blood cells (erythrocytes), white blood cells (leukocytes), platelets (thrombocytes), or blood collected in blood collection tubes (e.g. EDTA-, heparin-, citrate-, PAXgene- , Tempus-tubes) including components or fractions thereof. For example, a blood sample may be taken from a subject suspected to be affected or to be suspected to be affected by prostate cancer, prior to initiation of a therapeutic treatment, during the therapeutic treatment and/or after the therapeutic treatment. Preferably, the blood sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml. In the context of the present invention said "body fluid sample" allows for a non-invasive diagnosis/and or prognosis of a subject.

Preferably, when the blood sample is collected from the subject the RNA-fraction, especially the the miRNA fraction, is guarded against degradation. For this purpose special collection tubes (e.g. PAXgene RNA tubes from Preanalytix, Tempus Blood RNA tubes from Applied Biosystems) or additives (e.g. RNAlater from Ambion, RNAsin from Promega) that stabilize the RNA fraction and/or the miRNA fraction are employed.

The biological sample, preferably the body fluid sample may be from a subject (e.g. human or mammal) that has been therapeutically treated or that has not been therapeutically treated. In one embodiment, the therapeutical treatment is monitored on the basis of the detection of the miRNA or set of miRNAs by the polynucleotide or set of polynucleotides of the invention. It is also preferred that total RNA or a subfraction thereof, isolated (e.g. extracted) from a biological sample of a subject (e.g. human or animal), is used for detecting the miRNA or set of miRNAs by the polynucleotide or set of polynucleotides or primer pairs of the invention.

The term "non-invasive", as used in the context of the present invention, refers to methods for obtaining a biological sample, particularly a body fluid sample, without the need for an invasive surgical intervention or invasive medical procedure. In the context of the present invention, a blood drawn represents a non-invasive procedure, therefore a blood-based test (utilizing blood or fractions thereof) is a non-invasive test. Other body fluid samples for non-invasive tests are e.g. urine, sputum, tears, mothers mild, cerumen, sweat, saliva, vaginal secretion, vomit, etc.

The term "diagnosis" as used in the context of the present invention refers to the process of determining a possible disease or disorder and therefore is a process attempting to define the (clinical) condition of a subject. The determination of the expression level of a set of miRNAs according to the present invention correlates with the (clinical) condition of a subject. Preferably, the diagnosis comprises (i) determining the occurrence/presence of prostate cancer, (ii) monitoring the course of prostate cancer, (iii) staging of prostate cancer, (iv) measuring the response of a patient with prostate cancer to therapeutic intervention, and/or (v) segmentation of a subject suffering from prostate cancer.

The term "prognosis" as used in the context of the present invention refers to describing the likelihood of the outcome or course of a disease or a disorder. Preferably, the prognosis comprises (i) identifying of a subject who has a risk to develop prostate cancer, (ii) predicting/estimating the occurrence, preferably the severity of occurrence of prostate cancer, and/or(iii) predicting the response of a subject with prostate cancer to therapeutic intervention.

The term "suffering or suspected to be suffering from prostate cancer" as used in the context of the present invention comprises the diagnosis and/or prognosis of prostate cancer in a suspect as defined above.

In a first aspect, the present invention relates to a method for diagnosing and/or prognosing of prostate cancer comprising the steps of:
(i) determining an expression profile of a predetermined set comprising at least two miRNAs representative for prostate cancer in a body fluid sample from a subject, and
(ii) comparing said expression profile to a reference expression profile, wherein the comparison of said expression profile to said reference expression profile allows for the diagnosis and/or prognosis of prostate cancer.

It is preferred that the body fluid sample is a blood sample, particularly preferred it is a whole blood, PBMC, serum or plasma sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

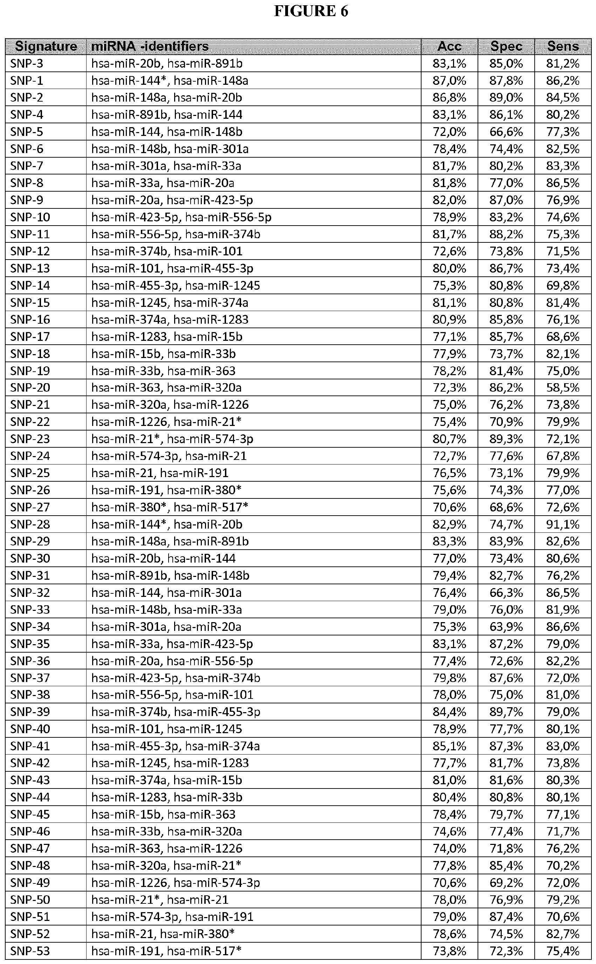

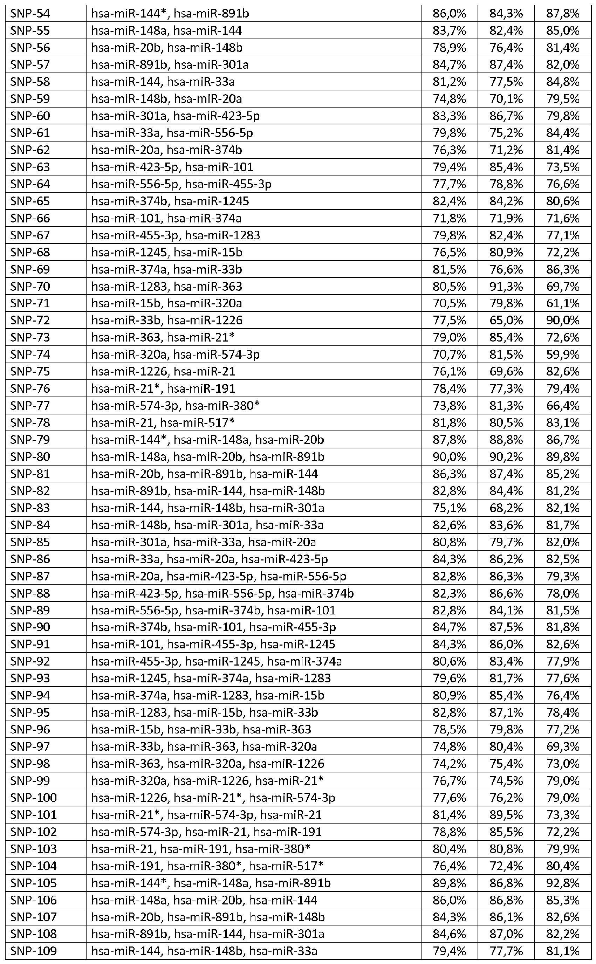

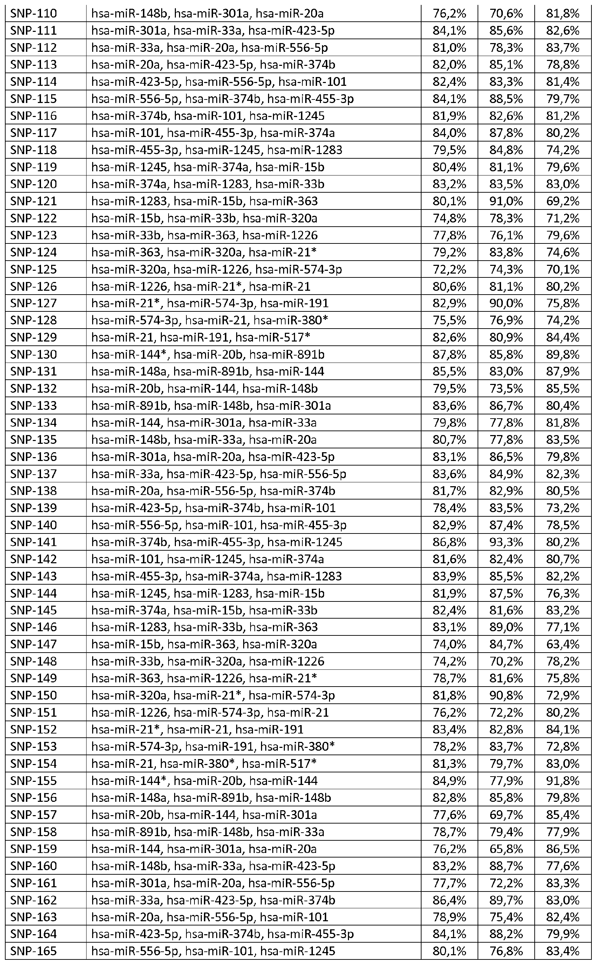

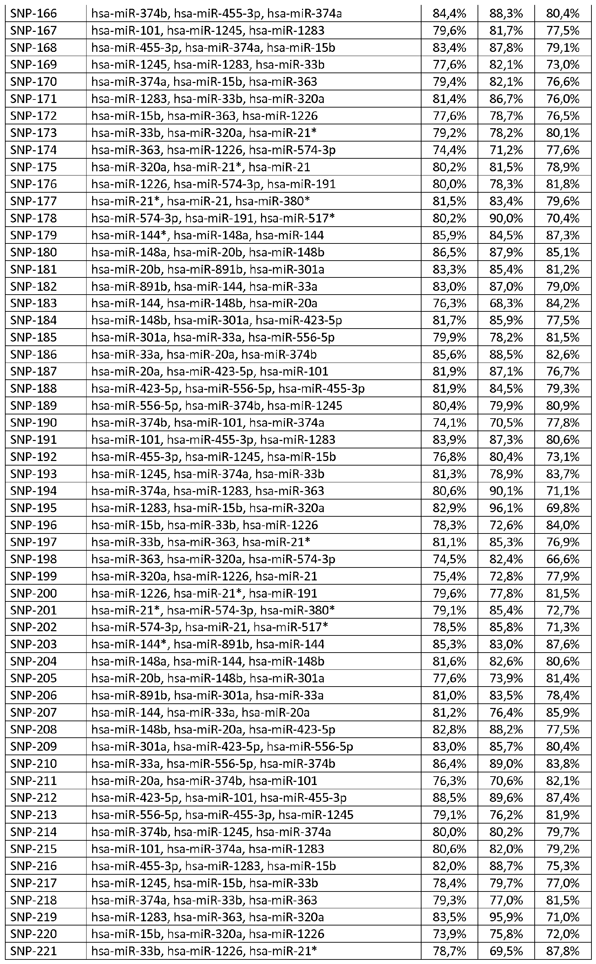

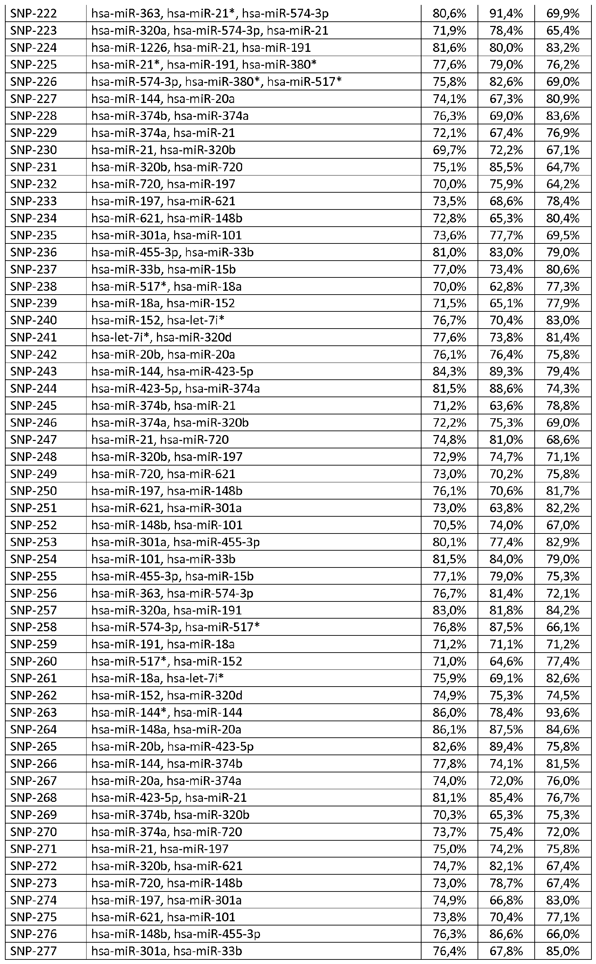

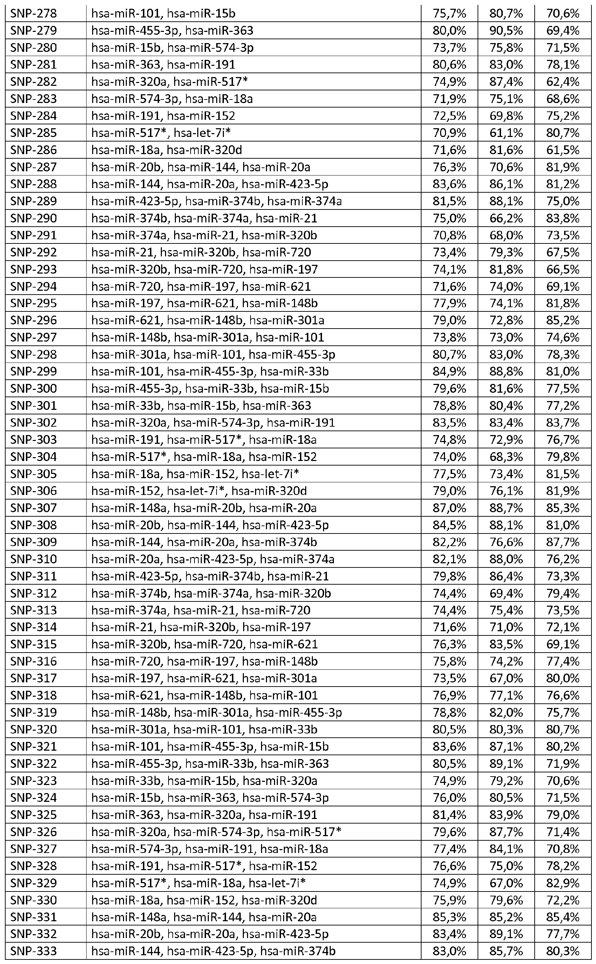

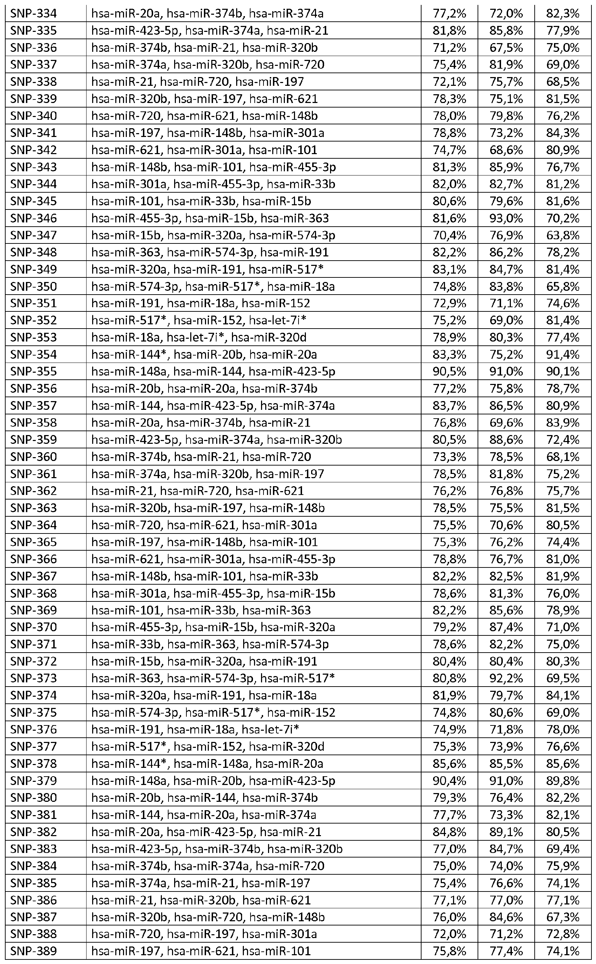

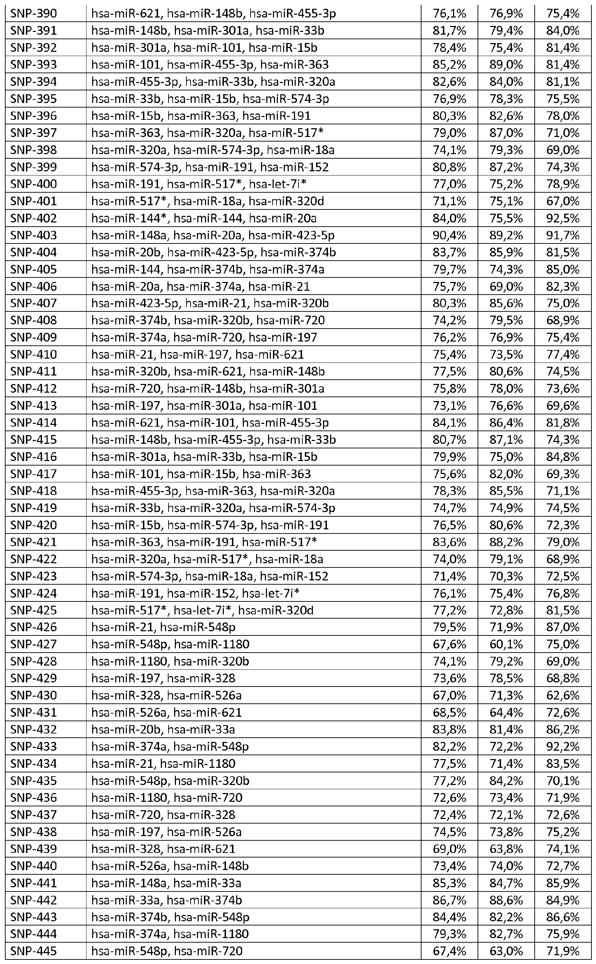

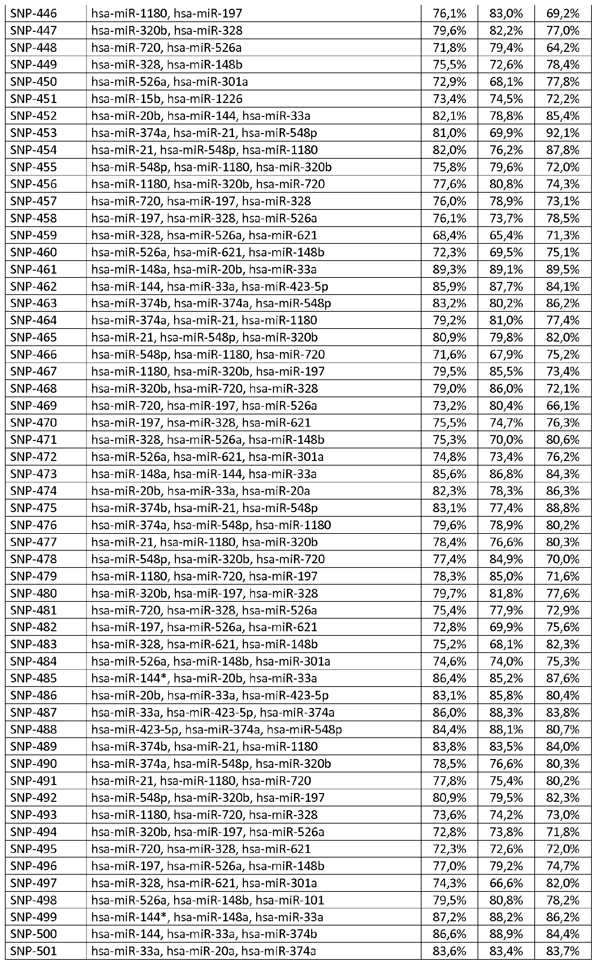

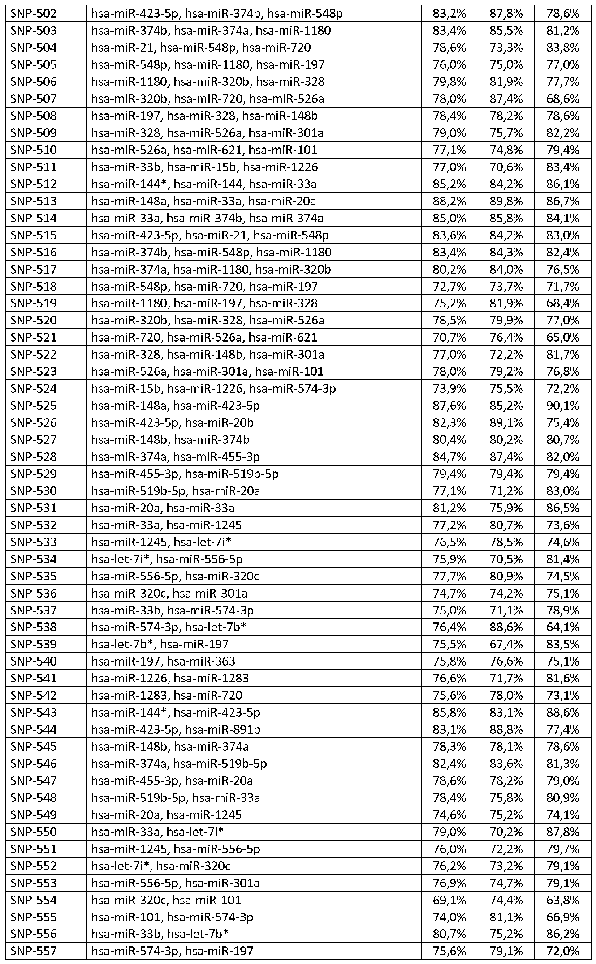

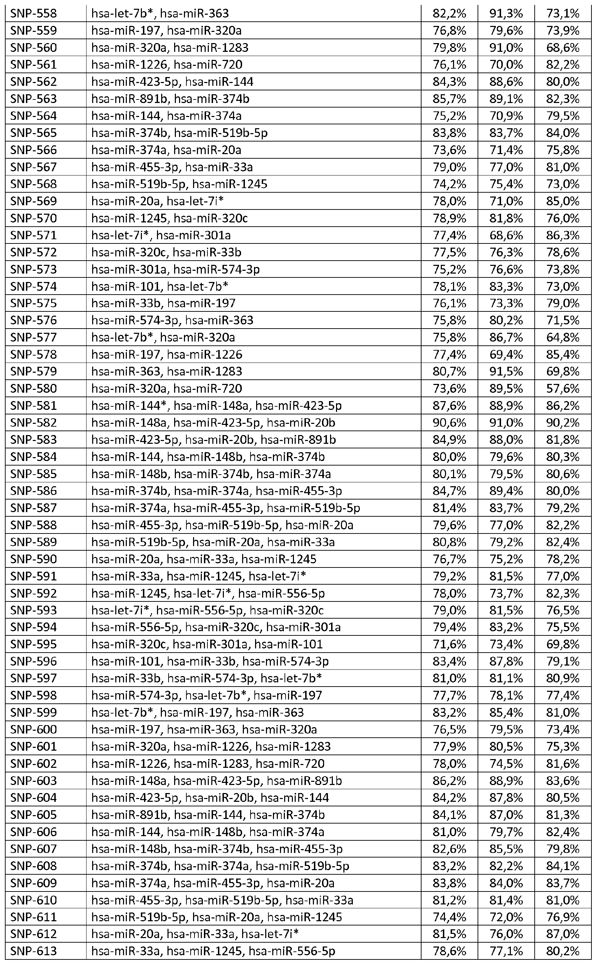

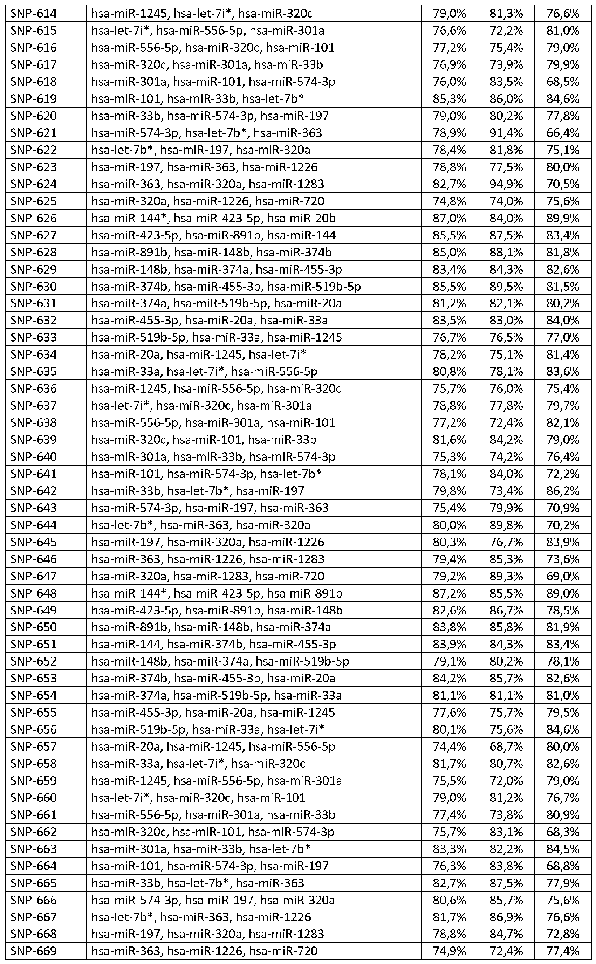

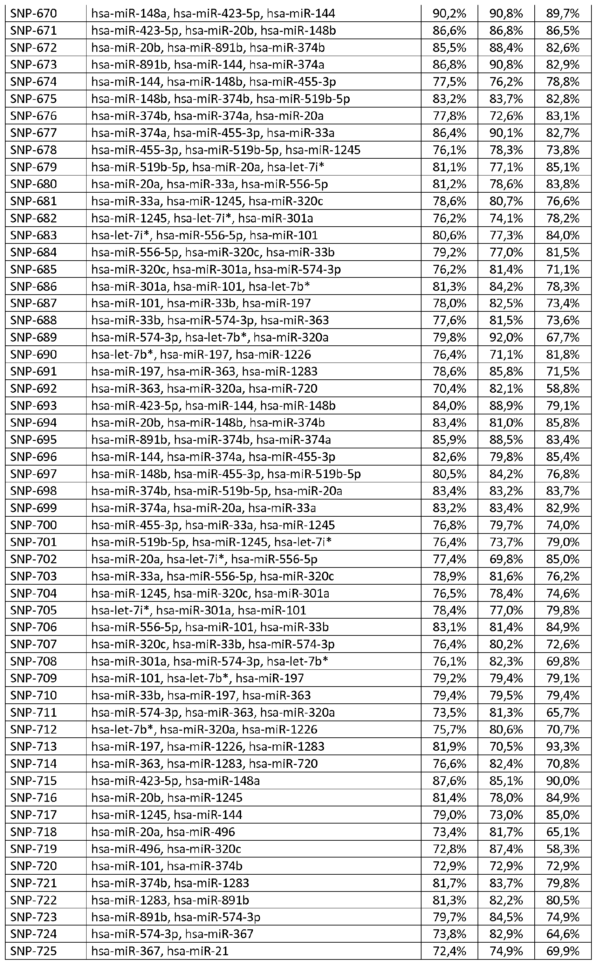

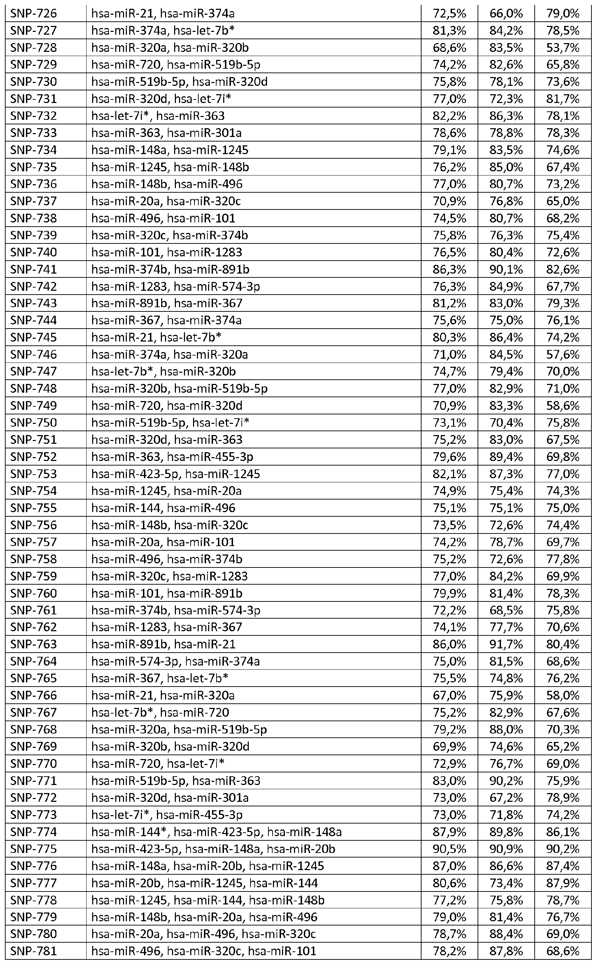

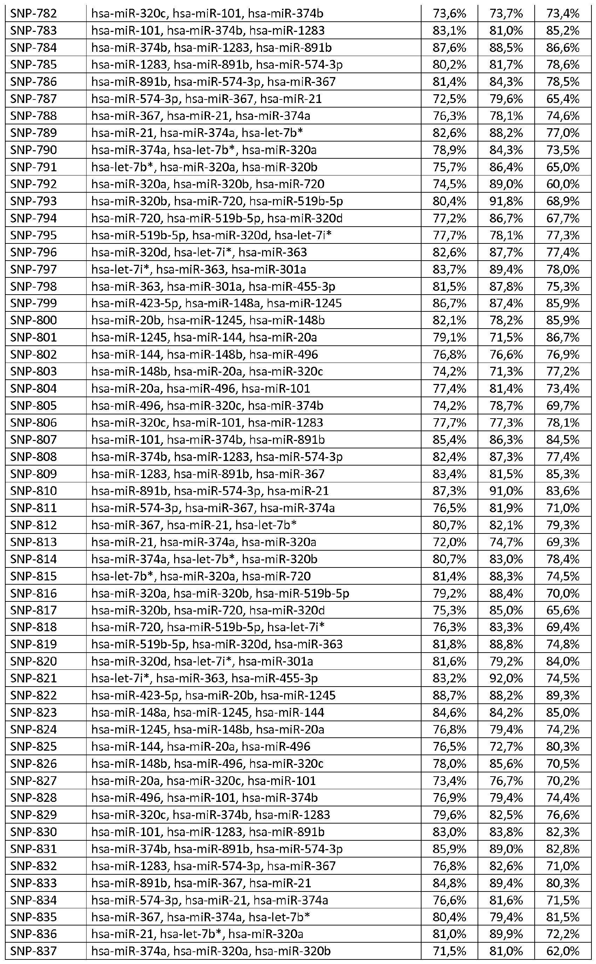

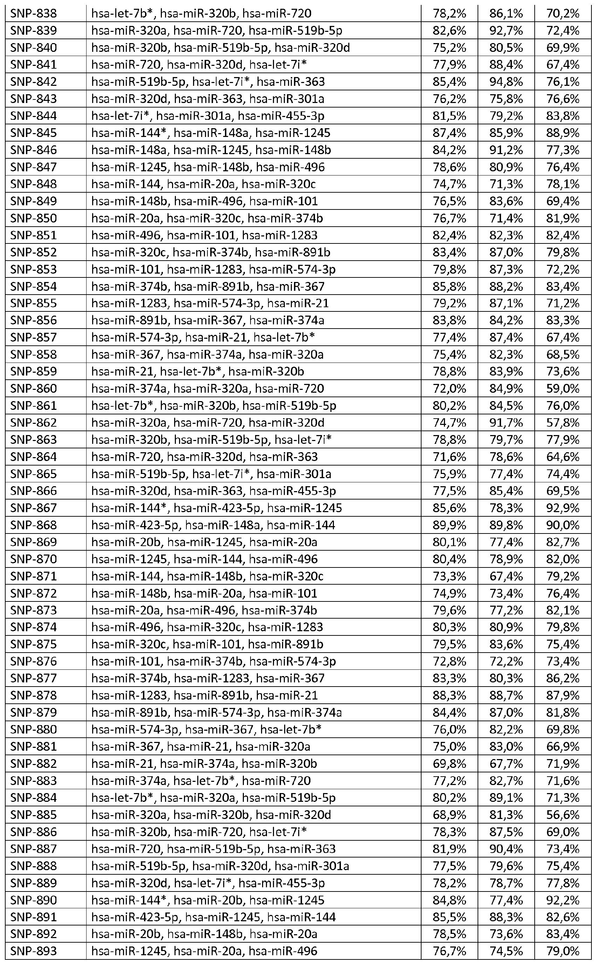

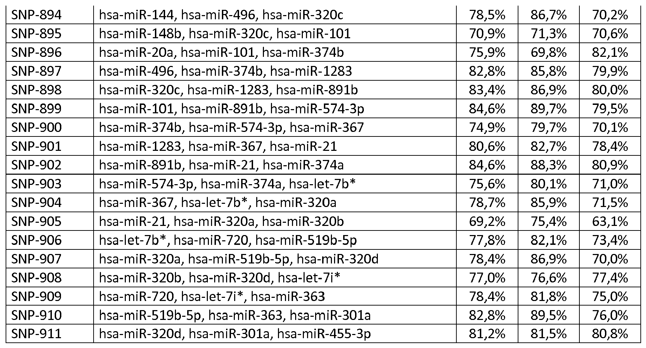

Further provided is a predetermined set comprising at least two miRNAs is selected from the sets of miRNAs listed in Figure 6 (SNP-1 to SNP-911). It is also preferred that the predetermined set comprising at least two miRNAs comprises at least one set of miRNAs listed in Figure 6.

Further, in a preferred embodiment of the method of the present invention, for determining an expression profile of the predetermined set comprising at least two miRNAs representative for prostate cancer in a body fluid sample from a subject comprises the miRNAs from one set or a plurality of sets of miRNAs listed in Figure 6.

For example, a set comprising 30 miRNAs representative for prostate cancer in a body fluid sample from a subject comprises at least the miRNAs from one predetermined set or several sets of miRNAs listed in Figure 6. Alternatively, a set comprising 29, 28, 27,26, 25, 24, 23, 22, 21, 20, 19, 18, 17, 16, 15, 14, 13, 12, 11, 10, 9, 8, 7, 6, 5, 4 or 3 miRNAs representative for prostate cancer comprises at least the miRNAs from one set or several sets of miRNAs listed in Figure 6.

Further, in another preferred embodiment of the method of the present invention, for determining an expression profile of the predetermined set comprising at least two miRNAs representative for prostate cancer in a body fluid sample from a subject comprises combinations of sets of miRNAs listed in Figure 6.

For example, said predetermined set comprising 30 miRNAs representative for prostate cancer in a body fluid sample from a subject comprises at least 2, e.g. 2, 3, 4, 5 or 6, sets of miRNAs listed in Figure 6. Alternatively, said set comprising 29, 28, 27 ,26, 25, 24, 23, 22, 21, 20, 19, 18, 17, 16, 15, 14, 13, 12, 11, 10, 9, 8, 7, 6, 5 or 4 miRNAs comprises a least 2, e.g. 2, 3, 4, 5 or 6, sets of miRNAs listed in Figure 6.

The reference expression profile may be obtained from at least two subjects (e.g. human or animal). Preferably the reference expression profile is an average expression profile (data) of at least 2 to 400 subjects, more preferably at least 20 to 200 subjects, and most preferably at least 40 to 150 subjects, with one known clinical condition which is prostate cancer or a specific form of prostate cancer.

It is particularly preferred that the reference expression profile is an algorithm or mathematical function. Preferably the algorithm or mathematical function is obtained from a reference expression profile (data) of at least two subjects, preferably the algorithm or mathematical function is obtained from an average reference expression profile (data) of at least 2 to 400 subjects, more preferably of at least 20 to 200 subjects, and most preferably of at least 40 to 150 subjects.

It is preferred that the algorithm or mathematical function is obtained using a machine learning approach.

Preferably, the algorithm or mathematical function is saved on a data carrier comprised in the kit (according to the seventh aspect of the invention) or the computer program, wherein the algorithm or mathematical function is comprised, is saved on a data carrier comprised in the kit.

It is preferred that the miRNA expression profile may be generated by any convenient means, e.g. nucleic acid hybridization (e.g. to a microarray), nucleic acid amplification (PCR, RT-PCR, qRT-PCR, high-throughput RT-PCR), ELISA for quantitation, next generation sequencing (e.g. ABI SOLID, Illumina Genome Analyzer, Roche/454 GS FLX), flow cytometry (e.g. LUMINEX) and the like, that allow the analysis of differential miRNA expression levels between samples of a subject (e.g. diseased) and a control subject (e.g. healthy, reference sample).

Nucleic acid hybridization may be performed using a microarray/biochip or *in situ* hybridization. *In situ* hybridization is preferred for the analysis of a single miRNA or a set comprising a low number of miRNAs (e.g. a set of at least 2 to 50 miRNAs such as a set of 2, 5, 10, 20, 30, or 40 miRNAs). The microarray/biochip, however, allows the analysis of a single miRNA as well as a complex set of miRNAs (e.g. a all known miRNAs or subsets therof).

Nucleic acid amplification may be performed using real time polymerase chain reaction (RT-PCR) such as real time quantitative polymerase chain reaction (RT qPCR). The standard real time polymerase chain reaction (RT-PCR) is preferred for the analysis of a single miRNA or a set comprising a low number of miRNAs (e.g. a set of at least 2 to 50 miRNAs such as a set of 2, 5, 10, 20, 30, or 40 miRNAs), whereas high-throughput RT-PCR technologies (e.g. OpenArray from Applied Biosystems, SmartPCR from Wafergen, Biomark System from Fluidigm) are also able to measure large sets of miRNAS (e.g a set of 10, 20, 30, 50, 80, 100, 200 or more) or all known miRNAs in a high parallel fashion. RT-PCR is particularly suitable for detecting low abandoned miRNAs.

In a second aspect, the invention relates to a set comprising polynucleotides for detecting a predetermined set comprising at least two miRNAs for diagnosing and/or prognosing of prostate cancer in a body fluid sample from a subject.

It is preferred that the body fluid sample is a blood sample, particularly preferred it is a whole blood, PBMC, serum or plasma sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

It is preferred that the predetermined set comprising at least two miRNAs is selected from the set of miRNAs listed in Figure 6. It is preferred that the predetermined set comprising at least two miRNAs comprises at least one set of miRNAs listed in Figure 6.

It is preferred that the polynucleotides comprised in the set of the present invention are complementary to the miRNAs comprised in the predetermined set, wherein the nucleotide sequences of said miRNAs are preferably selected from the group consisting of miRNAs or set of miRNAs listed in Figure 6, a fragment thereof, and a sequence having at least 80%, 85%, 90% or 95% sequence identity thereto.

For example, the polynucleotides of the present invention are for detecting a predetermined set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 miRNAs wherein the set of miRNAs comprises at least one, e.g. 1, 2, 3, 4, 5 or 6, of the set of miRNAs listed in Figure 6.

In a third aspect, the invention relates to the use of set of polynucleotides according to the second aspect of the invention for diagnosing and/or prognosing prostate cancer in a subject.

In a fourth aspect, the invention relates to a set of at least two primer pairs for determining the expression level of a predetermined set of miRNAs in a body fluid sample of a subject suffering or suspected of suffering from prostate cancer.

It is preferred that the body fluid sample is a blood sample, particularly preferred it is a whole blood, PBMC, serum or plasma sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

It is preferred that the predetermined set comprising at least two miRNAs is selected from the sets of miRNAs listed in Figure 6. It is preferred that the predetermined set comprising at least two miRNAs comprises at least one set of miRNAs listed in Figure 6.

It is preferred that the set of at least two primer pairs for determining the expression level of a predetermined set of miRNAs in a body fluid sample of a subject suffering or suspected of suffering from prostate cancer are primer pairs that are specific for at least one set of miRNAs listed in Figure 6.

It is preferred that the set of at least two primer pairs of the present invention are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40 or more miRNAs, and wherein the set of miRNAs comprises at least one of the sets listed in Figure 6.

For example, the set of at least two primer pairs of the present invention are for detecting a predetermined set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 or 2 miRNAs wherein the predetermined set of miRNAs comprises at least one of the set of miRNAs listed in Figure 6. Furthermore, the said primer pairs may be used for amplifying cDNA transcripts of the set of miRNAs listed in Figure 6.

It is understood that the primer pairs for detecting a predetermined set of miRNAs may consist of specific and or non-specific primers. Additionally, the set of primer pairs may be complemented by other substances or reagents (e.g. buffers, enzymes, dye, labelled probes) known to the skilled in the art for conducting real time polymerase chain reaction (RT-PCR).

In a fifth aspect, the invention relates to the use of a set of primer pairs according to the fourth aspect of the invention for diagnosing and/or prognosing prostate cancer in a subject.

In a sixth aspect, the invention relates to means for diagnosing and/or prognosing of prostate cancer in a body fluid sample of a subject.
Preferably, the invention relates to means for diagnosing and/or prognosing of prostate cancer in a body fluid sample of a subject comprising
(i) a set of at least two polynucleotides according to the second aspect of the invention or
(ii) a set of at least two primer pairs according the fourth aspect of the invention.

It is preferred that the body fluid sample is a blood sample, particularly preferred it is a whole blood, PBMC, serum or plasma sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

It is preferred that the set of at least two polynucleotides or the set of at least 2 primer pairs are for detecting a predetermined set comprising at least two miRNAs for diagnosing and/or prognosing of prostate cancer in a body fluid sample, e.g. blood sample, from a subject, e.g. patient, human or animal, wherein the set of miRNAs is selected from the sets of miRNAs listed in Figure 6.

It is preferred that the set of at least two primer pairs for determining the expression level of a set of miRNAs in a body fluid sample of a subject suffering or suspected of suffering from prostate cancer are primer pairs that are specific for at least one set of miRNAs listed in Figure 6.

It is also preferred that said means for diagnosing and/or prognosing of prostate cancer comprise, of a set of beads comprising a at least two polynucleotides according to the second aspect of the present invention. It is especially preferred that the beads are employed within a flow cytometer setup for diagnosing and/or prognosing of prostate cancer, e.g. in a LUMINEX system (*www.luminexcorp.com).*

In a seventh aspect, the invention relates to a kit for diagnosing and/or prognosing of prostate cancer in a subject.

Preferably, the invention relates to a kit for diagnosing and/or prognosing of prostate cancer comprising
(i) means for determining an expression profile of a predetermined set comprising at least two miRNAs representative for prostate cancer in a body fluid sample from a subject, and
(ii) at least one reference.

It is preferred that the body fluid sample is a blood sample, particularly preferred it is a whole blood, PBMC, serum or plasma sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

Said means may comprise of at least two polynucleotides according to the second aspect of the present invention, a set of at least 2 primer pairs according to the fourth aspect of the invention; means according to the sixth aspect of the present invention; primers suitable to perform reverse transcriptase reaction and/or real time polymerase chain reaction such as quantitative polymerase chain reaction; and/or means for conducting next generation sequencing.

In an eighth aspect, the invention relates to a predetermined set of miRNAs in a body fluid sample isolated from a subject for diagnosing and/or prognosing of prostate cancer.

It is preferred that the body fluid sample is a blood sample, particularly preferred it is a whole blood, PBMC, serum or plasma sample, more particularly preferred it is a whole blood sample.

It is preferred that the subject is a mammal including both a human and another mammal, e.g. an animal such as a mouse, a rat, a rabbit, or a monkey. It is particularly preferred that the subject is a human.

It is preferred that the predetermined set comprising at least two miRNAs is selected from the set of miRNAs listed in Figure 6. It is preferred that the predetermined set comprising at least two miRNAs comprises at least one set of miRNAs listed in Figure 6.

In a ninth aspect, the invention relates to the use of a set of miRNAs according to the eighth aspect of the invention for diagnosing and/or prognosing of prostate cancer in a subject. The invention will now be illustrated by the following figures and the non-limiting experimental examples.

### Figures

Figure 1: Overview of miRNA sequences published in the miRNA database 14.0 plus additional miRNA sequences.
Figure 2: Overview of all miRNAs that are found to be differentially regulated in blood samples of prostate cancer patients, grouped accordingly to their results in t-tests.

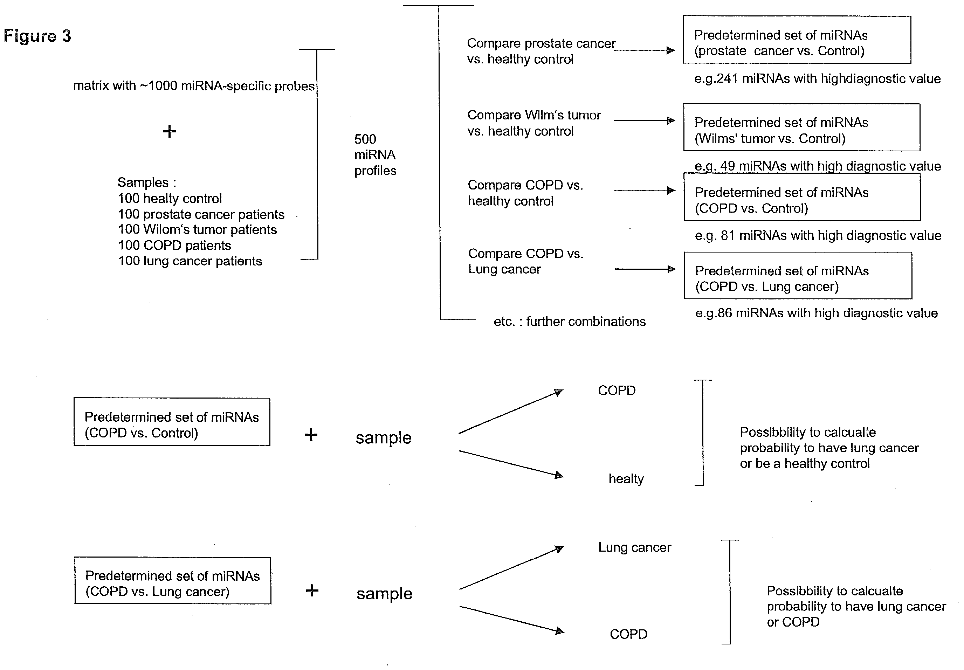

Figure 3: General overview of the method of diagnosing and/or predicting the state of health employing predetermined sets of miRNAs.
Figure 4: Prostate cancer patients against healthy controls - classification of: according to t-test with the 270 miRNAs with the lowest p-values (see Figure 2) lead to an accuracy 82.8 % a specificity of 87.5 % and a sensitivity of 71.9 %
   red = prostate cancer patients (1= derived from 1 independent sample collection); blue = healthy controls (1,2,3,4,5 = derived from 5 independent sample collections)
Figure 5: Overview of miRNAs that are found to be differentially regulated between healhy control and subjects suffering from prostate cancer. Experimental details: SEQ ID NO: sequence identification number, miRNA: identifier of the miRNA according to miRBase, median g1: median intensity obtained from microarray analysis for healthy controls, median g2: median intensity obtained from microarray analysis for individuals with prostate cancer, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.
Figure 6: Predetemined sets of miRNAs (miRNA signatures SNP-1 to 911) that allow for effective diagnosis and/or prognosis of subjects suffering or sujects suspected to suffering from prostate cancer. Experimental details: SEQ ID NO: sequence identification number, miRNA: identifier of the miRNA according to miRBase,Acc = accuracy, Spec = specificity, Sens = sensitivity

### References

Alvarez-Garcia, I. and E. A. Miska (2005). "MicroRNA functions in animal development and human disease." Development 132(21): 4653-62.
Benjamini, Y. and Y. Hochberg (1995). "Controlling the false discovery rate: A practical and powerful approach to multiple testing." J R Statist Soc B 57: 289-300.
Bolstad, B. M., R. A. Irizarry, et al. (2003). "A comparison of normalization methods for high density oligonucleotide array data based on variance and bias." Bioinformatics 19(2): 185-93.
Calin, G. A. and C. M. Croce (2006). "MicroRNA-cancer connection: the beginning of a new tale." Cancer Res 66(15): 7390-4.
Calin, G. A. and C. M. Croce (2006). "MicroRNA signatures in human cancers." Nat Rev Cancer 6(11): 857-66.
Chen, X., Y. Ba, et al. (2008). "Characterization of microRNAs in serum: a novel class of biomarkers for diagnosis of cancer and other diseases." Cell Res 18(10): 997-1006.
Crawford, M., E. Brawner, et al. (2008). "MicroRNA-126 inhibits invasion in non-small cell lung carcinoma cell lines." Biochem Biophys Res Commun 373(4): 607-12.
Esquela-Kerscher, A. and F. J. Slack (2006). "Oncomirs - microRNAs with a role in cancer." Nat Rev Cancer 6(4): 259-69.
Feitelson, M. A. and J. Lee (2007). "Hepatitis B virus integration, fragile sites, and hepatocarcinogenesis." Cancer Lett 252(2): 157-70.
Gilad, S., E. Meiri, et al. (2008). "Serum microRNAs are promising novel biomarkers." PLoS ONE 3(9): e3148.
Griffiths-Jones, S., R. J. Grocock, et al. (2006). "miRBase: microRNA sequences, targets and gene nomenclature." Nucleic Acids Res 34(Database issue): D140-4.
Griffiths-Jones, S., S. Moxon, et al. (2005). "Rfam: annotating non-coding RNAs in complete genomes." Nucleic Acids Res 33(Database issue): D121-4.
Griffiths-Jones, S., H. K. Saini, et al. (2008). "miRBase: tools for microRNA genomics." Nucleic Acids Res 36(Database issue): D154-8.
Guo, L., Z. X. Huang, et al. (2008). "Differential Expression Profiles of microRNAs in NIH3T3 Cells in Response to UVB Irradiation." Photochem Photobiol.
Harris, T. A., M. Yamakuchi, et al. (2008). "MicroRNA-126 regulates endothelial expression of vascular cell adhesion molecule 1." Proc Natl Acad Sci U S A 105(5): 1516-21.
Hayashita, Y., H. Osada, et al. (2005). "A polycistronic microRNA cluster, miR-17-92, is overexpressed in human lung cancers and enhances cell proliferation." Cancer Res 65(21): 9628-32.
He, L., J. M. Thomson, et al. (2005). "A microRNA polycistron as a potential human oncogene." Nature 435(7043): 828-33.
Henschke, C. I. and D. F. Yankelevitz (2008). "CT screening for lung cancer: update 2007." Oncologist 13(1): 65-78.
Hochberg, Y. (1988). "A sharper bonferroni procedure for multiple tests of significance." Biometrica 75: 185-193.
Ichimi, T., H. Enokida, et al. (2009). "Identification of novel microRNA targets based on microRNA signatures in bladder cancer." Int J Cancer.
Jemal, A., R. Siegel, et al. (2008). "Cancer statistics, 2008." CA Cancer J Clin 58(2): 71-96.
Johnson, S. M., H. Grosshans, et al. (2005). "RAS is regulated by the let-7 microRNA family." Cell 120(5): 635-47.
Keller, A., N. Ludwig, et al. (2006). "A minimally invasive multiple marker approach allows highly efficient detection of meningioma tumours." BMC Bioinformatics 7: 539.
Lee, R. C., R. L. Feinbaum, et al. (1993). "The C. elegans heterochronic gene lin-4 encodes small RNAs with antisense complementarity to lin-14." Cell 75(5): 843-54.
Lu, J., G. Getz, et al. (2005). "MicroRNA expression profiles classify human cancers." Nature 435(7043): 834-8.
Mann, H. and F. Wilcoxon (1947). "On a test whether one of two random variables is stochastically larger than the other." Ann Mat Stat 18: 50-60.
Sassen, S., E. A. Miska, et al. (2008). "MicroRNA: implications for cancer." Virchows Arch 452(1): 1-10.
Scott, W. J., J. Howington, et al. (2007). "Treatment of non-small cell lung cancer stage I and stage II: ACCP evidence-based clinical practice guidelines (2nd edition)." Chest 132(3 Suppl): 234S-242S.
Shannon, C. (1984). "A mathematical theory of communication." The Bell System Technical Journal 27: 623-656.
Stahlhut Espinosa, C. E. and F. J. Slack (2006). "The role of microRNAs in cancer." Yale J Biol Med 79(3-4): 131-40.
Takamizawa, J., H. Konishi, et al. (2004). "Reduced expression of the let-7 microRNAs in human lung cancers in association with shortened postoperative survival." Cancer Res 64(11): 3753-6.
Team, R. D. C. (2008). R: A Language and Environment for Statistical Computing.. Vienna, Austria, R Foundation for Statistical Computing.
Tong, A. W. (2006). "Small RNAs and non-small cell lung cancer." Curr Mol Med 6(3): 339-49.
Vapnik, V. (2000). The Nature of Statistical Learning Theory., Springer.
Volinia, S., G. A. Calin, et al. (2006). "A microRNA expression signature of human solid tumours defines cancer gene targets." Proc Natl Acad Sci U S A 103(7): 2257-61.
Vorwerk, S., K. Ganter, et al. (2008). "Microfluidic-based enzymatic on-chip labeling of miRNAs." N Biotechnol 25(2-3): 142-9.
Wilcoxon, F. (1945). "Individual comparisons by ranking methods." Biometric Bull 1: 80-83.
Williams, A. E. (2008). "Functional aspects of animal microRNAs." Cell Mol Life Sci 65(4): 545-62.
Yanaihara, N., N. Caplen, et al. (2006). "Unique microRNA molecular profiles in lung cancer diagnosis and prognosis." Cancer Cell 9(3): 189-98.
Zhang, B., X. Pan, et al. (2007). "microRNAs as oncogenes and tumour suppressors." Dev Biol 302(1): 1-12.
Zhang, B., Q. Wang, et al. (2007). "MicroRNAs and their regulatory roles in animals and plants." J Cell Physiol 210(2): 279-89.

## Claims

1. A method of diagnosing prostate cancer, comprising the steps of:
(a) determining an expression profile of a predetermined set of miRNAs in a blood cellular fraction comprising erythrocytes, leukocytes, and thrombocytes from a patient, particularly a human patient; and
(b) comparing said expression profile to a reference expression profile,
wherein the comparison of said determined expression profile to said reference expression profile allows for the diagnosis of prostate cancer, and
wherein the predetermined set of miRNAs comprises at least one set of miRNAs listed in Figure 6.

2. The method according to claim 1, wherein the expression profile is determined by nucleic acid hybridization, nucleic acid amplification, polymerase extension, sequencing, mass spectroscopy, flow cytometry or any combinations thereof.

3. Use of a set of polynucleotides for detecting a predetermined set of miRNAs for diagnosing prostate cancer in a blood cellular fraction comprising erythrocytes, leukocytes, and thrombocytes from a patient, particularly a human patient, wherein the predetermined set of miRNAs comprises at least one set of miRNAs listed in Figure 6.

4. Use of a set of primer pairs for determining the expression level of a predetermined set of miRNAs in a blood cellular fraction comprising erythrocytes, leukocytes, and thrombocytes of a patient for diagnosing prostate cancer, wherein the predetermined set of miRNAs comprises at least one set of miRNAs listed in Figure 6.

5. Use of a kit for diagnosing prostate cancer in a blood cellular fraction comprising erythrocytes, leukocytes, and thrombocytes of a subject, wherein said kit comprises:
(a) means for determining an expression profile of a predetermined set of miRNAs in a blood cellular fraction comprising erythrocytes, leukocytes, and thrombocytes of a subject, wherein the predetermined set of miRNAs comprises at least one set of miRNAs listed in Figure 6, wherein said means comprise,
(ai) a set of polynucleotides as defined in claim 3, and/or
(aii) a set of primer pairs as defined in claim 4,
and
(b) at least one reference expression profile for a particular condition.

6. Use of a set of miRNAs isolated from a blood cellular fraction comprising erythrocytes, leukocytes, and thrombocytes from a subject for diagnosing prostate cancer, wherein the set of miRNAs comprises at least one set of miRNAs listed in Figure 6.

## Patentansprüche

1. Verfahren zur Diagnose von Prostatakrebs, welches die Schritte umfasst:
(i) Ermitteln eines Expressionsprofils eines vorbestimmten Sets von miRNAs in einer Blutzellfraktion, die Erythrozyten, Leukozyten und Thrombozyten umfasst, eines Patienten, insbesondere eines Menschen als Patienten, und
(ii) Vergleichen des Expressionsprofils mit einem Referenzexpressionsprofil, wobei der Vergleich des ermittelten Expressionsprofils mit dem Referenzexpressionsprofil die Diagnose von Prostatakrebs erlaubt, und wobei das vorbestimmte Set von miRNAs mindestens ein Set von miRNAs umfasst, welches in Figur 6 aufgeführt ist.

2. Verfahren nach Anspruch 1, wobei das Expressionsprofil mittels Nukleinsäurehybridisierung, Nukleinsäureamplifikation, Polymeraseverlängerung, Sequenzierung, Massenspektrometrie, Durchflusszytometrie oder mittels jeder Kombination davon ermittelt wird.

3. Verwendung eines Sets von Polynukleotiden zur Detektion eines vorbestimmten Sets von miRNAs zur Diagnose von Prostatakrebs in einer Blutzellfraktion, die Erythrozyten, Leukozyten und Thrombozyten umfasst, eines Patienten, insbesondere eines Menschen als Patienten, wobei das vorbestimmte Set von miRNAs mindestens ein Set von miRNAs umfasst, welches in Figur 6 aufgeführt ist.

4. Verwendung eines Primerpaares zur Ermittlung des Expressionslevels eines vorbestimmten Sets von miRNAs in einer Blutzellfraktion, die Erythrozyten, Leukozyten und Thrombozyten umfasst, eines Patienten zur Diagnose von Prostatakrebs, wobei das vorbestimmte Set von miRNAs mindestens ein Set von miRNAs umfasst, welches in Figur 6 aufgeführt ist.

5. Verwendung eines Kits zur Diagnose von Prostatakrebs in einer Blutzellfraktion, die Erythrozyten, Leukozyten und Thrombozyten umfasst, eines Subjekts wobei das Kit umfasst:
(a) Mittel zur Ermittlung eines Expressionsprofils eines vorbestimmten Sets von miRNAs in einer Blutzellfraktion, die Erythrozyten, Leukozyten und Thrombozyten umfasst, eines Subjekts, wobei das vorbestimmte Set von miRNAs mindestens ein Set von miRNAs umfasst, welches in Figur 6 aufgeführt ist, wobei die Mittel umfassen
(ai) ein Set von Polynukleotiden wie in Anspruch 3 definiert, und/oder
(aii) ein Set von Primerpaaren wie in Anspruch 4 definiert, und
(b) mindestens ein Referenzexpressionsprofil für den gesunden Zustand.

6. Verwendung eines Sets von miRNAs isoliert aus einer Blutzellfraktion, die Erythrozyten, Leukozyten und Thrombozyten umfasst, eines Subjekts zur Diagnose von Prostatakrebs, wobei das Set von miRNAs mindestens ein Set von miRNAs umfasst, welches in Figur 6 aufgeführt ist.

## Revendications

1. Procédé de diagnostic du cancer de la prostate, comprenant les étapes de :
(a) détermination d'un profil d'expression d'un ensemble prédéterminé de miARN dans une fraction cellulaire du sang comprenant des érythrocytes, des leucocytes et des thrombocytes d'un patient, en particulier d'un patient humain ; et
(b) comparaison dudit profil d'expression à un profil d'expression de référence,
la comparaison dudit profil d'expression déterminé audit profil d'expression de référence permettant le diagnostic du cancer de la prostate, et
l'ensemble prédéterminé de miARN comprenant au moins un ensemble de miARN énumérés dans la figure 6.

2. Procédé selon la revendication 1, dans lequel le profil d'expression est déterminé par une hybridation d'acides nucléiques, une amplification d'acides nucléiques, une extension par polymérase, un séquençage, une spectroscopie de masse, une cytométrie en flux ou toute combinaison de ceux-ci.

3. Utilisation d'un ensemble de polynucléotides permettant de détecter un ensemble prédéterminé de miARN pour le diagnostic du cancer de la prostate dans une fraction cellulaire du sang comprenant des érythrocytes, des leucocytes et des thrombocytes d'un patient, en particulier d'un patient humain, l'ensemble prédéterminé de miARN comprenant au moins un ensemble de miARN énumérés dans la figure 6.

4. Utilisation d'un ensemble de paires d'amorces permettant de déterminer le niveau d'expression d'un ensemble prédéterminé de miARN dans une fraction cellulaire du sang comprenant des érythrocytes, des leucocytes et des thrombocytes d'un patient pour le diagnostic du cancer de la prostate, l'ensemble prédéterminé de miARN comprenant au moins un ensemble de miARN énumérés dans la figure 6.

5. Utilisation d'un kit pour le diagnostic du cancer de la prostate dans une fraction cellulaire du sang comprenant des érythrocytes, des leucocytes et des thrombocytes d'un sujet, ledit kit comprenant :
(a) des moyens permettant de déterminer un profil d'expression d'un ensemble prédéterminé de miARN dans une fraction cellulaire du sang comprenant des érythrocytes, des leucocytes et des thrombocytes d'un sujet, l'ensemble prédéterminé de miARN comprenant au moins un ensemble de miARN énumérés dans la figure 6, lesdits moyens comprenant,
(ai) un ensemble de polynucléotides selon la revendication 3, et/ou
(aii) un ensemble de paires d'amorces selon la revendication 4,
et
(b) au moins un profil d'expression de référence pour l'état de bonne santé.

6. Utilisation d'un ensemble de miARN isolés d'une fraction cellulaire du sang comprenant des érythrocytes, des leucocytes et des thrombocytes d'un sujet pour le diagnostic du cancer de la prostate, l'ensemble de miARN comprenant au moins un ensemble de miARN énumérés dans la figure 6.
